# EUROPEAN PATENT APPLICATION

(11) **EP 4 556 004 A1**
(43) Date of publication of application: **21.05.2025**
(21) Application number: 23306996.2
(22) Date of filing: 17.11.2023
(51) Int. Cl.: A61K 31/122, A61P 11/00, A61P 31/12

(54) **HOP DERIVED COMPOUNDS FOR USE IN THE TREATMENT OF DISEASES CAUSED BY CORONAVIRUSES**

(71) Applicant: Centre National de la Recherche Scientifique, 75016 Paris (FR); Institut National de la Santé et de la Recherche Médicale, 75013 Paris (FR); Institut Pasteur de Lille, 59019 Lille Cedex (FR); Université de Strasbourg, 67081 Strasbourg Cedex (FR); Université de Lille, 59800 Lille (FR); Centre Hospitalier Universitaire de Lille, 59037 Lille Cedex (FR); Université de Haute Alsace, 68093 Mulhouse Cedex (FR)
(72) Inventor: ELHABIRI, Mourad, 67200 Strasbourg (FR); SERON, Karin, 59800 LILLE (FR); RIVIERE, Céline, 59650 Villeneuve d Ascq (FR)
(74) Representative: Novagraaf Technologies

(57) **Abstract**

The present invention belongs to the field of compounds for use in therapeutic treatment, and more particularly hop derived compounds for use in the treatment of diseases caused by coronaviruses.

The main field of application is human health, through the development of new active beta-acid-type antivirals against SARS-CoV-2

The present invention relates to hop derived compounds according to the invention for use in the treatment of diseases caused by a virus chosen from coronaviruses belonging to the Coronaviridae family.

## Description

### Technical field

The present invention belongs to the field of compounds for use in therapeutic treatment, and more particularly hop derived compounds for use in the treatment of diseases caused by coronaviruses.

The main field of application is human health, through the development of new active beta-acid-type antivirals against SARS-CoV-2
The present invention relates to hop derived compounds according to the invention for use in the treatment of diseases caused by a virus chosen from coronaviruses belonging to the Coronaviridae family.

### Technological background

The emergence of SARS-CoV-2, responsible for the COVID-19 pandemic, has highlighted the lack of specific treatments against coronaviruses. The current public health challenges mean that it is essential to put in place an effective strategy at global level and to combine vaccination with a broad arsenal of therapies essential to the treatment of infection by SARS-CoV-2, as well as any other coronavirus that may emerge in the future. The inventors, comprising 3 teams specialising in virology, phytochemistry and chemistry, have identified a family of molecules (prenylated acylphloroglucinols) derived from hops and active against SARS-CoV-2. Various natural analogues were purified from the plant and synthetic analogues were also obtained, making it possible to consolidate a structure-activity relationship (SAR) study and identify the most effective molecules (IC₅₀ less than 10 µM). Several of these were identified as being active on several coronaviruses (HCoV-229E, SARS-CoV-2 and MERS-CoV), demonstrating their pan-coronavirus activity. These preliminary in-vitro results, backed up by results obtained in a preclinical model of human bronchial epithelium cells, encourage us to capitalise on these results by filing a patent application. To date, there have been very few reports describing the possible antiviral properties of acylphloroglucinol derivatives, with, for example, activity against the Chikungunya virus (CHIKV) showing promising inhibition in post treatment assay [1]. This reinforces our discovery and the need for such intellectual property protection.

Before the emergence of Severe Acute Respiratory Syndrome or SARS (SARS-CoV-1 in 2002), only two human coronaviruses (HCoV), HCoV-229E and HCoV-OC43, causing only mild respiratory tract infections (15-30% of common colds), were known. In 2012, the emergence of MERS-CoV, with a mortality rate of over 30%, alerted the community to the need to develop effective therapeutic options to combat HCoV infections. None of the antiviral agents identified *in vitro* had reached clinical trials. Moreover, no vaccine is currently effective against this virus. A new coronavirus, SARS-CoV-2, appeared at the end of 2019 in Wuhan (China), giving rise to a new epidemic by a pneumonia-like illness (COVID-19). SARS-CoV-2's strong capacity to mutate (alpha, beta, gamma, delta and omicron variants) makes it increasingly contagious, and many countries, including France, were facing successive epidemic waves.

In September 2023, more than 6.9 million deaths and over 695 million people testing positive had been recorded worldwide (about 40.1 million people infected in France and 167,000 deaths). The COVID-19 pandemic demonstrates that viral respiratory infections are a permanent threat to global health and that effective therapeutic solutions need to be developed as a matter of urgency.

Preventive measures and containment limit the spread of the virus. To date, five vaccines against COVID-19 (messenger RNA vaccines COMIRNATY from Pfizer/BioNTech and mRNA-1273 from Moderna, adenovirus vaccines VAXZEVRIA from AstraZeneca and As26.COV2.S from Janssen, adjuvanted protein vaccine NUVAXOVID from Novavax) have been approved for marketing in Europe. Their use in France follows the vaccination strategy laid down by the European Commission Haute Autorité de Santé (HAS). At the same time, several therapeutic approaches have been implemented with varying degrees of success. The repositioning of certain existing antiviral drugs (Keletra, Remdesivir, Favipivavir, Avigan, Interferon-β1a) and certain immunomodulators (Anakinra, Tocilizumab, Sarilumab and Tofacitinib) have not been convincing in treating SARS-CoV-2. Remdesivir, an antiviral against the repositioned Ebola virus, has been granted emergency approval by the FDA, but has not been approved by the HAS in France. Several therapeutic approaches are attempting to develop an oral formulation of Remdesivir (JGL-2020 from Gilead and Jubilant, ODBG-P-RVn from the University of California San Diego or GS-621763 from Gilead and Georgia State University).

The most effective treatments (around 80% efficacy) are based on neutralising monoclonal antibodies offered by several laboratories (Bamlanivimab/Etesevimab from Eli Lilly, Ronapreve from Regeneron and Roche, Evusheld from AstraZeneca, Regkirona from Celltrion, Xevudy from GlaxoSmithKline and Vir Biotechnology and ADG20 from Adagio). These antibodies specifically recognise the Spike surface protein (S protein) of SARS-CoV-2.

However, the successive emergence of several variants of the Omicron family (more than 1,000 different variants listed in March 2023) has rendered all the monoclonal antibodies available on the market ineffective.

Only Dexamethasone, a synthetic corticosteroid with anti-inflammatory and anti-allergic properties, was authorised by the European Medicines Agency, and has been shown to be effective against severe forms of COVID-19, particularly those requiring oxygen administration.

Drug repositioning continues to be a widely used approach. Molnupiravir (Merck's Lagevrio), a repositioned drug developed to combat the Venezuelan equine encephalitis virus, is used in the United States and Great Britain. However, it has not been authorised by the HAS, and recent studies have shown its ineffectiveness in severe forms of the disease. The number of deaths seems to diminish the relevance of using this compound in the future.

In terms of new anti-SARS-CoV-2 treatments, Pfizer recently launched a protease inhibitor, Paxlovid (nirmatrelvir + ritonavir), which reduces the risk of hospitalisation and death by almost 90% if the treatment is taken during the first few days after the onset of symptoms. Paxlovid has been granted emergency approval by the FDA and HAS. Several other therapeutic approaches are in development (e.g. S-217622 in phase III by Shionogi, Lufotrelvir in phase I by Pfizer, PBI-0451 in phase I by Pardes, EDP-235 in phase I by Entana, 13b-K by the University of Lübeck).

To date, the therapeutic arsenal against SARS-CoV-2 is therefore limited to RNA, adenovirus and recombinant protein vaccines, dexamethasone (anti-inflammatory) and Paxlovid. Despite these advances, the therapeutic arsenal remains limited, and new broad-spectrum antivirals against endemic, epidemic or zoonotic CoVs are urgently needed.

There is therefore a need to broaden the therapeutic arsenal that can be made available to the public for treatment against infection by SARS-CoV-2, but also against any other HCoV that may emerge in the future.

### Detailed invention

The inventors surprisingly found out that a family of hop-derived compounds were very potent candidates for broadening the therapeutic arsenal that can be made available to the public for treatment against infection by SARS-CoV-2, but also against any other HCoV that may emerge in the future.

On a first aspect, the invention relates to a compound of formula (I): 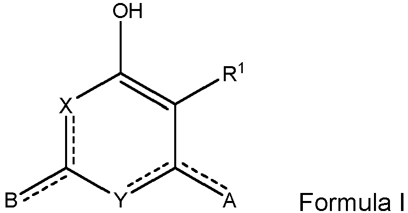 wherein,
- R¹ is a substituted or unsubstituted, saturated or unsaturated, linear or branched, acyl group comprising 10 or fewer carbon atoms, optionally comprising an aryl moiety;
- A represents O or OH
- B represents O, OH, or OR⁶, in which R⁶ represents group or a C₁ to C₃ alkyl chain optionally comprising a C₁ to C₃ alkoxy or a phenyl;
provided that
- when A is O, then Y is C(R²)₂, in which each R² group independently represents H or a linear or branched C₅ to C₁₀ unsaturated hydrocarbon chain comprising at least one alkene moiety;
- when A is OH, then Y is CR³, in which R³ represents H or a linear or branched C₅ to C₁₀ unsaturated hydrocarbon chain comprising at least one alkene moiety;
- when B is O, then X is C(R⁴)₂, in which each R⁴ independently represents H or a linear or branched C₅ to C₁₀ unsaturated hydrocarbon chain comprising at least one alkene moiety;
- when B is OH or OR⁶, then X is CR⁵, in which R⁵ represents H, a linear or branched C₅ to C₁₀ unsaturated hydrocarbon chain comprising at least one alkene moiety, or a substituted or unsubstituted, saturated or unsaturated, linear or branched, acyl group comprising 10 or fewer carbon atoms, optionally comprising an aryl moiety;
- wherein the symbol 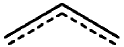 represents a chain comprising one double bond, the position of the double bond being linked to the nature of A and/or B,
for use in the treatment of diseases caused by a virus chosen from coronaviruses belonging to the Coronaviridae family.

Advantageously, the virus may belong to the Orthocoronavirinae subfamily. Preferably, the virus belongs to:
- the Alphacoronavirus genus and the Duvinacovirus subgenus (HCoV-229E, Human coronavirus 229E),
- the Betacoronavirus genus and the Sarbecovirus subgenus (SARS-CoV, Severe acute respiratory syndrome coronavirus; SARS-CoV-2, Severe acute respiratory syndrome coronavirus-2, original Wuhan strain and D614G, Alpha, Beta, Delta and Omicron variants), or
- the Betacoronavirus genus and Merbecovirus subgenus (MERS-CoV, Middle-East respiratory syndrome coronavirus).

Advantageously, when A is O, the compound of formula I may be in the form of a mixture of tautomers depending on the solvent conditions. For example, the tautomerization is possible in chloroform, but there is no tautomerization in alcohol, water or DMSO.

For use according to the invention, some of the compounds may be of natural origin and may be extracted from certain species belonging to the genus *Humulus,* in particular *H. lupulus* L. or *H. scandens* (Lour.) Merr. (= *H. japonicus* Siebold & Zucc.) for example. Crude extracts from several *H. lupulus* genotypes (commercial and wild hops), as well as fractions enriched with this said compounds, may be for use according to the invention.

Advantageously, the compound for use according to the invention, may be of natural or synthetic origin.

Advantageously, the compound according to the invention may be chosen from the compounds of formula (I), wherein R¹ is a substituted or unsubstituted, saturated or unsaturated, linear or branched, acyl group comprising 10 or fewer carbon atoms, optionally comprising an aryl moiety. The R¹ group is defined such as the 10 or fewer carbon atoms relate to the main acyl chain thereby excluding the optional aryl moiety or substituent. The main acyl chain may comprise an aryl moiety and/or may bear a substituent. The compound according to the invention may thus be chosen from the compounds of formula (I), wherein R¹ is substituted by a fluorescent moiety or a halo group, preferably fluoro group. The compound according to the invention may be chosen from the compounds of formula (I), wherein R¹ comprises or bears an aryl moiety. Preferably, the compound according to the invention may be chosen from the compounds of formula (I), wherein R¹ comprises 4 to 6 carbon atoms. Alternatively, the compound according to the invention may be chosen from the compounds of formula (I), wherein R¹ comprises 2 to 10 carbon atoms.

Preferably, the R¹ may be chosen from the following structures: 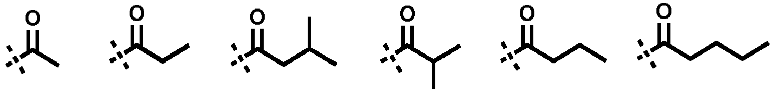 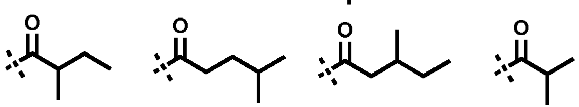 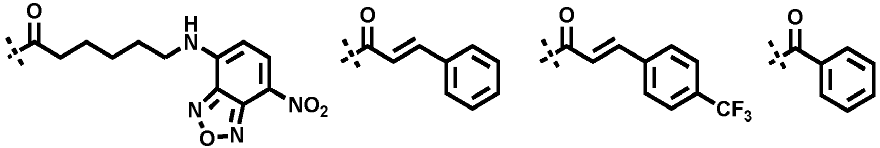 in which 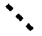 represents the point of attachment to the compound according to the invention.

Advantageously, each R² group may independently represent H or a linear or branched C₅ to C₁₀ unsaturated hydrocarbon chain comprising at least one alkene moiety. Preferably, each R² group independently represents H or a group chosen from the following structures (prenyl and geranyl): 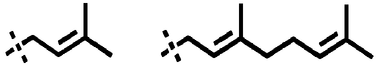 in which 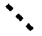 represents the point of attachment to the main carbon atom of Y, on the compound according to the invention.

Advantageously, the R³ group may represent H or a linear or branched C₅ to C₁₀ unsaturated hydrocarbon chain comprising at least one alkene moiety. Preferably, the R³ group independently represents H or a group chosen from the following structures (prenyl and geranyl): 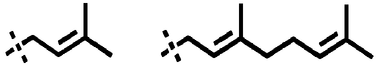 in which 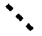 represents the point of attachment to the main carbon atom of Y, on the compound according to the invention.

Advantageously, each R⁴ group may independently represent H or a linear or branched C₅ to C₁₀ unsaturated hydrocarbon chain comprising at least one alkene moiety. Preferably, each R⁴ group independently represents H or a group chosen from the following structures (prenyl and geranyl): 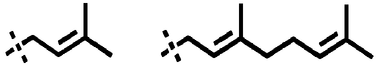 in which 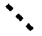 represents the point of attachment to the main carbon atom of X, on the compound according to the invention.

Advantageously, the R⁵ group may represent H, a linear or branched C₅ to C₁₀ unsaturated hydrocarbon chain comprising at least one alkene moiety or a substituted or unsubstituted, saturated or unsaturated, linear or branched, acyl group comprising 10 or fewer carbon atoms, optionally comprising an aryl moiety. Preferably, the R⁵ group represents H or a group chosen from the following structures (prenyl and geranyl): 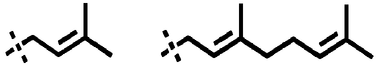 in which 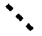 represents the point of attachment to the main carbon of X, on the compound according to the invention.

Advantageously, the compound according to the invention may be chosen from the compounds of formula (I), wherein the linear or branched C₅ to C₁₀ unsaturated hydrocarbon chain(s) comprising at least one alkene moiety may be selected from prenyl and geranyl groups.

Advantageously, the R⁵ group may represent H or a substituted or unsubstituted, saturated or unsaturated, linear or branched, acyl group comprising 10 or fewer carbon atoms, optionally comprising an aryl moiety. Preferably, each R⁵ group independently represents H or a group chosen from the following structures: 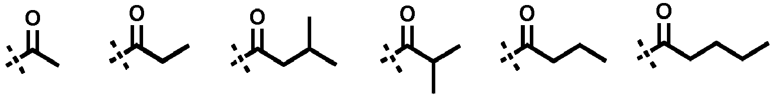 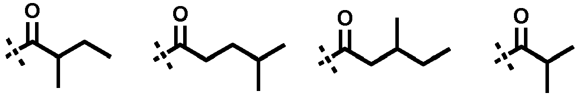 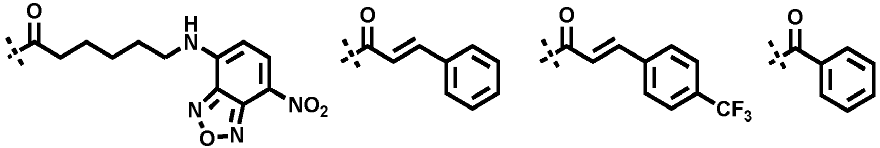 in which 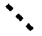 represents the point of attachment to the main carbon of X, on the compound according to the invention.

Advantageously, the compound according to the invention may be chosen from the compounds of formula (I), wherein A is O and B is OH or OR⁶: 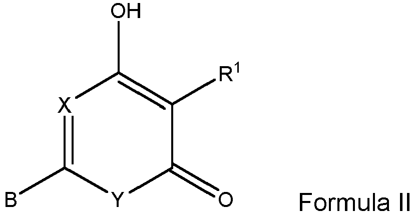 wherein B is OH or OR⁶, and R¹, R⁶, X and Y are defined as above (X is CR⁵, Y is C(R²)₂).

Advantageously, the compound of formula II may be in the form of a mixture of tautomers depending on the solvent conditions. For example, the tautomerization is possible in chloroform, but there is no tautomerization in alcohol, water or DMSO.

Advantageously, the compound according to the invention may be chosen from the following compounds:

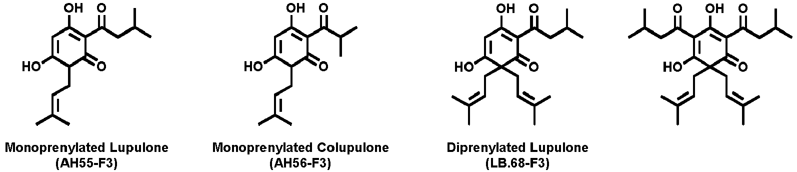

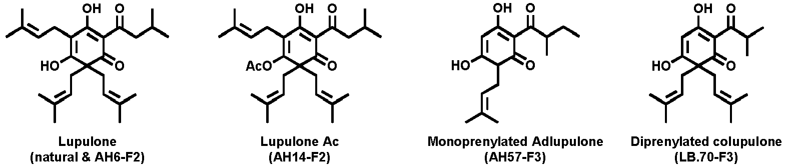

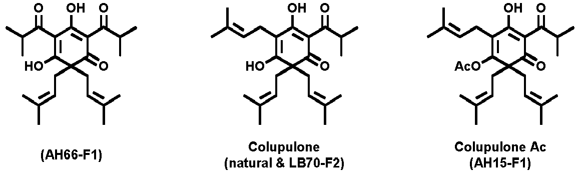

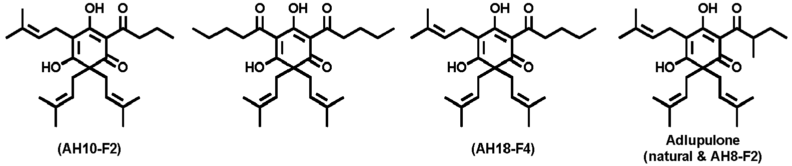

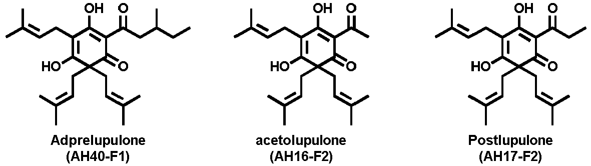

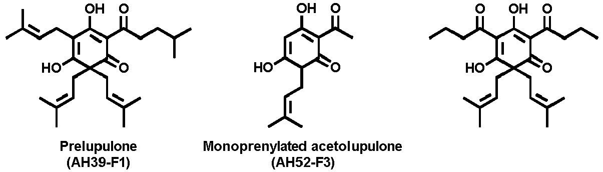

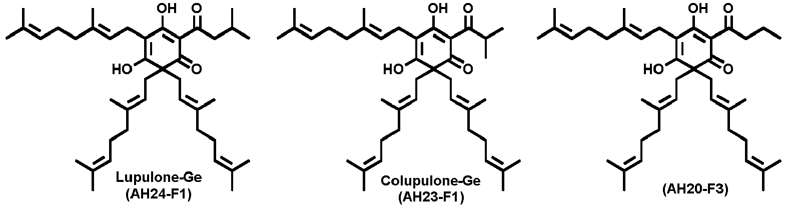

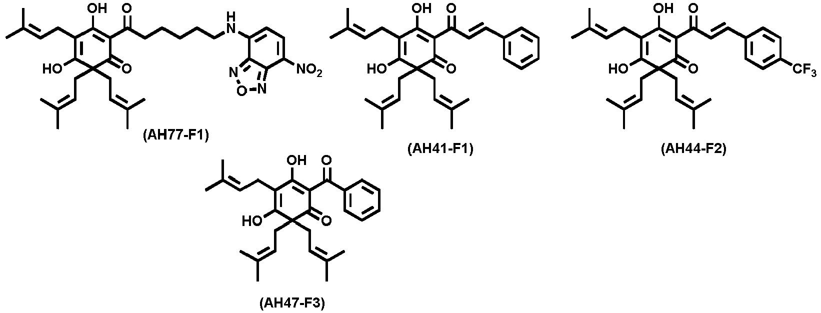

Advantageously, the compound according to the invention may be chosen from the compounds of formula (I), wherein A and B are OH: 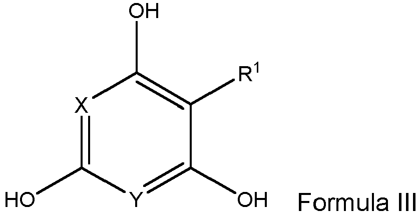 wherein R¹, X and Y are defined as above (X is CR⁵, Y is CR³). The compound according to the invention may be chosen from the compounds of formula (III), wherein R1 and R5 are identical. The compound according to the invention may be chosen from the compounds of formula (III), wherein R³ and R⁵ are H. The compound according to the invention may be chosen from the compounds of formula (III), wherein R³ and R⁵ are prenyl groups. The compound according to the invention may be chosen from the compounds of formula (III), wherein R³ is a prenyl group and R⁵ is a substituted or unsubstituted, saturated or unsaturated, linear or branched, acyl group comprising 10 or fewer carbon atoms, optionally comprising an aryl moiety.

Advantageously, the compound according to the invention may be chosen from the following compounds:

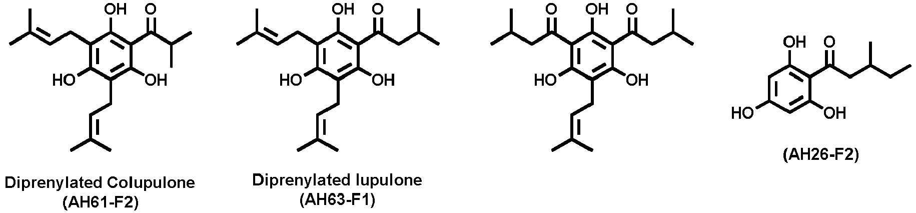

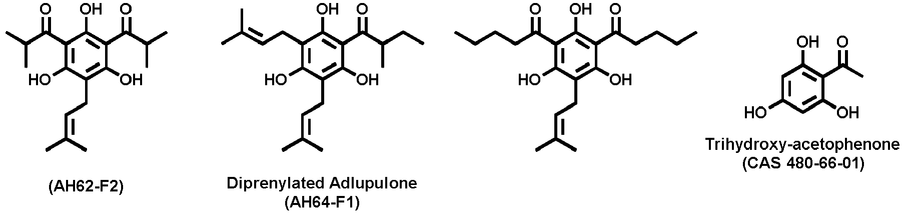

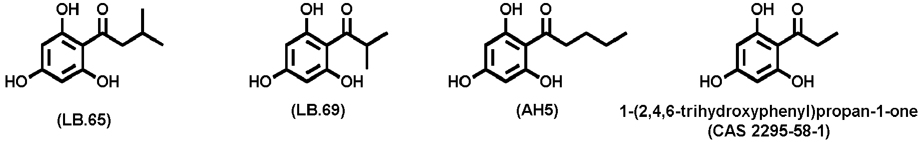

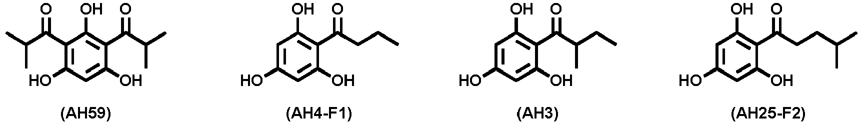

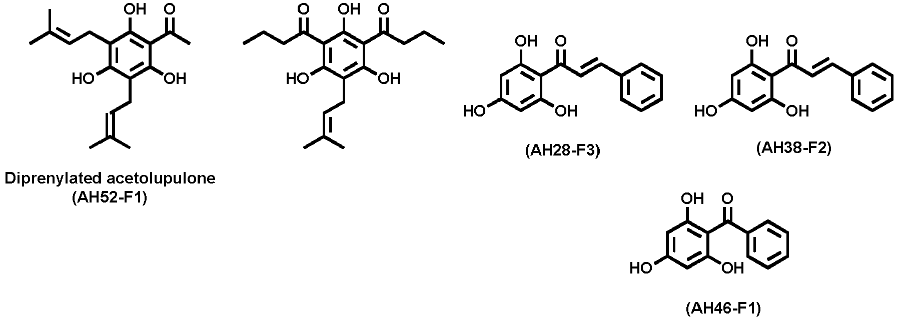

Advantageously, the compound according to the invention may be chosen from the compounds of formula (I), wherein A and B are O: 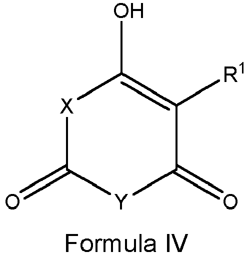 wherein R¹, X and Y are defined as above (X is C(R⁴)₂ and Y is C(R²)₂). The compound according to the invention may be chosen from the compounds of formula (IV), wherein R³ and R⁵ are prenyl.

Advantageously, the compound according to the invention may be chosen from the following compounds:

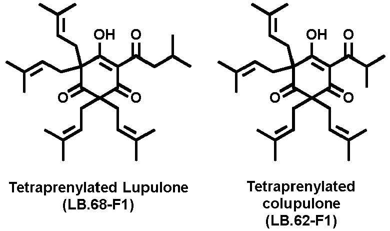

Advantageously, the compound according to the invention may be chosen from the compounds of formula (I), belonging to the class of prenylated acylphloroglucinols, commonly referred to the beta-acids series.

As meant herein, "prenylated acylphloroglucinols" or "beta-acids" are compounds of formula (I), wherein R² and R⁵ are prenyl groups. Beta-acids thus bear a total of three prenyl groups. A is O and B is OH or OR⁶. Preferably, the compound according to the invention may be chosen from the beta-acids compounds of formula (V): 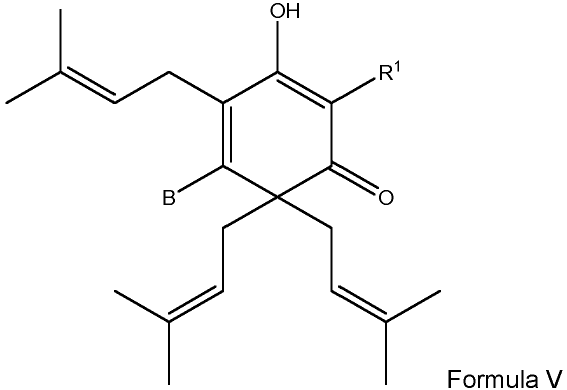 wherein B is OH or OR⁶, R¹ and R⁶ being defined as above.

Advantageously, the compound according to the invention may be chosen from the following compounds:

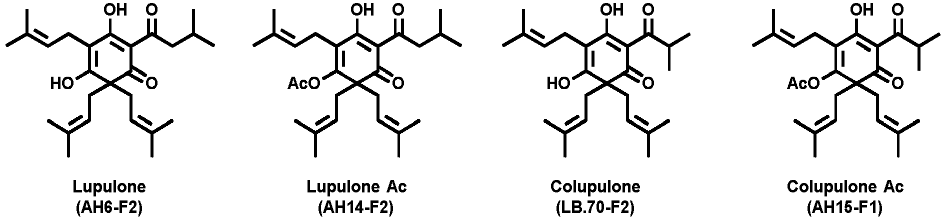

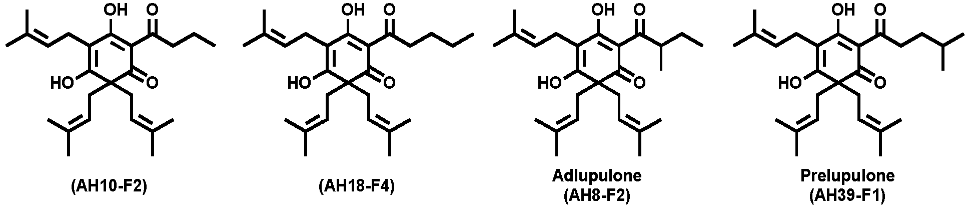

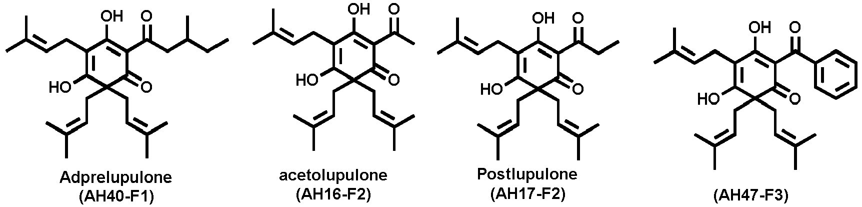

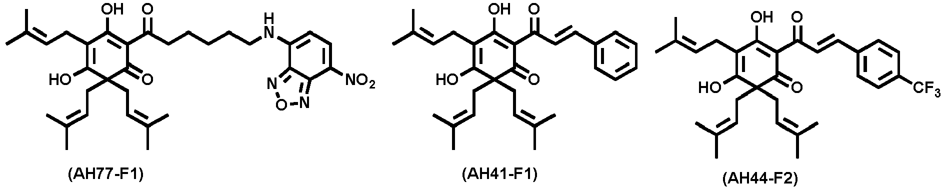

Advantageously, the compound according to the invention may be chosen from the compounds of formula (I), belonging to the class of geranylated beta-acids
As meant herein, "geranylated beta-acids" are "beta-acids" according to the invention, wherein R² and R⁵ are geranyl groups, instead of prenyl groups. Geranylated beta-acids thus bear a total of three geranyl groups. A is O and B is OH or OR⁶. Preferably, the compound according to the invention may be chosen from the geranylated beta-acids compounds of formula (VI): 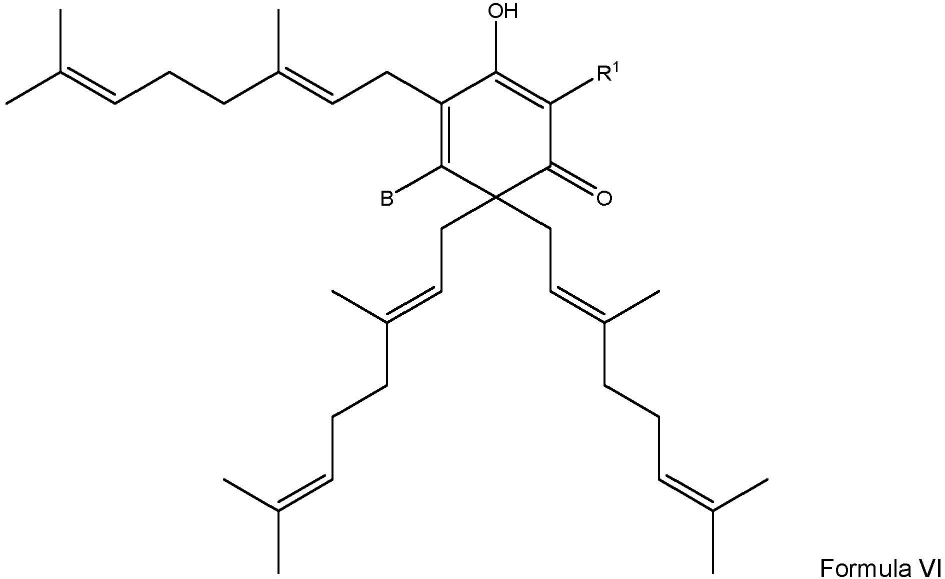 wherein B is OH or OR⁶, R¹ and R⁶ being defined as above.

Advantageously, the compound according to the invention may be chosen from the following compounds:

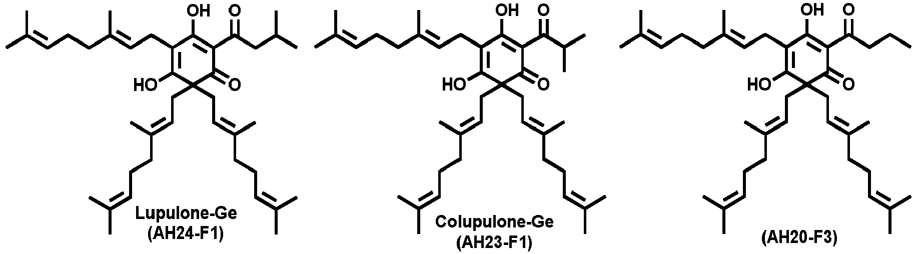

Advantageously, the compound according to the invention may be chosen from the compounds of formula (I), belonging to the class of tetraprenylated beta-acids.

As meant herein, "tetraprenylated beta-acids" are compounds of formula (I), wherein R² and R⁴ are prenyl groups. Tetraprenylated beta-acids thus bear a total of four prenyl groups. A is O and B is O. Preferably, the compound according to the invention may be chosen from the beta-acids compounds of formula (VII): 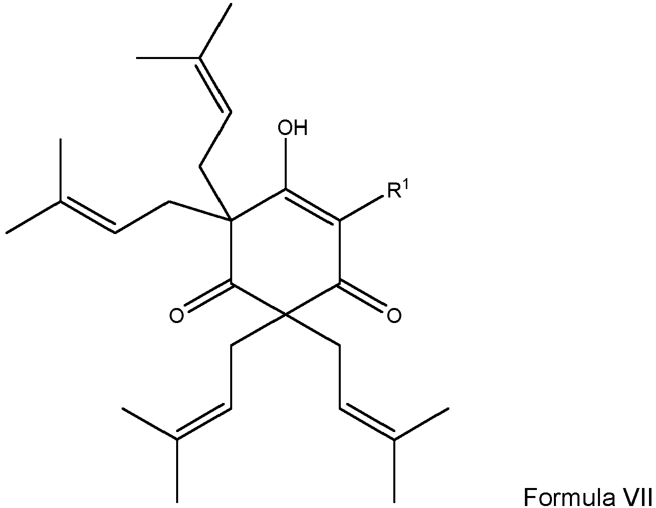 wherein R¹ is defined as above.

Advantageously, the compound of formula VII may be in the form of a mixture of tautomers depending on the solvent conditions. For example, the tautomerization is possible in chloroform, but there is no tautomerization in alcohol, water or DMSO. Advantageously, the compound according to the invention may be chosen from the following compounds:

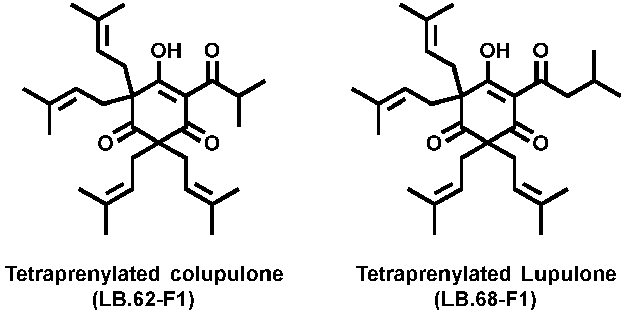

Advantageously, the compound according to the invention may be chosen from the compounds of formula (I), belonging to the class of diprenylated beta-acids.

As meant herein, "diprenylated beta-acids" are compounds of formula (I), wherein R³ and R⁵ are prenyl groups. Diprenylated beta-acids thus bear a total of two prenyl groups. A is OH and B is OH or OR⁶. Preferably, the compound according to the invention may be chosen from the beta-acids compounds of formula (VIII): 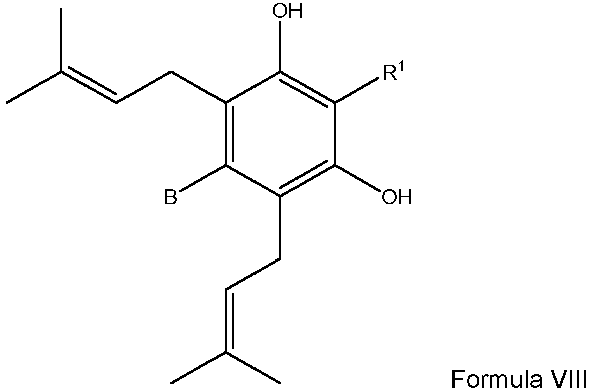 wherein B is OH or OR⁶, R¹ and R⁶ being defined as above.

Advantageously, the compound according to the invention may be chosen from the following compounds:

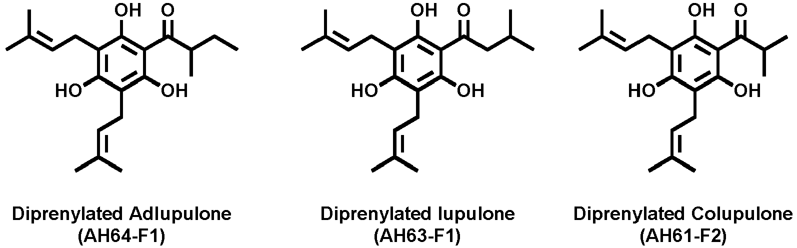

Advantageously, the compound according to the invention may be chosen from the compounds of formula (I), belonging to the class of gem-diprenylated beta-acids.

As meant herein, "gem-diprenylated beta-acids" are compounds of formula (I), wherein R⁵ is H or a substituted or unsubstituted, saturated or unsaturated, linear or branched, acyl group comprising 10 or fewer carbon atoms, optionally comprising an aryl moiety, and each R² are prenyl groups. Gem-diprenylated beta-acids thus bear a total of two germinal prenyl groups. A is O and B is OH or OR⁶. Preferably, the compound according to the invention may be chosen from the beta-acids compounds of formula (IX): 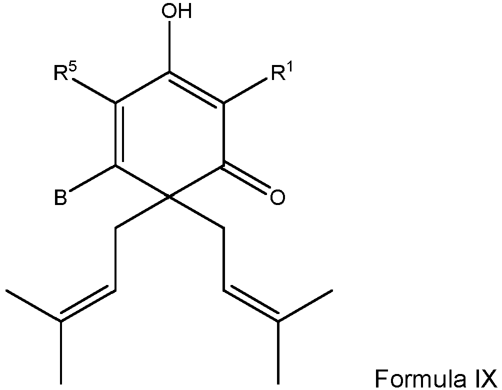 wherein B is OH or OR⁶, R¹, R⁵ and R⁶ being as defined above.

Advantageously, the compound of formula IX may be in the form of a mixture of tautomers depending on the solvent conditions. For example, the tautomerization is possible in chloroform, but there is no tautomerization in alcohol, water or DMSO. Advantageously, the compound according to the invention may be chosen from the following compounds:

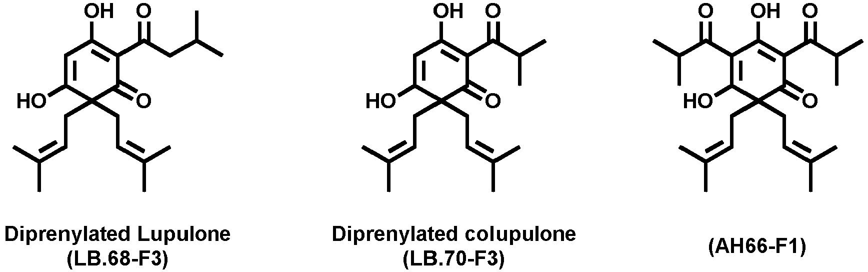

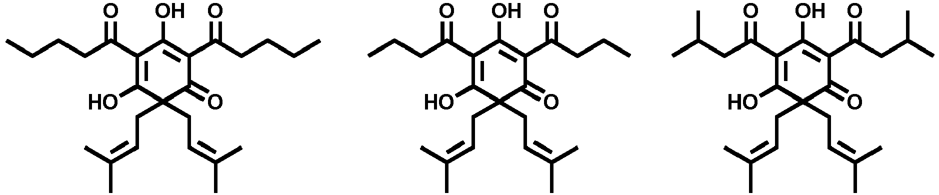

Advantageously, the compound according to the invention may be chosen from the compounds of formula (I), belonging to the class of monoprenylated beta-acids.

As meant herein, "monoprenylated beta-acids" are compounds of formula (I), wherein only R³ is a prenyl group. Monoprenylated beta-acids thus bear only one prenyl group. A is O or OH and B is OH or OR⁶. Preferably, the compound according to the invention may be chosen from the beta-acids compounds of formula (X): 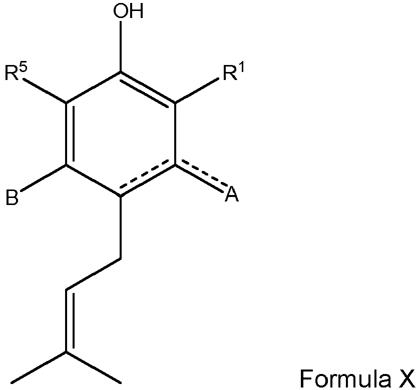 wherein A is O or OH and B is OH or OR⁶, R⁵ is H or a substituted or unsubstituted, saturated or unsaturated, linear or branched, acyl group comprising 10 or fewer carbon atoms, optionally comprising an aryl moiety and R⁶ is defined as above.

Advantageously, when A is O, the compound of formula X may be in the form of a mixture of tautomers depending on the solvent conditions. For example, the tautomerization is possible in chloroform, but there is no tautomerization in alcohol, water or DMSO.

Advantageously, the compound according to the invention may be chosen from the following compounds:

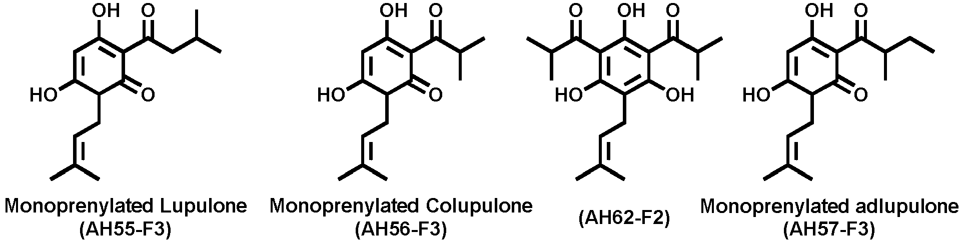

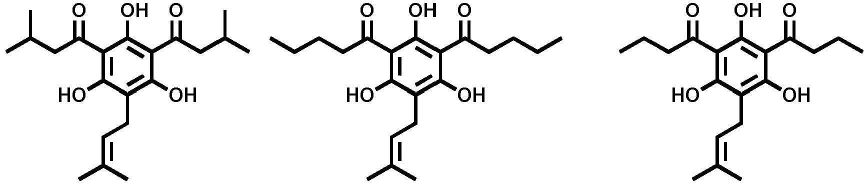

On another aspect, the invention also relates to the compounds of formula I, II, III, IV, V, VI, VII, VIII, IX and X as described above. The compounds of formula I, II, III, IV, V, VI, VII, VIII, IX and X as described above may be natural compounds or synthetic compounds. It is meant by synthetic that they may not be found in natural hop.

Advantageously, the invention relates to the synthetic compounds of formula I, II, III, IV, V, VI, VII, VIII, IX and X, as described above.

Advantageously, the invention relates to the compounds of formula (VIII-2): 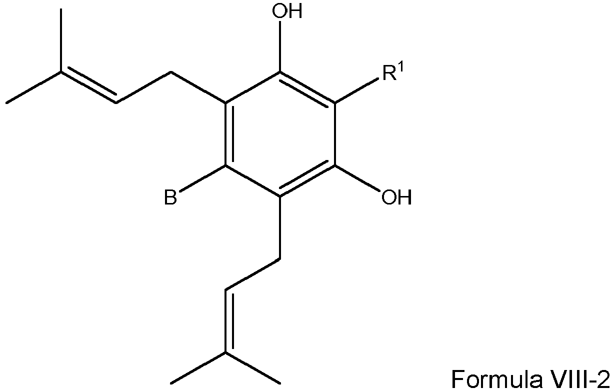 wherein:
- B is OH or OR⁶, R⁶ being defined as above, and
- R¹ is a substituted or unsubstituted, saturated or unsaturated, linear or branched, acyl group comprising 10 or fewer carbon atoms and comprising an aryl moiety, preferably R¹ is chosen from the following structures:

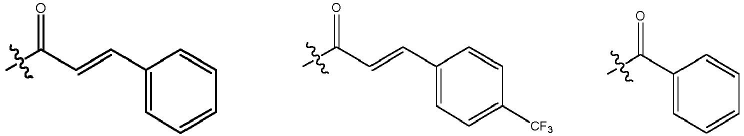

Advantageously, the invention relates to the compounds of formula (VIII-3): 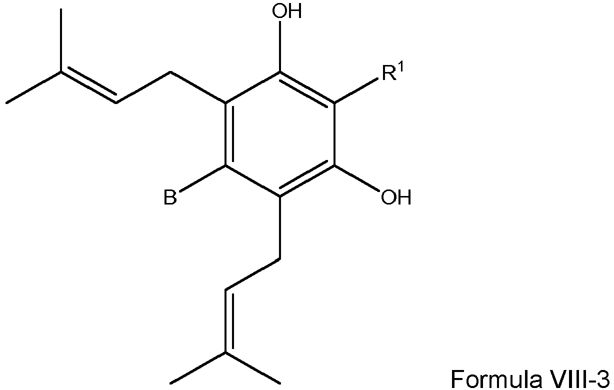 wherein:
- B is OH or OR⁶, R⁶ being defined as above, and
- R¹ is a substituted or unsubstituted, saturated or unsaturated, linear or branched, acyl group comprising 10 or fewer carbon atoms substituted by a fluorescent moiety, preferably R¹ is the following structure:

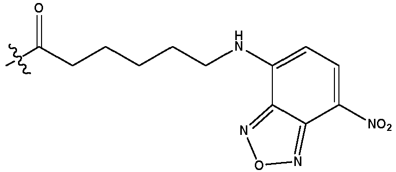

Advantageously, the invention relates to the compounds of formula (IX-2): 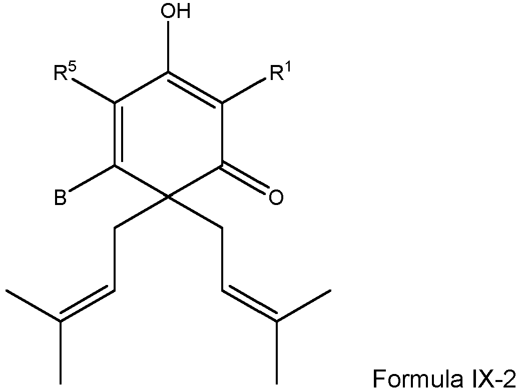 wherein B is OH or OR⁶, R¹, R⁵ and R⁶ being as defined above. Preferably, R¹ and R⁵ are branched acyl groups comprising 4 or fewer carbon atoms, or linear acyl group comprising 10 or fewer carbon atoms.

Advantageously, the invention relates to the compounds of formula (X): 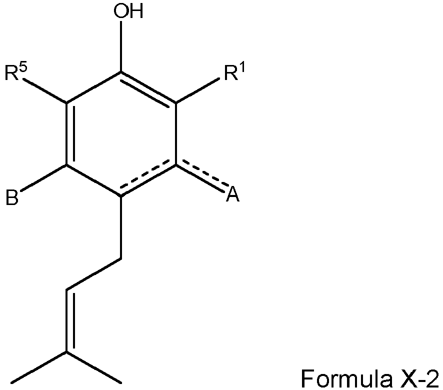 wherein A is O or OH and B is OH or OR⁶, R⁵ is H or a substituted or unsubstituted, saturated or unsaturated, linear or branched, acyl group comprising 10 or fewer carbon atoms, optionally comprising an aryl moiety and R⁶ is defined as above. Preferably, R¹ and R⁵ are branched acyl groups comprising 4 or fewer carbon atoms, or linear acyl group comprising 10 or fewer carbon atoms.

Advantageously, the invention relates to the compounds of formula (VI-2): 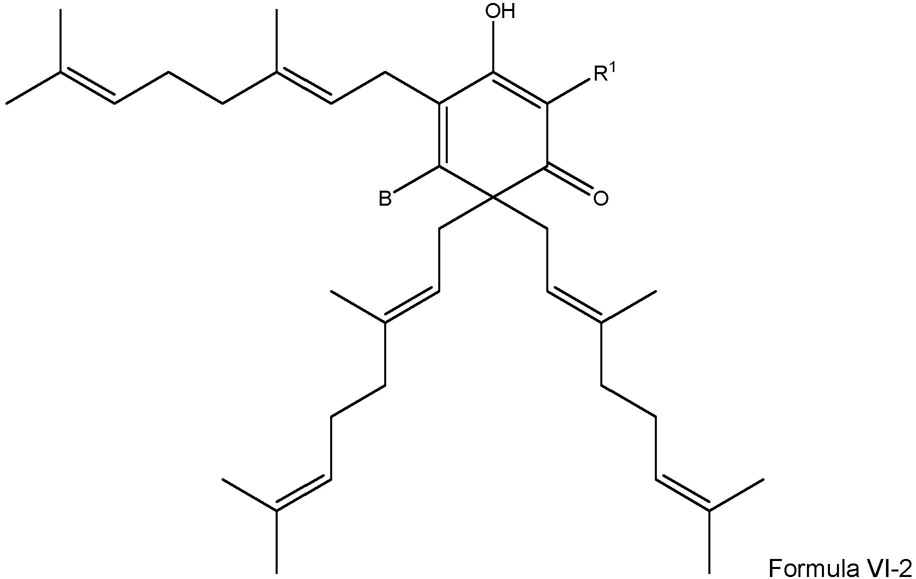 wherein B is OH or OR⁶, R¹ and R⁶ being defined as above. Preferably, R¹ is a substituted or unsubstituted, saturated or unsaturated, linear or branched, acyl group comprising 4 or fewer carbon atoms, optionally comprising an aryl moiety.

### Brief description of the figures

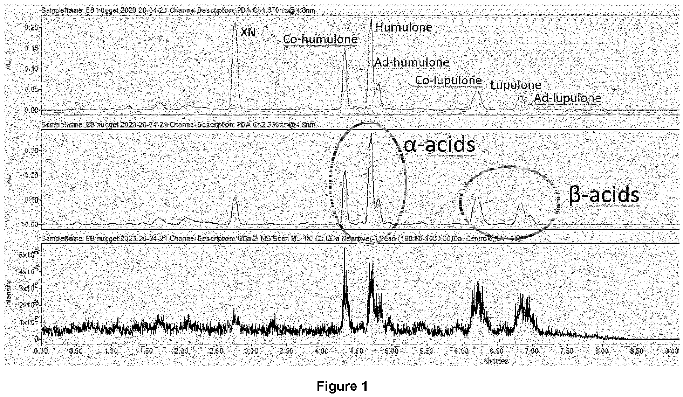

Figure 1. Crude hydro-ethanolic (ethanol/water 90:10) extract of hop cones (Nugget cultivar) from solid/liquid extraction (Nugget cultivar). Chromatograms at 370 nm and at 330 nm of the hop cones crude extract, as well as Total Ion Chromatogram (TIC) in negative mode. Polar phenolic compounds (between t_{R} 0 to 2.5 min), XN = xanthohumol (t_{R} = 2.8 min), alpha-acids (co-humulone, humulone, ad-humulone) between t_{R} 4 to 5 min, beta-acids (co-lupulone, lupulone, ad-lupulone) between t_{R} 6 to 7 min.

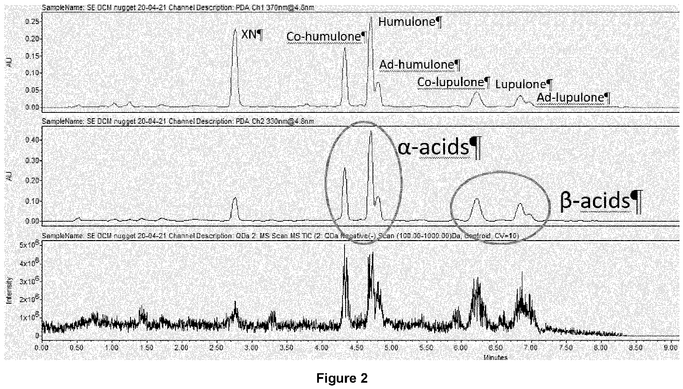

**Figure 2****.** DCM sub-extract of hop cones (Nugget cultivar) from liquid/liquid extraction (slight concentration in prenylated phenolic compounds and elimination of polar compounds). Chromatograms at 370 nm and at 330 nm of the hop cones DCM sub-crude extract, as well as Total Ion Chromatogram (TIC) in negative mode. XN = xanthohumol (t_{R} = 2.8 min), alpha-acids (co-humulone, humulone, ad-humulone) between t_{R} 4 to 5 min, beta-acids (co-lupulone, lupulone, ad-lupulone) between t_{R} 6 to 7 min.

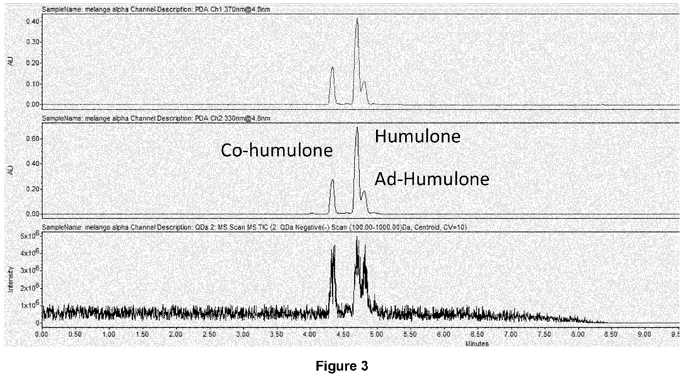

**Figure 3****.** Alpha acid fraction from Nugget cultivar obtained by preparative chromatography (CPC, HPLC). Chromatograms at 370 nm and at 330 nm of the alpha acid fraction, as well as Total Ion Chromatogram (TIC) in negative mode. alpha-acids (co-humulone, humulone, ad-humulone) between t_{R} 4 to 5 min.

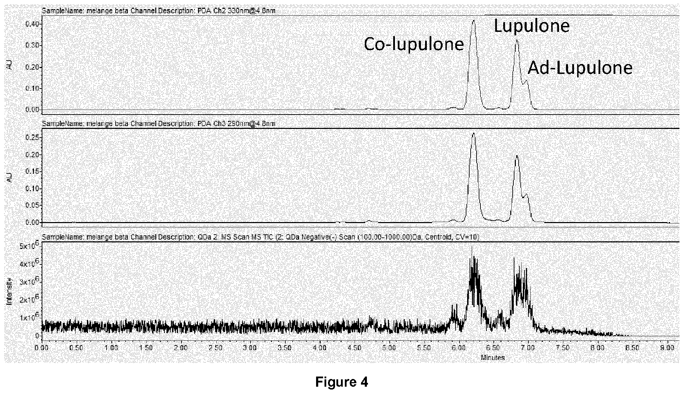

**Figure 4****.** Beta-acid fraction from Nugget cultivar obtained by preparative chromatography (CPC, HPLC). Chromatograms at 370 nm and at 330 nm of the beta-acid fraction, as well as Total Ion Chromatogram (TIC) in negative mode. Beta-acids (co-lupulone, lupulone, ad-lupulone) between t_{R} 6 to 7 min.

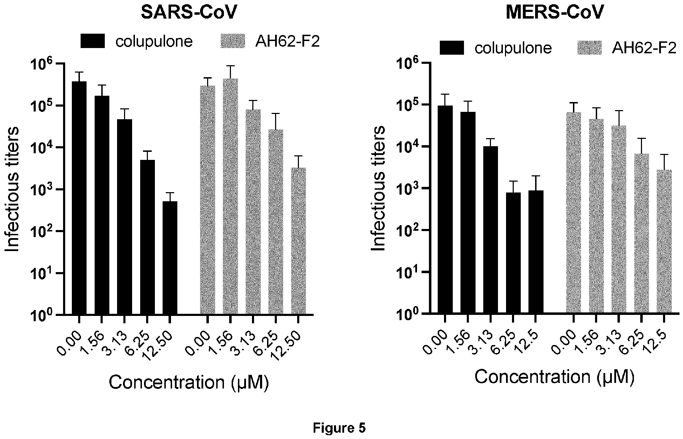

**Figure 5****.** Antiviral activity of colupulone and AH62-F2 on SARS-CoV and MERS-CoV in cell culture. Calu3 cells were inoculated with SARS-CoV and MERS-CoV in the presence of 1.56, 3.13, 6.25 or 12.5 µM of colupulone or AH62-F2 for 1h. The inoculum was removed and replaced with culture medium containing the compounds at the same concentrations for 15 h. Supernatants were collected and viral titers were determined by TCID50. Data are expressed as mean +/- SEM of 3 independent experiments performed in duplicates.

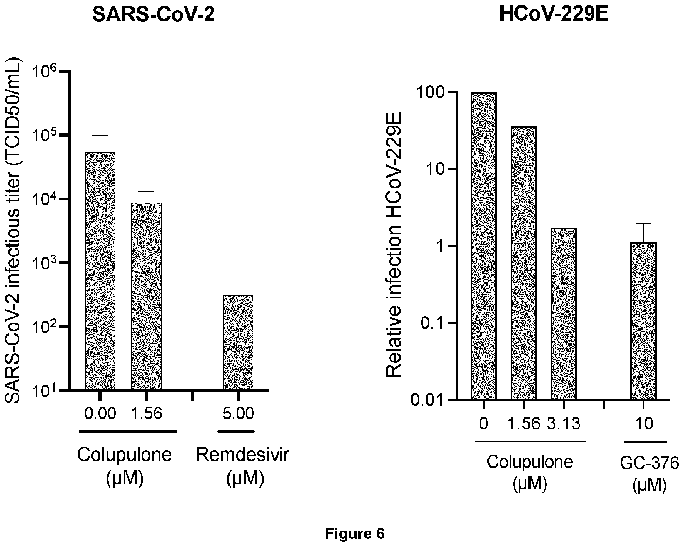

**Figure 6****. Antiviral efficacy of colupulone in human primary nasal epithelial cells.** Mucilair^{™} cells were inoculated with SARS-CoV-2 or HCoV-229E for 1 h at the apical side. Inoculum was removed. Colupulone at 1.56 µM for SARS-CoV-2, or 1.56 and 3.13 µM for HCoV-229E, 5 µM remdesivir, or 10 µM GC-376, were added in the basolateral medium. For SARS-CoV-2, assay, 42 h post-inoculation, viruses were collected from the apical surface and viral titers were determined by infectivity titrations. Data represent one experiment performed in duplicate. For HCoV-229E, 24h post-inoculation, cells were lysed and luciferase activity quantified. Data are expressed relative to the control DMSO. Results are expressed as mean ± SEM of 2 experiments.

### Examples

### Example 1 (General Information for Extraction, Separation and Characterization)

### General Experimental Procedures for Extraction and Purification of Alpha and Beta-Acids

For extraction and fractionation, synthesis grade ethanol (EtOH) and dichloromethane (DCM) were furnished by VWR Prolabo^{®} (Fontenay-sous-Bois, France). Water was bidistilled. All organic solvents for Centrifugal Partition Chromatography (CPC) purification were High Pressure Liquid Chromatography (HPLC) grade except for the n-heptane which was synthesis grade (Carlo Erba Reagents^{®}, Val-de-Reuil, France). Ethyl acetate (EtOAc) and methanol (MeOH) were purchased from Fisher Chemicals^{®} (Illkirch, France). Water was purified using Millipore Integral 5 (France) water purification system with a resistivity of not less than 18 MΩ.cm⁻¹. For analyses, acetonitrile (LC-MS grade) was purchased in Carlo Erba Reagents^{®}, whereas methanol (LC-MS grade) came from Fischer Chemical^{®}. The chloroform-d6 (CDCl₃) and methanol-d4 (CDsOD) for Nuclear Magnetic Resonance (NMR) experiments were obtained from Euriso-Top (Gif-sur-Yvette, France).

Ultra-High Performance Liquid Chromatography (UHPLC) analyses were carried out using an Acquity UPLC^{®} H-Class Waters^{®} system (Waters, Guyancourt, France) equipped with a diode array detector (DAD) and an Acquity QDa ESI-Quadrupole Mass Spectrometer. The software used was Empower 3. The stationary phase was a Waters^{®} Acquity BEH C18 column (2,1 × 50 mm, 1,7 µm) connected to a 0.2 µm in-line filter. Preparative HPLC was performed using a Shimadzu^{®} HPLC system equipped with a LC-20AP binary high-pressure pump, a CBM-20A controller, and a SPD-M20A diode array detector. The software used was LabSolution. The stationary phase was a VisionHT HL C18 (5 µm, 250 × 22 mm) column (Grace^{®}, France). CPC was performed using an Armen instrument 1 L rotor (SCPC-1000-L) provided by Gilson^{®} (Saint-Avé, France). CPC analyses were monitored using Shimadzu^{®} pump and detector. Nuclear Magnetic Resonance (NMR) spectra were recorded on a Bruker^{®} DPX-500 spectrometer. Acylphloroglucinol derivatives were solubilized in deuterated chloroform (CDCl₃). For beta-acids, NMR was also performed in CDsOD at 0°C.

### Example 2 (Extraction)

### Crude Hydro-Ethanolic Extract and DCM Sub-Extract

Female hop plants (*Humulus lupulus* L., cultivar 'Nugget') were harvested at maturity stage at the Beck farm (Bailleul, Northern France), at the time of harvesting of hop by producers in September 2019 and 2020. Voucher specimens were kept at the Faculty of pharmacy in Lille (UMRt BioEcoAgro) under reference NugBeck2019 and NugBeck2020. After drying for 10 days at room temperature, protected from light, cones were powdered with a blender. Crude hydro-alcoholic extracts of cones were obtained after an ethanol/water (9:1; v/v; 15 mL/g) mixture-based extraction with three successive macerations of four hours and one overnight, stirring in the dark. The percentage yield (PY) on a dry weight basis of cones crude hydro-ethanolic extract was 35.5%. The crude extract of female cones was fractionated by a liquid-liquid separation using dichloromethane (DCM) and water to obtain two sub-extracts. The corresponding sub-extracts (DCM sub-extract and aqueous sub-extract) were obtained with percentages yields of 52% and 48% respectively (Figure 1 and Figure 2).

### Example 3 (Fractionation)

### Alpha and Beta-Acids Fractions (Antiviral activities in Table 4)

Alpha acids (humulone, cohumulone, adhumulone) and beta-acids (lupulone, colupulone and adlupulone) were purified from the DCM sub-extract of cones in several steps. A first fractionation was performed by CPC. Using the Arizona solvent system P: n-heptane/EtOAc/MeOH/water (6:5:6:5; v/v), the rotor was entirely filled at 50 mL/min with the aqueous stationary phase in the ascending mode with rotation (500 rpm). Then, the rotation speed was increased from 500 to 1200 rpm. The organic mobile phase was pumped into the column in ascending mode at a flow rate of 30 mL/min. DCM sub-extract (6 g), initially dissolved in 40 mL of the organic/aqueous phase mixture (1:1), was filtered on a Büchner funnel and was injected immediately after the displacement of stationary phase (approximatively 190 mL). Fractions of 30 mL were collected every min. The CPC was run in ascending mode for 45 min and then switched to descending mode (the organic phase becomes the stationary phase) for 22 additional minutes at 30 mL/min with the same rotor speed (1200 rpm). The content of the outgoing organic phase and then aqueous phase was monitored by online UV absorbance measurement at 254 nm, 330 nm and 370 nm. According to the chromatograms and previous TLC developed with toluene/ethyl acetate/formic acid (73:18:9; v/v), 5 sub-fractions (MC1 to MC5) from ascending mode and 3 sub-fractions (MC6 to MC8) from descending mode were put together. Acylphloroglucinol derivatives (alpha- and beta-acids) were purified, with purity greater than 95%, from sub-fractions MC1 and MC2 using preparative HPLC. The mobile phase was composed of water (solvent A) and acetonitrile (solvent B). The following proportions of solvent B were: 75-100% (0-20 min) and 100% (20-25 min) at 15 mL/min. Injections with 500 µL of a 100 mg/mL fraction solution in methanol were performed. By this process, alpha acid fraction (Figure 3) and beta-acid fraction (Figure 4) were first obtained.

### Example 4 (Purification of Main Alpha- and Beta-Acids and Structural Elucidation)

In a second time, each main compound (humulone cohumulone, adhumulone, lupulone, colupulone and adlupulone) were purified independently by preparative HPLC with the same method than described previously in example 2 (antiviral activities in Table 2 and Table 3). Their degree of purity was determined according to the chromatogram obtained with PDA MaxPlot. Structures of purified compounds were determined using NMR and HR-MS, as well as by comparison of retention times with reference compounds purified previously in the lab. Mono- (¹H and ¹³C) and bidimensional (COSY, HSQC, HMBC) spectra were carried out for each compound.

### Co-humulone (C₂₀H₂₈O₅, MW = 348.44 g/mol)

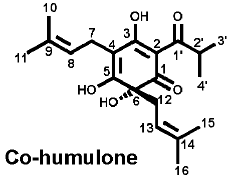

**¹H NMR** (500 MHz, CDsOD at 0°C) δ 5.15 (C**H**, *t, J* = 7.2, 7.2 Hz, H-8), 4.97 (C**H**, *t, J* = 7.9, 7.9 Hz, H-13), 3.82 (C**H**, *m,* H-2'), 3.12 (C**H₂ₐ,** *m,* H-7a), 3.03 (C**H_{2b},** *m,* H-7b), 2.54 (C**H₂,** *d, J* = 7.9 Hz, H-12), 2.17 (C**H**, *m,* H-3'), 1.75 (C**H₃,** *s,* H-10), 1.69 (C**H₃,** s, H-11), 1.63 (C**H₃,** *s,* H-15), 1.53 (C**H₃,** *s,* H-16), 1.17 (C**H₃,** *d, J* = 6.8 Hz, H-3'), 1.11 (C**H₃,** *d, J* = 6.8 Hz, H-4'). **¹³C NMR** (500 MHz, CDsOD at 0°C) δ 204.90 (C-1'), 195.96 (C-1), 190.06 (C-3), 172.04 (C-5), 136.10 (C-14), 130.88 (C-9), 121.78 (C-8), 116.31 (C-13), 108.26 (C-2), 104.77 (C-4), 78.33 (C-6), 40.84 (C-12), 34.67 (C-2'), 24.79 (C-15), 24.67 (C-11), 20.50 (C-7), 18.64 (C-3'), 17.50 (C-4'), 16.52 (C-16), 16.49 (C-10)
**Degree of purity** of co-humulone according to the chromatogram obtained with PDA MaxPlot: **95%**
**MS (ESI-)** [M - H]⁻ *m*/*z* 347.23
**UV spectrum:** 237.8, 284.1, 322.3 nm

### Humulone (C₂₁H₃₀O₅, MW = 362.47 g/mol)

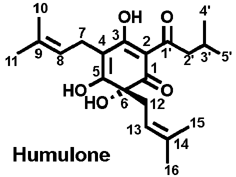

**¹H NMR** (500 MHz, CDsOD at 0°C) δ 5.15 (C**H**, *t, J* = 7.2, 7.2 Hz, H-8), 4.94 (C**H**, *t, J* = 7.9, 7.9 Hz, H-13), 3.11 (C**H₂ₐ,** *m,* H-7a), 3.02 (C**H_{2b},** *m,* H-7b), 2.83 (C**H₂ₐ,** *m,* H-2a'), 2.75 (C**H_{2b},** *m,* H-2'b), 2.54 (C**H₂,** *m,* H-12), 2.13 (C**H**, *m,* H-3'), 1.75 (C**H₃,** *s,* H-10), 1.69 (C**H₃,** *s,* H-11), 1.63 (C**H₃,** *s,* H-15), 1.53 (C**H₃,** *s,* H-16), 1.00 (C**H₃,** *d, J* = 6.8 Hz, H-4'), 0.98 (C**H₃,** *d, J* = 6.8 Hz, H-5'). **¹³C NMR** (500 MHz, CDsOD at 0°C) δ 200.05 (C-1'), 196.16 (C-1), 189.92 (C-3), 171.97 (C-5), 136.15 (C-14), 130.86 (C-9), 121.79 (C-8), 116.23 (C-13), 108.50 (C-4), 106.21 (C-2), 78.09 (C-6), 47.13 (C-2'), 40.80 (C-12), 25.90 (C-3'), 24.78 (C-15), 24.68 (C-11), 21.77 (C-4'), 21.68 (C-5'), 20.51 (C-7), 16.52 (C-16), 16.50 (C-10)
**Degree of purity** of humulone according to the chromatogram obtained with PDA MaxPlot: **95%**
**MS (ESI-)** [M - H]⁻ *m*/*z* 361.31
**UV spectrum:** 237.8, 284.1, 322.3 nm

### Ad-humulone (C₂₁H₃₀O₅, MW = 362.47 g/mol)

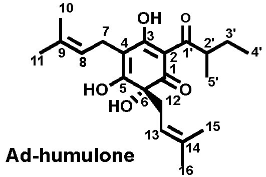

**¹H NMR** (500 MHz, CDsOD at 0°C) δ 5.14 (C**H**, *t, J* = 7.2, 7.2 Hz, H-8), 4.95 (C**H**, *t, J* = 7.9, 7.9 Hz, H-13), 3.73 (C**H**, *m,* H-2'), 3.12 (C**H₂ₐ,** *m,* H-7a), 3.02 (C**H_{2b},** *m,* H-7b), 2.53 (C**H₂,** *m,* H-12), 1.79 (C**H**_{**2**a}, *m,* H-3'a), 1.75 (C**H₃,** *s,* H-10), 1.69 (C**H₃,** *s,* H-11), 1.63 (C**H₃,** *s,* H-15), 1.52 (C**H₃,** *s,* H-16), 1.41 (C**H_{2b},** *m,* H-3'b), 1.08 (C**H₃,** *d, J* = 6.8 Hz, H-5'), 0.97 (C**H₃,** *t, J* = 7.4, 7.4 Hz H-4'). **¹³C-NMR** (500 MHz, CDsOD at 0°C) 204.4 (C-1'), 196.0 (C-1), 190.4 (C-3), 171.4 (C-5), 136.1 (C-14), 130.9 (C-9), 121.7 (C-8), 116.3 (C-13), 108.5 (C-4), 105.4 (C-2), 78.2 (C-6), 40.9 (C-2'), 40.8 (C-12), 26.9 (C-3'), 24.8 (C-15), 24.7 (C-11), 20.5 (C-7), 16.5 (C-16), 16.5 (C-10), 15.0 (C-5'), 10.8 (C-4')
**Degree of purity** of ad-humulone according to the chromatogram obtained with PDA MaxPlot: **98%**
**MS (ESI-)** [M - H]⁻ *m*/*z* 361.31
**UV spectrum:** 286.4, 318.7 nm

### Co-lupulone (C₂₅H₃₆O₄, MW = 400.56 g/mol)

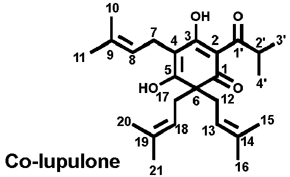

**¹H NMR** (500 MHz, CDCl₃) *δ* 7.0 (O**H**), 6.47 (O**H**), 5.17 (C**H**, *t, J* = 7.2, 7.5 Hz, H-8), 4.84 (C**H**, *t, J* = 7.7, 7.7 Hz, H-13), 4.79 (C**H**, *t, J* = 7.7, 7.7 Hz, H-18), 4.23 (C**H**, *m,* H-2', minor tautomer), 4.05 (C**H**, *m,* H-2'), 3.22 (C**H₂,** *dd, J* = 7.2, 7.5 Hz, H-7), 2.71 (C**H₂,** *m,* H-12a), 2.68 (C**H₂,** *m,* H-17a), 2.65 (C**H₂,** *m,* H-12b), 2.53 (C**H₂,** *m,* H-17b), 2.15 (C**H**, *m,* H-2'), 1.82 (C**H₃,** *s,* H-10), 1.81 (C**H₃,** *s,* H-11), 1.61 (C**H₃,** *s,* H-20), 1.59 (C**H₃,** *s,* H-15), 1.59 (C**H₃,** *s,* H-21), 1.57 (C**H₃,** *s,* H-16), 1.16 (C**H₃,** *s,* H-3'), 1.14 (C**H₃,** *s,* H-4'). **¹³C NMR** (500 MHz, CDCl₃): 207.1 (C-1'), 195.4 (C-1), 189.7 (C-3), 172.5 (C-5), 137.4 (C-9), 135.4 (C-14), 134.9 (C-19), 120.9 (C-8), 118.2 (C-13), 117.7 (C-18), 109.8 (C-4), 107.3 (C-2), 57.1 (C-6), 37.6 (C-17), 36.4 (C-12), 35.4 (C-2'), 25.9 (C-20), 25.9 (C-15), 25.9 (C-10), 21.1 (C-7), 18.9 (C-3'), 18.8 (C-4'), 17.9 (C-21), 17.9 (C-16), 17.8 (C-11)
**¹H NMR** (500 MHz, CDsOD at 0°C) *δ* 5.03 (C**H**, *t, J* = 6.9, 6.9 Hz, H-8), 4.77 (C**H**, *t, J* = 7.4, 6.9 Hz, H-13 et H-18), 3.99 (C**H₂,** *m,* H-2'), 3.11 (C**H₂,** *d, J* = 6.9 Hz, H-7), 2.61 (C**H₂,** *m,* H-12 et H-17), 1.70 (C**H₃,** *s,* H-10), 1.69 (C**H₃,** *s,* H-11), 1.60 (C**H₃,** *s,* H-21 et H-16), 1.57 (C**H₃,** *s,* H-20 et H-15), 1.11 (C**H₃,** *s,* H-5'), 1.10 (C**H₃,** *s,* H-4'). **¹³C NMR** (500 MHz, CDCl₃): 207.6 (C-1'), 196.6 (C-1), 189.6 (C-3), 173.4 (C-5), 134.3 (C-9), 131.1 (C-14), 131.1 (C-19), 121.9 (C-8), 117.8 (C-13), 117.8 (C-18), 110.7 (C-4), 106.6 (C-2), 57.6 (C-6), 37.5 (C-17), 37.5 (C-12), 35.9 (C-2'), 24.7 (C-20), 24.7 (C-15), 24.7 (C-10), 20.2 (C-7), 17.8 (C-3'), 17.8 (C-4'), 17.8 (C-10), 16.7 (C-21), 16.6 (C-16)
**Degree of purity** of co-lupulone according to the chromatogram obtained with PDA MaxPlot: 99%
**MS (ESI-)** [M - H]⁻ *m*/*z* 399.38
**UV spectrum:** 275.7, 334.2 nm

### Tautomerism of Co-lupulone in CDCl₃

In CDCl₃, beta-acids such as co-lupulone can exist under two states due to tautomerization (prototropy) triggered by the solvent composition. Their relative proportions could be determined by integration of the protons in 2' position by ¹H NMR. In other solvents such as CDsOD or DMSO-*d₆*, a single tautomer (major) dominates in solution.

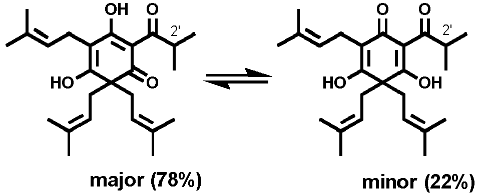

### Lupulone (C₂₆H₃₈0₄, MM = 414.59 g/mol)

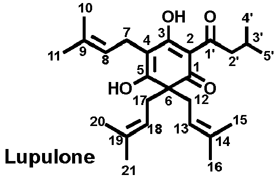

**¹H NMR** (500 MHz, CDCl₃) *δ* 6.99 (O**H**), 6.47 (O**H**), 5.17 (C**H**, *t, J* = 7.2, 7.5 Hz, H-8), 4.82 (C**H**, *t, J* = 7.7, 7.7 Hz, H-13), 4.79 (C**H**, *t, J* = 7.7, 7.7 Hz, H-18), 3.22 (C**H₂,** *dd, J* = 7.2, 7.5 Hz, H-7), 3.06 (C**H₂,** *d, J* = 6.9 Hz, H-2', minor tautomer), 2.94 (C**H₂,** *d, J =* 6.9 Hz, H-2'), 2.71 (C**H₂,** *m,* H-12a), 2.68 (C**H₂,** *m,* H-17a), 2.63 (C**H₂,** *m,* H-12b), 2.53 (C**H₂,** *m,* H-17b), 2.15 (C**H**, *m,* H-3'), 1.82 (C**H₃,** *s,* H-10), 1.81 (C**H₃,** *s,* H-11), 1.60 (C**H₃,** *s,* H-20), 1.59 (C**H₃,** *s,* H-15), 1.58 (C**H₃,** *s,* H-21), 1.58 (C**H₃,** *s,* H-16), 0.99 (C**H₃,** *s,* H-5'), 0.98 (C**H₃,** *s,* H-4'). **¹³C NMR** (500 MHz, CDCl₃) *δ* 202.1 (C-1'), 195.7 (C-1), 189.7 (C-3), 172.6 (C-5), 137.4 (C-9), 135.3 (C-14), 135.0 (C-19), 120.9 (C-8), 118.2 (C-13), 117.7 (C-18), 110.0 (C-4), 108.7 (C-2), 57.0 (C-6), 48.0 (C-2'), 37.6 (C-17), 36.4 (C-12), 26.0 (C-3'), 25.9 (C-20), 25.9 (C-15), 25.8 (C-10), 22.7 (C-5'), 22.6 (C-4'), 21.1 (C-7), 17.9 (C-21), 17.9 (C-16), 17.8 (C-11)
**¹H NMR** (500 MHz, CDsOD at 0°C) *δ* 5.02 (C**H**, *t, J* = 6.9, 6.9 Hz, H-8), 4.77 (C**H**, *t, J* = 7.4, 6.9 Hz, H-13 et H-18), 3.11 (C**H₂,** *d, J* = 6.9 Hz, H-7), 2.85 (C**H₂,** *d, J* = 6.9 Hz, H-2'), 2.61 (C**H₂,** *m,* H-12 et H-17), 2.08 (C**H**, *m,* H-3'), 1.76 (C**H₃,** *s,* H-10), 1.70 (C**H₃,** *s,* H-11), 1.60 (C**H₃,** *s,* H-21 et H-16), 1.57 (C**H₃,** *s,* H-20 et H-15), 0.98 (C**H₃,** *s,* H-5'), 0.97 (C**H₃,** *s,* H-4'). **¹³C NMR** (500 MHz, CDCl₃) *δ* 202.7 (C-1'), 196.9 (C-1), 189.4 (C-3), 173.7 (C-5), 134.3 (C-9), 131.1 (C-14), 131.1 (C-19), 121.9 (C-8), 117.8 (C-13), 117.8 (C-18), 110.8 (C-4), 107.7 (C-2), 57.4 (C-6), 48.9 (C-2'), 37.4 (C-17), 37.4 (C-12), 25.7 (C-3'), 24.7 (C-20), 24.7 (C-15), 24.7 (C-11), 21.8 (C-5'), 21.8 (C-4'), 20.2 (C-7), 16.7 (C-10), 16.6 (C-21), 16.6 (C-16)
**Degree of purity** of lupulone according to the chromatogram obtained with PDA MaxPlot: **99%**
**MS (ESI-)** [M - H]⁻ *m*/*z* 413.40
**UV spectrum:** 275.7, 331.8 nm

### Tautomerism of Lupulone in CDCl₃

In CDCl₃, beta-acids such as lupulone can exist under two states due to tautomerization (prototropy). Their relative proportions could be determined by integration of the protons in 2' position by ¹H NMR. In other solvents such as CDsOD or DMSO-*d₆*, a single tautomer (major) dominates in solution.

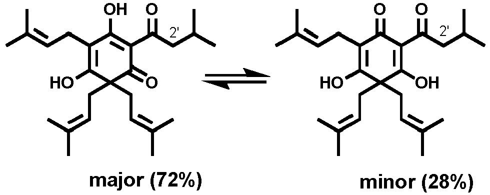

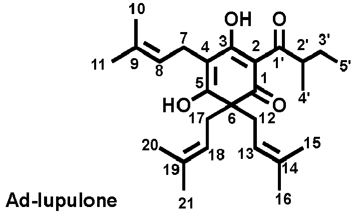

### Ad-lupulone (C₂₆H₃₈0₄, MM = 414.59 g/mol)

**¹H NMR** (500 MHz, CDsOD at 0°C) *δ* 5.04 (C**H**, *t, J* = 6.9, 6.9 Hz, H-8), 4.79 (C**H**, *t, J* = 7.4, 6.9 Hz, H-13 et H-18), 3.91 (C**H**, *m,* H-2'), 3.11 (C**H₂,** *d, J* = 6.9 Hz, H-7), 2.60 (C**H₂,** *m,* H-12 et H-17), 1.76 (C**H₃,** *s,* H-10), 1.73 (C**H₂ₐ,** *m,* H-3'a), 1.69 (C**H₃,** *s,* H-11), 1.59 (C**H₃,** *s,* H-21 et H-16), 1.57 (C**H₃,** *s,* H-20), 1.56 (C**H₃,** *s,* H-15), 1.35 (C**H_{2b},** *m,* H-3'b), 1.09 (C**H₃,** *d*, H-4'), 0.91 (C**H₃,** *t*, H-5'). **¹³C NMR** (500 MHz, CDCl₃) *δ* 206.7 (C-1'), 197.04 (C-1), 188.6 (C-3), 175.9 (C-5), 134.0 (C-9), 130.7 (C-14), 130.7 (C-19), 122.2 (C-8), 118.1 (C-13), 118.0 (C-18), 110.3 (C-4), 107.0 (C-2), 57.8 (C-6), 42.4 (C-2'), 37.7 (C-17), 37.4 (C-12), 26.3 (C-3'), 24.7 (C-20), 24.7 (C-15), 24.7 (C-11), 20.3 (C-7), 16.7 (C-21), 16.7 (C-16), 16.6 (C-10), 15.7 (C-4'), 11.1 (C-5')
**Degree of purity** of ad-lupulone according to the chromatogram obtained with PDA MaxPlot: **97%**
**MS (ESI-)** [M - H]⁻ *m*/*z* 413.44
**UV spectrum:** 276.9, 331.8 nm

### Example 5 (Wild Hop Extracts and Quantification)

### Preparation of Wild Hop Hydro-Ethanolic Extracts for Antiviral Assays (Table 5 for the Antiviral Activities)

Wild hop come from samples taken *in-situ* by BioEcoAgro team and preserved *ex-situ* in an experimental hop farm, as well as in the form of vitro-plants [2,3]. Cones were dried at low temperature, stored at -20°C under nitrogen atmosphere and then grounded in liquid nitrogen. Hydro-ethanolic extracts of hops, 4 wild (Aves 1, Hem 1, Douai 5, Aves 5) and 1 commercial (Nugget) hops were performed in 25 mL Erlenmeyer using 500 mg of dried cone powder with 10 mL of ethanol-water mixture (9:1, v/v). A 10-hours maceration session was carried out in on an orbital shaker. Afterwards, sample mixture was filtered by gravity on filter paper. Supernatant was then transferred into a tube. The exhausted matrix was then re-extracted two further times following the same protocol, and the supernatants were pooled with the first extract. At the end of the three maceration sessions, the solvent extraction was then evaporated in a Genevac^{™} centrifugal concentrator to obtain a crude hydro-ethanolic extract. Hydro-ethanolic extracts were then transferred in tared hemolysis tubes by dilution in methanol secondly evaporated using the Genevac^{™}.

### Quantification of Beta-Acids in Wild Hops and Commercial Nugget Hops

*Preparation of crude extracts*: Protocol adapted from the previous one was used for the preparation of hydro-ethanolic extracts for quantification. Extractions were performed in Eppendorf tubes, on 50 mg of hop powder in 1 mL of solvent mix, during three one-hour sessions of maceration, and supernatants were collected by tubes centrifugation. Hydro-ethanolic extracts for quantitation were prepared in quintuplicate and injected at 1 mg.mL⁻¹ on UHPLC-UV-MS for quantification of main hop specialized metabolites and in particular beta-acids.

### UHPLC-UV-MS Parameters

Analyses of purity and quantification of beta-acids (co-, n- and ad-lupulone) in each hydro-ethanolic extract was performed by UHPLC-UV-MS with the equipment and the column described in part "General experimental procedures". The mobile phase consisted of 0.1% formic acid in water, and of 0.1% formic acid in acetonitrile. The gradient of acetonitrile was: 50% (0-1 min), 50-75% (1-3 min), 75% (3-5 min), 75-100% (5-7 min) and 100% (7-9.5 min) at 0.3 mL/min. Column temperature was set at 30°C. Solutions of crude extracts were prepared in MeOH at 100 µg/mL for cones. Injection volume was 2 µL. The main beta-acids were identified based on the retention time of the purified standards and their mass spectra. The ionization was performed in negative mode. Cone voltage was set at 10V. Probe temperature was 600 °C. Capillary voltage was 0.8 kV. The MS-Scan mode was used from 100 to 1000 Da. Beta-acids were quantified in crude extracts according to the International Conference for Harmonization (ICH) [4] of Technical Requirements for Pharmaceuticals for Human Use guideline Q2-R1 (ICH, 2005). Quantification was carried out in UV at 330 nm for acylphloroglucinols.

### Validation of Quantification Method

The quantitation method was set up using standards purified in the laboratory according to the protocol detailed by Bocquet et al. (2019) [5]. Stock solutions of co-, n-lupulone and ad-lupulone were elaborated at a concentration of 1 mg.mL⁻¹ in methanol in triplicate and stored at - 20 _{°}C prior to use for quantification. Ten working solutions (from 100 µg.mL⁻¹ to 0.1 µg.mL⁻¹) were then prepared from these stock solutions. Calibration curves were established by plotting peak area (y) as a function of the nominal concentration for each calibration level (x) and then fitted by weighted (1/x) least square linear regression. Linearity and sensitivity of the method were determined (Table 1). For these reference compounds, the limit of detection (LOD) and the limit of quantification (LOQ) were experimentally determined as the lowest concentration with signal-to-noise ratio of 3 and 10, respectively (Table 1).

**Table 1. Linearity and sensitivity of the quantification method. The precision of the chromatographic system was tested by performing intra- and inter-day multiple injections of the hop extract and then checking the RSD % of retention times and peak areas. Five injections were performed each day for three consecutive days (n = 3, k = 3). Beta-acids were identified based on the retention time of purified standards and their mass spectra and were quantified using the quantification method previously set up on the Empower software. Data were exported from Empower to Excel and were converted from µg.mL-1 to % of dry weight using the total extract volume and the dry weight.**

| | Linearity range (µg/ml) | LOD (ng/ml) | LOQ (ng/ml) | Equation | R² |
|---|---|---|---|---|---|
| Colupulone (330 nm) | 0.5-50 | 50 | 250 | y =7620x+1370 | 0.999842 |
| Lupulone (330 nm) | 0.25-50 | 50 | 250 | y =7250x+2440 | 0.998409 |
| Adlupulone (330 nm) | 0.5-50 | 50 | 250 | y =3990-359 | 0.999862 |

### Example 6 (General Information for Synthesis)

Starting materials, reagents and dry solvents were obtained from Sigma-Aldrich, ABCR GmbH & Co., Alfa Aesar, Fluorochem and Apollo Scientific and used without further purification. Solvents were obtained from Carlo Erba, VWR and Fisher Scientific. All reactions were performed in standard glassware. Thin-layer chromatography (TLC) were performed using Merck silica gel plates (60 F-254, 0.25 mm) on aluminium sheets and revealed under UV lamp (325 and 254 nm). Crude mixtures were purified by flash column chromatography on silica gel 60 (230-400 mesh, 0.040-0.063 mm) purchased from VWR. NMR spectra were recorded on a Bruker Avance 400 apparatus (¹H NMR, 400 MHz - ¹³C NMR, 101 MHz - ¹⁹F NMR, 377 MHz) or Bruker Avance III HD 500 MHz apparatus (¹H NMR, 500 MHz - ¹³C NMR, 126 MHz - ¹⁹F NMR, 471 MHz) at the ECPM. All chemical shifts (δ) are quoted in parts per million (ppm). The chemical shifts are referred to the used partial deuterated NMR solvent (e.g.; CDCl₃. ¹H NMR, 7.26 ppm and ¹³C NMR, 77.16 ppm). The coupling constants (J) are given in Hertz (Hz). Resonance patterns are reported with the following notations: br (broad), s (singlet), d (doublet), t (triplet), q (quartet), m (multiplet), tt (triplet of triplets), qu (quintet), sext (sextet), sept (septet), hep (heptet), tsept (triplet of septets), etc. High-resolution mass spectrometry (HRMS) analyses were performed with a Bruker MicroTOF mass analyzer under ESI in the positive mode detection (measurement accuracy ≤15 ppm) at the Service de Spectrométrie de Masse Fédération Chimie Le Bel in Strasbourg.

### Example 7 (general protocols)

### General Procedures for Acylation, Prenylation, Geranylation and Acetylation of Phloroglucinol (Antiviral Activities in Table 1 and Table 2)

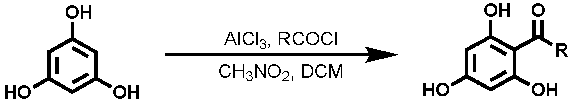

**General procedure A of acylation of phloroglucinol.** Phloroglucinol (1 equiv.) and anhydrous aluminium chloride (1.2 equiv.) were suspended in DCM (10 equiv.) in a round-bottomed flask equipped with a stirrer under argon. Nitromethane (1.2 equiv.) was then added drop by drop with stirring and cooled to 5°C until the reaction mixture became homogeneous. The reaction mixture was heated to 35 °C, which leads to the intensive formation of HCl. Acyl chloride (1.2 equiv.) was added dropwise and the mixture was refluxed for 90 min. The resulting mixture was decomposed with ice/HCl and then DCM and nitromethane were evaporated under vacuum. The residue was extracted with diethyl ether, washed with brine and dried with MgSO₄. After filtration and evaporation of solvents, the crude was purified by column chromatography on silica gel in DCM/MeOH 98:2 (v/v) to give a transparent oil, which was dried under high vacuum to give a crystalline foam.

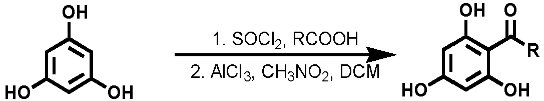

**General procedure B of acylation of phloroglucinol.** The carboxylic acid (1 equiv.) and SOCl₂ (8 equiv.) were added to a dry round-bottomed flask. The mixture was heated to reflux for 2 h. The excess SOCl₂ was then removed. The crude acyl chloride was used for the next step without further purification. Phloroglucinol (1 equiv.) and anhydrous aluminium chloride (1.2 equiv.) were suspended in DCM (10 equiv.) in a round-bottomed flask equipped with a stirrer under argon. Nitromethane (1.2 equiv.) was then added drop by drop with stirring and cooled to 5 °C until the reaction mixture became homogeneous. The reaction mixture was heated to 35 °C, resulting in the intense formation of HCl. Acyl chloride (1.2 equiv.) was added dropwise and the mixture was refluxed for 90 min. The resulting complex was decomposed with ice/HCl, followed by evaporation of DCM and nitromethane under vacuum. The residue was extracted with diethyl ether, washed with brine and dried with MgSO₄. After filtration and evaporation of solvents, the crude was purified by column chromatography on silica gel in DCM/MeOH 98:2 (v/v) to give a transparent oil, which was dried under high vacuum to give a crystalline foam.

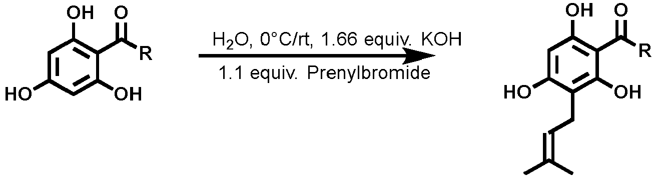

**General procedure of monoprenylation of acylated phloroglucinol.** To the stirred solution of acylphloroglucinol (1 equiv.) in water (94 equiv.) under an argon atmosphere at 0 °C, potassium hydroxide (1.66 equiv.) was added in one portion. The reaction mixture immediately turned yellow, at which point prenyl bromide (1.1 equiv.) was added drop by drop over several minutes. The reaction was allowed to reach room temperature and stirred for 2 hours. The reaction mixture was then acidified with aqueous 1N HCl and extracted with EtOAc. The combined organic layers were washed with brine, dried over MgSO₄, filtered and concentrated under reduced pressure. The residue was purified by column chromatography on silica gel in cyclohexane/EtOAc 8:2 (v/v).

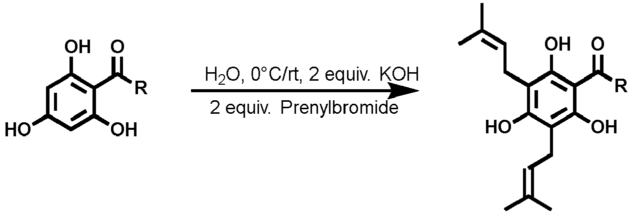

**General procedure of diprenylation of acylated phloroglucinol.** To the stirred solution of acylphloroglucinol (1 equiv.) in water (148 equiv.) under an argon atmosphere at 0 °C, potassium hydroxide (2 equiv.) was added in one portion. The reaction mixture immediately turned yellow, at which point prenyl bromide (2 equiv.) was added dropwise over several minutes. The reaction was allowed to reach room temperature and stirred for 2 hours. The reaction mixture was then acidified with 1N aqueous HCl and extracted with EtOAc. The combined organic layers were washed with brine, dried over MgSO₄, filtered and concentrated under reduced pressure. The residue was purified by column chromatography on silica gel in cyclohexane/EtOAc 98:2 (v/v).

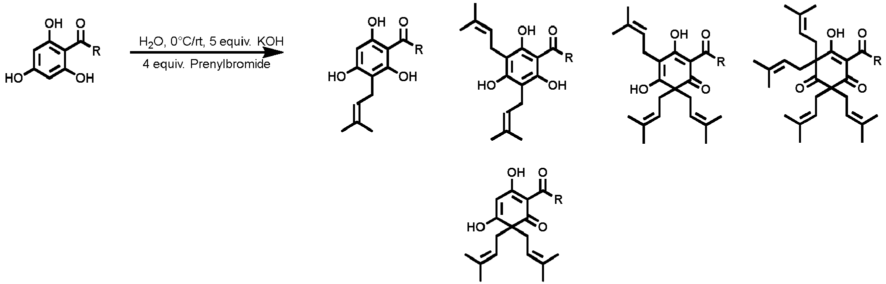

**General procedure of prenylation of acylated phloroglucinol.** To the stirred solution of acylated phloroglucinol (1 equiv.) in water (550 equiv.) under an argon atmosphere at 0 °C, potassium hydroxide (5 equiv.) was added in one portion. The reaction mixture immediately turned yellow, at which point prenyl bromide (4 equiv.) was added dropwise over several minutes. The reaction was allowed to reach room temperature and stirred for 2 hours. The reaction mixture was then acidified with 1N aqueous HCl and extracted with EtOAc. The combined organic layers were washed with brine, dried over MgSO₄, filtered and concentrated under reduced pressure. The residue was purified by column chromatography on silica gel in cyclohexane/EtOAc 9:1 (v/v).

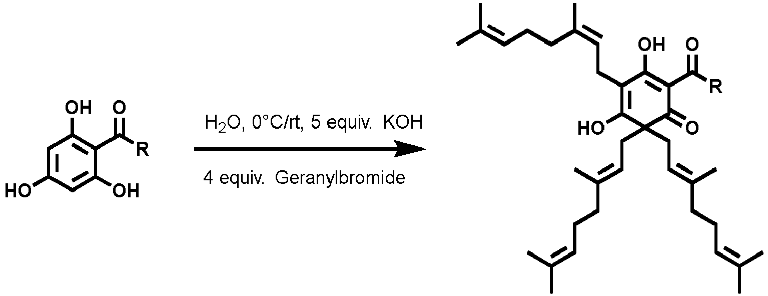

**General procedure of geranylation of acylated phloroglucinol.** To the stirred solution of acylphloroglucinol (1 equiv.) in water (550 equiv.) under an argon atmosphere at 0 °C, potassium hydroxide (5 equiv.) was added in one portion. The reaction mixture immediately turned yellow, at which point geranyl bromide (4 equiv.) was added dropwise over several minutes. The reaction was allowed to reach room temperature and stirred for 2 hours. The reaction mixture was then acidified with aqueous 1N HCl and extracted with EtOAc. The combined organic layers were washed with brine, dried over MgSO₄, filtered and concentrated under reduced pressure. The residue was purified by column chromatography on silica gel in pentane.

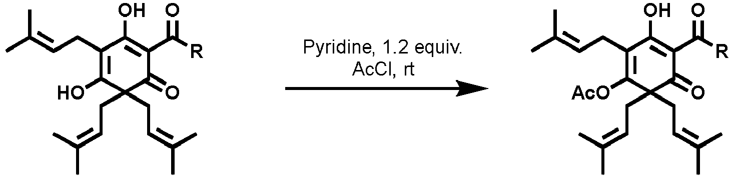

**General procedure of acetylation.** Acetyl chloride (1.2 equiv.) was added dropwise to a stirred solution of the triprenylated derivative (1 equiv.) in pyridine (113 equiv.) under an argon atmosphere at room temperature. When the reaction was complete, the reaction mixture was poured into 10 mL of 7% NaHCOs aqueous solution and the solution was extracted with EtOAc. The combined organic layers were dried over MgSO₄, filtered and concentrated under reduced pressure. The residue was purified by column chromatography on silica gel in cyclohexane/EtOAc 98:2 (v/v).

### Example 8 (Synthesis of natural beta-acids)

### Synthesis of Lupulone and Analogues

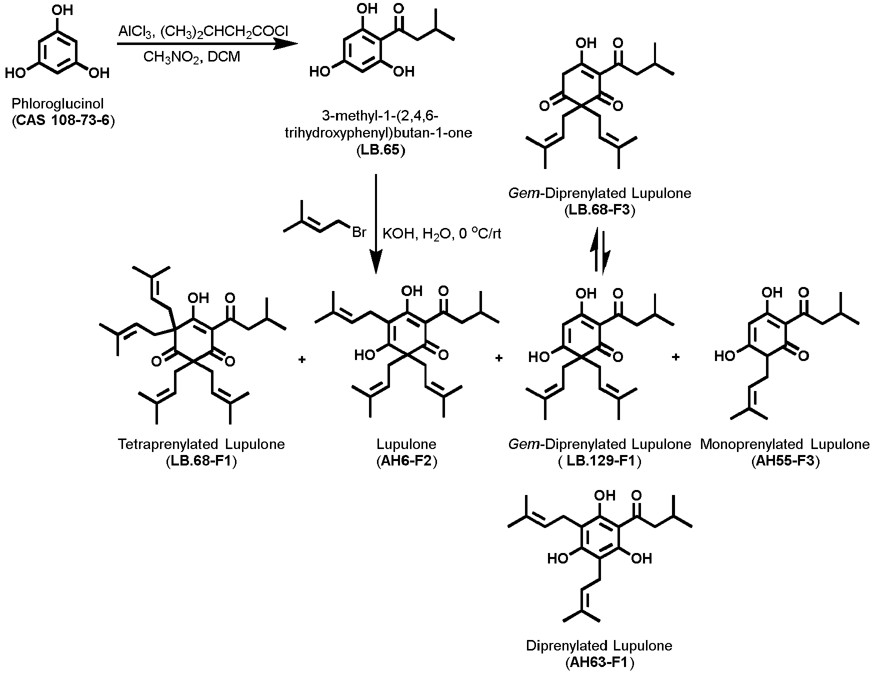

### 3-methyl-1-(2,4,6-trihydroxyphenyl)butan-1-one (LB.65)

According to the general procedure of acylation A, isovaleryl chloride was used. LB.65 was isolated as a light yellow solid (1067 mg, 69%). **MP** 145-146 °C. **¹H NMR** (400 MHz, CDsOD) δ 5.82 (s, 2H), 2.92 (d, J = 6.7 Hz, 2H), 2.29 - 2.12 (m, 1H), 0.96 (d, J = 6.7 Hz, 6H). **¹³C NMR** (101 MHz, CD₃OD) δ 207.01, 165.91, 165.76, 105.57, 95.72, 53.64, 26.67, 23.14. **HRMS (ESI+)** *m*/*z* calcd for C₁₁H₁₅O₄ [M + H]⁺ 211.0965, found 211.0978.

### 5-hydroxy-2,2,6,6-tetrakis(3-methylbut-2-en-1-yl)-4-(3-methylbutanoyl)cyclohex-4-ene-1,3-dione (L8.68-F1)

According to the general procedure of prenylation, 3-methyl-1-(2,4,6-trihydroxyphenyl)butan-1-one was used. LB.68-F1 was isolated as a transparent oil (56 mg, 24 %). **¹H NMR** (400 MHz, CDCl₃) δ 4.98 - 4.82 (m, 4H), 2.80 (d, *J* = 6.9 Hz, 2H), 2.64 (dd, *J* = 13.8, 8.0 Hz, 2H), 2.58 - 2.46 (m, 4H), 2.34 - 2.25 (m, 2H), 2.25 - 2.14 (m, 1H), 1.62 (s, 6H), 1.60 (s, 6H), 1.57 (s, 6H), 1.54 (s, 6H), 0.99 (d, *J* = 6.6 Hz, 6H). **¹³C NMR** (101 MHz, CDCl₃) δ 207.71, 203.82, 197.58, 194.90, 136.31, 134.76, 119.08, 118.45, 113.74, 65.70, 61.12, 47.81, 36.99, 34.04, 26.12, 26.01, 25.94, 22.86, 18.05, 18.00. **HRMS (ESI+)** *m*/*z* calcd for C₃₁H₄₆NaO₄ [M + Na]⁺ 505.3288, found 505.3296.

### 3,5-dihydroxy-4,6,6-tris(3-methylbut-2-en-1-yl)-2-(3-methylbutanoyl)cyclohexa-2,4-dien-1-one (AH6-F2) - Synthetic lupulone

According to the general procedure of prenylation, 3-methyl-1-(2,4,6-trihydroxyphenyl)butan-1-one was used. AH6-F2 was isolated as white crystals (152 mg, 39 %). **MP** 92-93 °C. **¹H NMR** (400 MHz, CDsOD) δ 5.07 - 4.98 (m, 1H), 4.83 - 4.71 (m, 2H), 3.10 (d, *J* = 7.0 Hz, 2H), 2.85 (d, *J* = 6.9 Hz, 2H), 2.71 - 2.49 (m, 4H), 2.08 (hep, *J* = 6.8 Hz, 1H), 1.73 (s, 3H), 1.70 - 1.65 (m, 3H), 1.60 - 1.56 (m, 6H), 1.56 - 1.51 (m, 6H), 0.95 (d, *J* = 6.7 Hz, 6H). **¹³C NMR** (101 MHz, CD₃OD) δ 204.03, 198.09, 190.99, 174.28, 135.69, 132.54, 123.29, 119.20, 112.44, 109.42, 58.73, 50.07, 38.68, 27.08, 26.04, 23.11, 21.67, 18.10, 17.98. **HRMS (ESI+)** *m*/*z* calcd for C₂₆H₃₈NaO₄ [M + Na]⁺ 437.2662, found 437.2666.

### 5-hydroxy-2,2-bis(3-methylbut-2-en-1-yl)-4-(3-methylbutanoyl)cyclohex-4-ene-1,3-dione (LB. 68-F3)

According to the general procedure of prenylation, 3-methyl-1-(2,4,6-trihydroxyphenyl)butan-1-one was used. LB.68-F3 was isolated as a transparent oil (11 mg, 7 %). **¹H NMR** (400 MHz, CDCl₃) δ 4.88 - 4.79 (m, 2H), 3.50 (s, 1H), 3.38 (s, 1H), 2.98 - 2.88 (m, 2H), 2.75 - 2.53 (m, 4H), 2.21 - 2.10 (m, 1H), 1.61 - 1.59 (m, 6H), 1.60 - 1.54 (m, 3H), 1.56 - 1.55 (m, 3H), 0.98 (d, *J* = 6.7 Hz, 6H). **¹³C NMR** (101 MHz, CDCl₃) δ 205.04, 204.30, 199.80, 188.83, 137.08, 136.03, 118.24, 117.98, 117.49, 114.56, 62.10, 55.93, 48.65, 48.46, 36.79, 36.29, 26.22, 26.05, 26.01, 25.71, 22.85, 22.72, 18.04, 17.97. **HRMS (ESI+)** *m*/*z* calcd for C₂₁H₃₀NaO₄ [M + Na]⁺ 369.2036, found 369.2053.

### 3,5-dihydroxy-6,6-bis(3-methylbut-2-en-1-yl)-2-(3-methylbutanoyl)cyclohexa-2,4-dien-1-one (LB.129-F1)

According to the general procedure of prenylation, 3-methyl-1-(2,4,6-trihydroxyphenyl)butan-1-one was used. LB.129-F1 was isolated as a light yellow solid (40 mg, 24 %). **MP** 84-85 °C. **¹H NMR** (400 MHz, CDCl₃) δ 10.14 (br s, 2H), 6.27 (s, 1H), 5.27 - 5.18 (m, 2H), 3.41 - 3.34 (m, 4H), 2.94 (d, *J* = 6.8 Hz, 2H), 2.35 - 2.16 (m, 1H), 1.86 - 1.82 (m, 6H), 1.81 - 1.77 (m, 6H), 0.96 (d, *J* = 6.7 Hz, 6H). **¹³C NMR** (126 MHz, CDCl₃) δ 206.27, 159.41, 159.30, 136.70, 121.79, 105.44, 104.79, 53.16, 26.03, 25.16, 22.99, 21.93, 18.06. **HRMS (ESI+)** *m*/*z* calcd for C₂₁H₃₀NaO₄ [M + Na]⁺ 369.2036, found 369.2056.

### 3-methyl-1-(2,4,6-trihydroxy-3,5-bis-(3-methylbut-2-en-1-yl)phenyl)butan-1-one (AH63-F1)

According to the general procedure of diprenylation, 3-methyl-1-(2,4,6-trihydroxyphenyl)butan-1-one was used. AH63-F1 was isolated as a light yellow oil (40 mg, 24%). **¹H NMR** (400 MHz, CDsOD) δ 5.12 (tsept, J = 6.9, 4.3, 1.4 Hz, 2H), 3.30 - 3.26 (m, 4H), 2.96 (d, J = 6.8 Hz, 2H), 2.28 - 2.17 (m, 1H), 1.79 - 1.73 (m, 6H), 1.69 - 1.64 (m, 6H), 0.95 (dd, J = 6.7, 2.3 Hz, 6H). **¹³C NMR** (101 MHz, CDsOD) δ 207.84, 160.99, 159.92, 132.71, 123.95, 108.63, 107.28, 54.15, 26.83, 25.94, 23.21, 22.66, 17.98. **HRMS (ESI+)** *m*/*z* calcd for C₂₁H₃₀NaO₄ [M + Na]⁺ 369.2036, found 369.2032

### 3,5-dihydroxy-6-(3-methylbut-2-en-1-yl)-2-(3-methylbutanoyl)cyclohexa-2,4-dien-1-one (AH55-F3)

According to the general procedure of monoprenylation, 3-methyl-1-(2,4,6-trihydroxyphenyl)butan-1-one was used. AH55-F3 was isolated as a white solid (43 mg, 33 %). **MP** 134-136 °C. **¹H NMR** (400 MHz, CDsOD) δ 5.90 (s, 1H), 5.23 - 5.15 (m, 1H), 3.22 -3.17 (m, 2H), 2.93 (d, J = 6.8 Hz, 2H), 2.23 (hept, J = 13.4, 6.7 Hz, 1H), 1.76 (d, J = 1.3 Hz, 3H), 1.66 (d, J = 1.4 Hz, 3H), 0.97 (d, J = 6.7 Hz, 6H). **¹³C NMR** (101 MHz, CD₃OD) δ 207.04, 165.04, 163.55, 161.33, 131.03, 124.57, 108.02, 105.46, 94.84, 53.78, 26.82, 25.96, 23.20 (2C), 22.16, 17.85. **HRMS (ESI+)** *m*/*z* calcd for C₁₆H₂₂NaO₄ [M + Na]⁺ 301.1410, found 301.1405.

### Synthesis of Co-Lupulone and Analogues

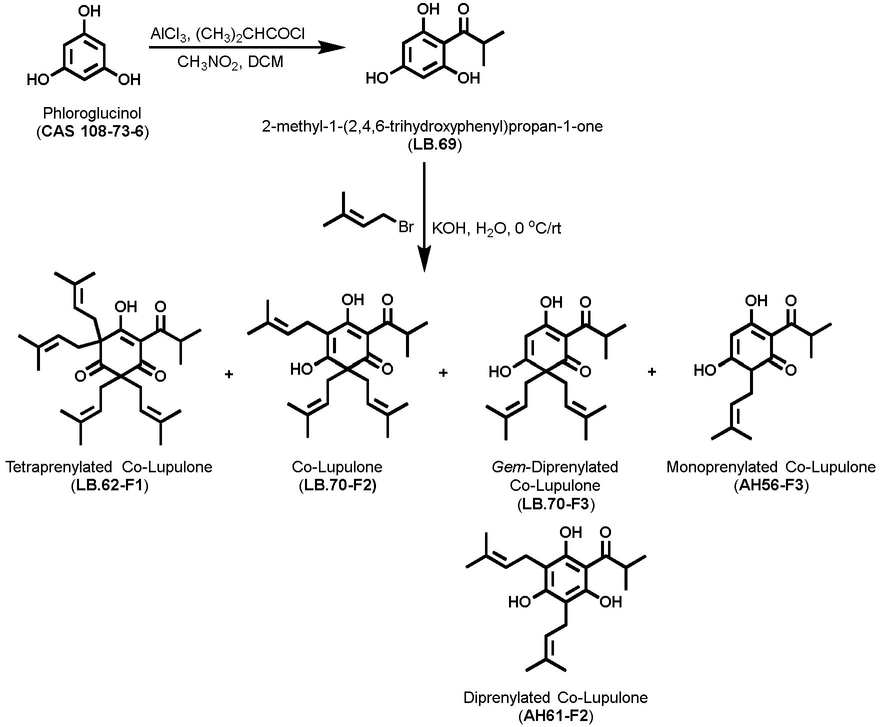

### 2-methyl-1-(2,4,6-trihydroxyphenyl)propan-1-one (LB.69)

According to the general procedure of acylation A, isobutyryl chloride was used. LB.69 was isolated as a light yellow crystalline foam (610 mg, 78%). **MP** 49-51 °C. **¹H NMR** (400 MHz, CDsOD) δ 5.81 (s, 2H), 3.97 (hept, *J* = 6.8 Hz, 1H), 1.13 (d, *J* = 6.7 Hz, 6H). **¹³C NMR** (101 MHz, CDsOD) δ 211.63, 165.84, 165.76, 104.60, 95.84, 39.92, 19.66. **HRMS (ESI+)** *m*/*z* calcd for C₁₀H₁₃O₄ [M + H]⁺ 197.0808, found 197.0803.

### 5-hydroxy-2,2,6,6-tetrakis(3-methylbut-2-en-1-yl)-4-(2-methylpropanoyl)cyclo-hex-4-ene-1,3-dione (L8.62-F1)

According to the general procedure of prenylation, 2-methyl-1-(2,4,6-trihydroxyphenyl)propan-1-one was used. LB.62-F1 was isolated as a white solid (196 mg, 23 %). **MP** 41-42 °C. **¹H NMR** (400 MHz, CDCl₃) δ 4.97 - 4.86 (m, 4H), 3.66 (hept, *J* = 6.7 Hz, 1H), 2.64 (dd, *J* = 13.7, 8.0 Hz, 2H), 2.58 - 2.48 (m, 4H), 2.33 - 2.22 (m, 2H), 1.62 (s, 6H), 1.60 - 1.58 (m, 6H), 1.57 (s, 6H), 1.54 - 1.51 (m, 6H), 1.17 (d, *J* = 6.8 Hz, 6H). **¹³C NMR** (101 MHz, CDCl₃) δ 208.41, 207.62, 197.89, 194.59, 136.44, 134.72, 119.08, 118.48, 112.61, 65.87, 61.23, 37.20, 35.67, 33.86, 26.10, 25.98, 19.34, 18.05, 17.97. **HRMS (ESI+)** *m*/*z* calcd for C₃₀H₄₄NaO₄ [M + Na]⁺ 491.3132, found 491.3123.

### 3,5-dihydroxy-4,6,6-tris(3-methylbut-2-en-1-yl)-2-(2-methylpropanoyl)cyclo-hexa-2,4-dien-1-one (LB.70-F2) - Synthetic co-lupulone

According to the general procedure of prenylation, 2-methyl-1-(2,4,6-trihydroxyphenyl)propan-1-one was used. LB.70-F2 was isolated as a white solid (226 mg, 31 %). **MP** 84-85 °C.**¹H NMR** (400 MHz, CDsOD) δ 5.04 - 4.96 (m, 1H), 4.80 - 4.71 (m, 2H), 3.97 (hept, *J* = 6.2 Hz, 1H), 3.09 (d, *J* = 7.1 Hz, 2H), 2.74 - 2.49 (m, 4H), 1.74 (s, 3H), 1.67 (s, 3H), 1.58 (s, 6H), 1.55 (s, 6H), 1.09 (d, *J* = 6.8 Hz, 6H). **¹³C NMR** (101 MHz, CD₃OD) δ 208.93, 197.87, 191.10, 174.47, 135.71, 132.50, 123.20, 119.14, 112.08, 107.91, 58.79, 49.00, 38.85, 37.34, 26.14, 21.61, 19.24, 18.11, 17.99. **HRMS (ESI+)** *m*/*z* calcd for C₂₅H₃₆NaO₄ [M + Na]⁺ 423.2506, found 423.2504.

### 2-methyl-1-(2,4,6-trihydroxy-3,5-bis-(3-methylbut-2-en-1-yl)phenyl)propan-1-one (AH61-F2)

According to the general procedure of diprenylation, 2-methyl-1-(2,4,6-trihydroxyphenyl)propan-1-one was used. AH61-F2 was isolated as a light yellow oil (35 mg, 21 %). **¹H NMR** (400 MHz, CD₃OD) δ 5.12 (tsept, J = 6.9, 5.4, 2.9, 1.5 Hz, 2H), 4.07 - 3.99 (m, 1H), 1.77 (s, 6H), 1.68 (s, 6H), 1.14 (d, J = 6.7 Hz, 6H).). **¹³C NMR** (101 MHz, CD₃OD) δ 212.61, 160.81, 159.83, 132.73, 123.96, 108.71, 106.32, 40.31, 25.94, 22.70, 19.94, 17.98. **HRMS (ESI+)** *m*/*z* calcd for C₂₀H₂₈NaO₄ [M + Na]⁺ 355.1880, found 355.1872

### 3,5-dihydroxy-6,6-bis(3-methylbut-2-en-1-yl)-2-(2-methylpropanoyl)cyclohexa-2,4-dien-1-one (LB.70-F3)

According to the general procedure of prenylation, 2-methyl-1-(2,4,6-trihydroxyphenyl)propan-1-one was used. LB.70-F3 was isolated as a transparent oil (110 mg, 18 %) with a complicated NMR involving 2 isomers I equilibrium.

### 2-methyl-1-(2,4,6-trihydroxy-3-(3-methylbut-2-en-1-yl)phenyl)propan-1-one (AH56-F3)

According to the general procedure of monoprenylation, 2-methyl-1-(2,4,6-trihydroxyphenyl)propan-1-one was used. AH56-F3 was isolated as a light yellow powder (60 mg, 22%). **¹H NMR** (400 MHz, CDsOD) δ 5.89 (s, 1H), 5.17 (tsept, J = 7.1, 2.8, 1.4 Hz, 1H), 4.00 (hept, J = 6.7 Hz, 1H), 3.17 (dt, J = 7.2, 1.2 Hz, 2H), 1.74 (d, J = 1.3 Hz, 3H), 1.64 (q, J = 1.3 Hz, 3H). **¹³C NMR** (101 MHz, CD₃OD) δ 211.72, 165.28, 163.40, 161.01, 131.02, 124.58, 108.13, 104.49, 94.94, 39.90, 25.95, 22.20, 19.79, 17.84. **HRMS (ESI+)** *m*/*z* calcd for C₁₅H₂₀NaO₄ [M + Na]⁺ 287.1254, found 287.1251

### Synthesis of Ad-Lupulone and Analogues

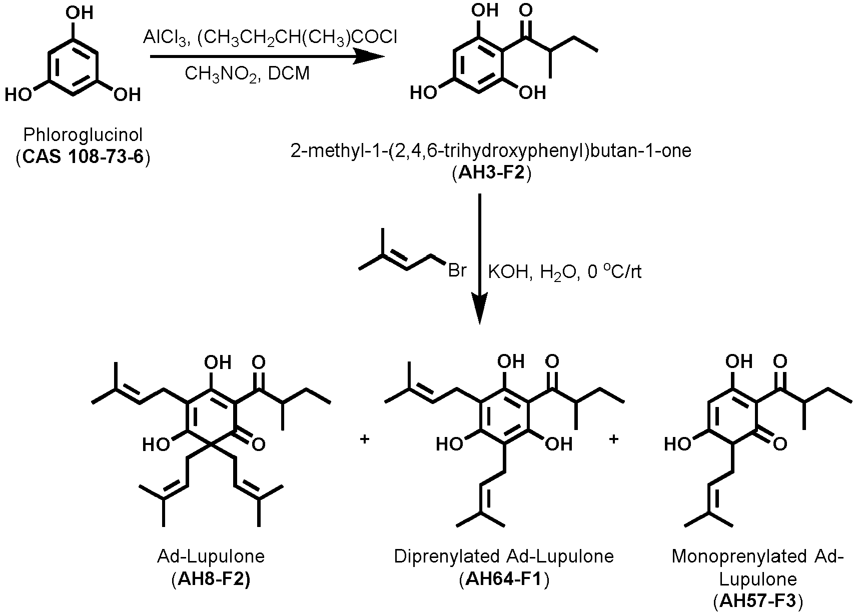

### 2-methyl-1-(2,4,6-trihydroxyphenyl)butan-1-one (AH3) [6]

According to the general procedure of acylation A, 2-methylbutyryl chloride was used. AH3 was isolated as a light yellow solid (840 mg, 50%). **¹H NMR** (400 MHz, CDsOD) δ 5.81 (s, 2H), 3.85 (h, J = 6.7 Hz, 1H), 1.89 - 1.71 (m, 1H), 1.44 - 1.28 (m, 1H), 1.11 (d, J = 6.8 Hz, 3H), 0.90 (t, J = 7.4 Hz, 3H). **¹³C NMR** (101 MHz, CDsOD) δ 211.43, 165.82, 165.76, 105.23, 95.85, 46.68, 28.10, 17.12, 12.32.

### 3,5-dihydroxy-4,6,6-tris(3-methylbut-2-en-1-yl)-2-(2-methylbutanoyl)cyclohexa-2,4-dien-1-one (AH8-F2) (Synthetic adlupulone)

According to the general procedure of prenylation, 2-methyl-1-(2,4,6-trihydroxyphenyl)butan-1-one was used. AH8-F2 was isolated as a light yellow oil (78 mg, 20%). **¹H NMR** (400 MHz, CD₃OD) δ 5.02 (tsept, J = 7.1, 1.5 Hz, 1H), 4.77 (qsept, J = 9.1, 7.7, 6.3, 1.4 Hz, 2H), 3.99- 3.82 (m, 1H), 3.10 (d, J = 7.0 Hz, 2H), 2.71 - 2.50 (m, 4H), 1.73 (d, J = 1.3 Hz, 3H), 1.67 (d, J = 1.4 Hz, 3H), 1.57 (d, J = 1.4 Hz, 6H), 1.55 (s, 6H), 1.41 - 1.31 (m, 2H), 1.08 (d, J = 6.8 Hz, 3H), 0.89 (t, J = 7.4 Hz, 3H). **¹³C NMR** (101 MHz, CDsOD) δ 203.22, 196.70, 189.30, 173.03, 134.34, 131.16, 122.97, 121.90, 117.80, 115.11, 42.11, 37.27, 24.64, 20.25, 16.70, 16.58, 12.99. **HRMS (ESI-**) *m*/*z* calcd for C₂₆H₃₇O₄ [M - H]⁻ 413.2697, found 413.2671

### 2-methyl-1-(2,4,6-trihydroxy-3,5-bis-(3-methylbut-2-en-1-yl)phenyl)butan-1-one (AH64-F1)

According to the general procedure of diprenylation, 2-methyl-1-(2,4,6-trihydroxyphenyl)butan-1-one was used. AH64-F1 was isolated as a light yellow oil (30 mg, 18%). **¹H NMR** (400 MHz, CDsOD) δ 5.88 (s, 1H), 5.17 (dddd, J = 8.5, 5.7, 2.8, 1.4 Hz, 1H), 3.87 (h, J = 6.7 Hz, 1H), 3.18 (d, J = 7.2 Hz, 2H), 1.81 (dqd, J = 13.8, 7.4, 6.0 Hz, 1H), 1.75-1.71 (m, 3H), 1.64 (q, J = 1.2 Hz, 3H), 1.36 (ddt, J = 14.4, 13.2, 7.0 Hz, 1H), 1.11 (d, J = 6.8 Hz, 3H), 0.90 (t, J = 7.4 Hz, 3H). **¹³C NMR** (101 MHz, CDsOD) δ 212.42, 160.81, 159.83, 132.74, 123.94, 108.65, 106.96, 47.08, 28.35, 25.94, 22.69, 17.98, 17.34, 12.35. **HRMS (ESI+)** *m*/*z* calcd for C₂₁H₃₀NaO₄ [M + Na]⁺ 369.2036, found 369.2032

### 2-methyl-1-(2,4,6-trihydroxy-3-(3-methylbut-2-en-1-yl)phenyl)butan-1-one (AH57-F3)

According to the general procedure of monoprenylation, 2-methyl-1-(2,4,6-trihydroxyphenyl)butan-1-one was used. AH57-F3 was isolated as a light yellow oil (90 mg, 34%). **¹H NMR** (400 MHz, CDsOD) δ 5.88 (s, 1H), 5.17 (tsept, J = 8.5, 5.7, 2.8, 1.4 Hz, 1H), 3.87 (sext, J = 6.7 Hz, 1H), 3.18 (d, J = 7.2 Hz, 2H), 1.81 (dsept, J = 13.8, 7.4, 6.0 Hz, 1H), 1.75 - 1.71 (m, 3H), 1.64 (q, J = 1.2 Hz, 3H), 1.36 (sept, J = 14.4, 13.2, 7.0 Hz, 1H), 1.11 (d, J = 6.8 Hz, 3H), 0.90 (t, J = 7.4 Hz, 3H). **¹³C NMR** (101 MHz, CDsOD) δ 211.50, 165.24, 165.22, 163.39, 163.36, 161.09, 161.04, 131.02, 124.60, 108.12, 105.13, 94.97, 48.66, 46.68, 28.21, 25.95, 22.20, 17.85, 17.25, 12.37. **HRMS (ESI+)** *m*/*z* calcd for C₁₆H₂₂NaO₄ [M + Na]⁺ 301.1410, found 301.1408

### Synthesis of Aceto-Lupulone and Analogues

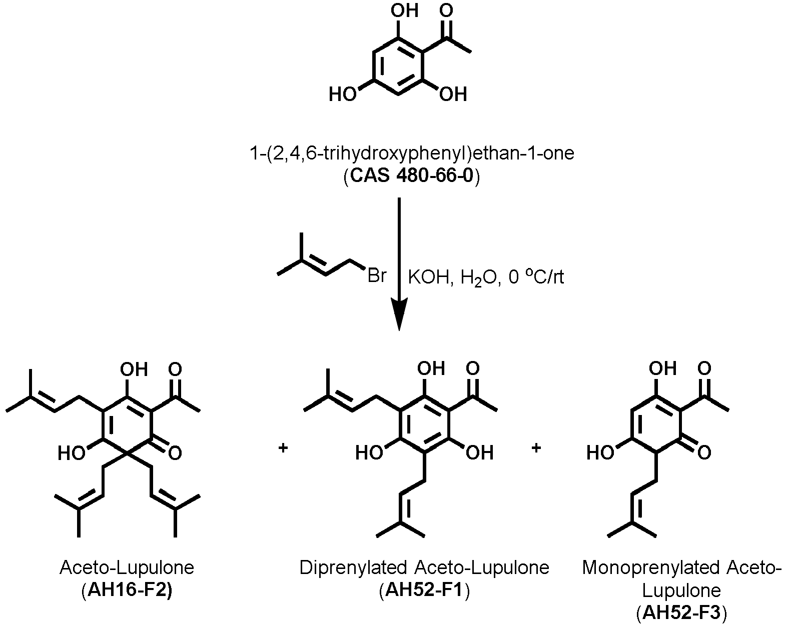

### 2-acetyl-3,5-dihydroxy-4,6,6-tris(3-methylbut-2-en-1-yl)cyclohexa-2,4-dien-1-one (AH16-F2) (Synthetic acetolupulone)

According to the general procedure of prenylation, 2',4',6'-trihydroxyacetophenone was used. AH16-F2 was isolated as a light yellow oil (180 mg, 27%). **¹H NMR** (400 MHz, CDsOD) δ 5.05 - 4.97 (m, 1H), 4.80 - 4.72 (m, 2H), 3.09 (d, J = 7.0 Hz, 2H), 2.69 - 2.54 (m, 4H), 2.51 (s, 3H), 1.73 (d, J = 1.3 Hz, 3H), 1.67 (d, J = 1.4 Hz, 3H), 1.58 (d, J = 1.3 Hz, 6H), 1.55 (d, J = 1.4 Hz, 6H). **¹³C NMR** (101 MHz, CDsOD) δ 201.45, 198.45, 190.51, 172.96, 135.79, 132.58, 123.20, 119.13, 112.14, 109.50, 58.80, 38.72, 29.16, 27.98, 26.02, 26.00, 21.57, 20.86, 18.06, 17.96. **HRMS (ESI+)** *m*/*z* calcd for C₂₃H₃₂NaO₄ [M + Na]⁺ 395.2193, found 395.2191

### 1-(2,4,6-trihydroxy-3,5-bis-(3-methylbut-2-en-1-yl)phenyl)ethan-1-one (AH52-F1)

According to the general procedure of monoprenylation, 1-(2,4,6-trihydroxyphenyl)ethan-1-one was used. AH52-F1 was isolated as a light yellow oil (35 mg, 10%). **¹H NMR** (400 MHz, CDsOD) δ 5.11 (tsept, J = 8.3, 5.7, 2.9, 1.4 Hz, 2H), 3.30 - 3.26 (m, 4H), 2.65 (s, 3H), 1.78 - 1.74 (m, 6H), 1.69 - 1.66 (m, 6H). **¹³C NMR** (101 MHz, CD₃OD) δ205.36, 161.36, 160.10, 132.71, 123.92, 119.12, 108.70, 107.30, 33.09, 27.98, 25.99, 25.93, 22.63, 17.97. **HRMS (ESI-)** *m*/*z* calcd for C₁₈H₂₃O₄ [M - H]⁻ 303.1602, found 303.1592

### 1-(2,4,6-trihydroxy-3-(3-methylbut-2-en-1-yl)phenyl)ethan-1-one (AH52-F3)

According to the general procedure of monoprenylation, 1-(2,4,6-trihydroxyphenyl)ethan-1-one was used. AH52-F3 was isolated as a light yellow oil (50 mg, 18%). **¹H NMR** (500 MHz, CD₃OD) δ 5.89 (s, 1H), 5.16 (tsept, J = 7.1, 2.8, 1.4 Hz, 1H), 3.17 (dt, J = 7.2, 1.1 Hz, 2H), 1.73 (s, 3H), 1.64 (s, 3H).). **¹³C NMR** (126 MHz, CDsOD) δ 204.58, 164.80, 163.91, 161.80, 131.07, 124.54, 107.92, 105.51, 94.76, 32.82, 25.95, 22.12, 17.83. **HRMS (ESI-)** *m*/*z* calcd for C₁₃H₁₅O₄ [M - H]⁻ 235.0970, found 235.0975

### Synthesis of Post-Lupulone

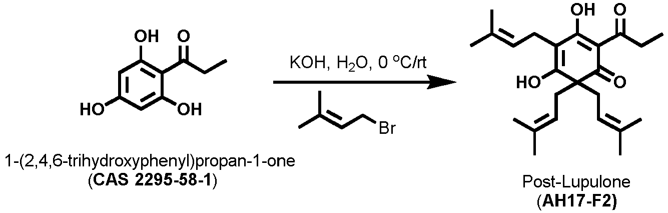

### 3,5-dihydroxy-4,6,6-tris(3-methylbut-2-en-1-yl)-2-propionylcyclohexa-2,4-dien-1-one (AH17-F2) (Synthetic postlupulone)

According to the general procedure of prenylation, 1-(2,4,6-trihydroxyphenyl)propan-1-one (flopropione) was used. AH17-F2 was isolated as a light yellow oil (128 mg, 20%). **¹H NMR** (400 MHz, CDsOD) δ 5.01 (brt, J = 7.0 Hz, 1H), 4.76 (brt, J = 7.6, 2H), 3.09 (d, J = 7.0 Hz, 2H), 3.06 - 2.92 (m, 2H), 2.69 - 2.52 (m, 4H), 1.73 (d, J = 1.3 Hz, 3H), 1.67 (d, J = 1.4 Hz, 3H), 1.59 - 1.53 (m, 12H), 1.11 (t, J = 7.3 Hz, 3H).5.81 (s, 2H), 3.97 (hept, *J* = 6.8 Hz, 1H), 1.13 (d, *J* = 6.7 Hz, 6H). **¹³C NMR** (101 MHz, CDsOD) δ 205.45, 198.07, 190.56, 174.51, 135.72, 132.54, 123.25, 119.16, 38.77, 35.16, 27.97, 26.02, 21.63, 18.08, 17.97, 9.31. **HRMS (ESI+)** *m*/*z* calcd for C₂₄H₃₄NaO₄ [M + Na]⁺ 409.2349, found 409.2344

### Synthesis of Pre-Lupulone

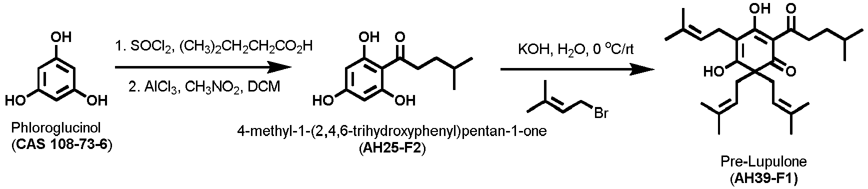

### 4-methyl-1-(2,4,6-trihydroxyphenyl)pent-1-one (AH25-F2) [7]

According to the general procedure of acylation B, 4-methylvaleric acid was used. AH25-F2 was isolated as a light yellow solid (764 mg, 43%). **¹H NMR** (400 MHz, CDsOD) δ 5.80 (s, 2H), 3.06 - 2.99 (m, 2H), 1.62 (dq, J = 12.9, 6.4 Hz, 1H), 1.57 - 1.49 (m, 2H), 0.93 (d, J = 6.4 Hz, 6H). **¹³C NMR** (101 MHz, CD₃OD) δ 207.79, 165.94, 165.77, 105.30, 95.70, 58.33, 42.92, 35.35, 29.29, 22.86, 18.35.

### 3,5-dihydroxy-4,6,6-tris(3-methylbut-2-en-1-yl)-2-(4-methylpentanoyl)cyclohexa-2,4-dien-1-one (AH39-F1) (Synthetic pre-lupulone)

According to the general procedure of prenylation, 4-methyl-1-(2,4,6-trihydroxyphenyl)pent-1-one was used. AH39-F1 was isolated as a light yellow oil (90 mg, 16%). **¹H NMR** (400 MHz, CDsOD) δ 5.06 - 4.97 (m, 1H), 4.81 - 4.71 (m, 2H), 3.09 (d, J = 7.0 Hz, 2H), 3.04 - 2.92 (m, 2H), 2.74 - 2.47 (m, 4H), 1.73 (d, J = 1.3 Hz, 3H), 1.67 (d, J = 1.4 Hz, 3H), 1.65 - 1.59 (m, 1H), 1.58 (d, J = 1.4 Hz, 6H), 1.55 (d, J = 1.4 Hz, 6H), 1.51 - 1.42 (m, 2H), 0.94 (d, J = 6.6 Hz, 6H). **¹³C NMR** (101 MHz, CDsOD) δ 204.89, 198.04, 190.86, 174.35, 135.65, 132.54, 123.26, 119.22, 112.29, 108.99, 58.78, 39.63, 38.74, 35.39, 29.33, 26.04, 26.02, 22.87, 21.64, 18.12, 17.98. **HRMS (ESI+)** *m*/*z* calcd for C₂₇H₄₀NaO₄ [M + Na]⁺ 451.2819, found 451.2814.

### Synthesis of Adpre-Lupulone

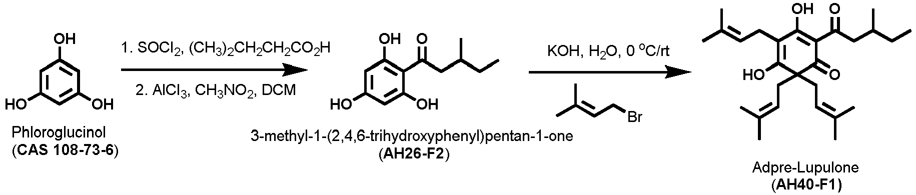

### 3-methyl-1-(2,4,6-trihydroxyphenyl)pent-1-one (AH26-F2)

According to the general procedure of acylation B, DL-3-methylvaleric acid was used. AH26-F2 was isolated as a light yellow solid (733 mg, 41%). **¹H NMR** (400 MHz, CD₃OD) δ 5.80 (s, 2H), 3.11 (dd, J = 14.6, 5.6 Hz, 1H), 2.75 (dd, J = 14.6, 8.0 Hz, 1H), 2.07 - 1.95 (m, 1H), 1.41 (m, 1H), 1.31 - 1.19 (m, 1H), 0.94 - 0.86 (m, 6H). **¹³C NMR** (101 MHz, CDsOD) δ 207.33, 165.98, 165.76, 105.66, 95.73, 51.82, 33.02, 30.93, 19.85, 11.74.

### 3,5-dihydroxy-4,6,6-tris(3-methylbut-2-en-1-yl)-2-(3-methylpentanoyl)cyclohexa-2,4-dien-1-one (AH40-F1) (Synthetic adpre-lupulone)

According to the general procedure of prenylation, 3-methyl-1-(2,4,6-trihydroxyphenyl)pent-1-one was used. AH40-F1 was isolated as a light yellow oil (85 mg, 15%). **¹H NMR** (400 MHz, CDsOD) δ 5.02 (brt, J = 6.9 Hz, 1H), 4.81 - 4.70 (m, 2H), 3.10 (d, J = 7.0 Hz, 2H), 3.06 - 2.97 (m, 1H), 2.74 (dd, J = 12.7, 7.7 Hz, 1H), 2.60 (t, J = 10.3 Hz, 4H), 1.89 (hept, J = 6.8 Hz, 1H), 1.73 (d, J = 1.3 Hz, 3H), 1.67 (d, J = 1.5 Hz, 3H), 1.57 (d, J = 1.3 Hz, 6H), 1.54 (d, J = 1.3 Hz, 6H), 1.45 - 1.37 (m, 1H), 1.33 - 1.25 (m, 1H), 0.92 (t, J = 7.1 Hz, 6H). **¹³C NMR** (101 MHz, CD₃OD) δ 13C NMR (126 MHz, CD₃OD) δ 147.41, 136.12, 132.58, 123.29, 119.23, 119.03, 118.57, 111.01, 94.15, 89.72, 74.27, 72.03, 39.06, 30.82, 29.50, 26.03, 26.01, 24.21, 21.53, 19.69, 18.10, 17.97, 11.70. **HRMS (ESI+)** *m*/*z* calcd for C₂₇H₄₁O₄ [M + H]⁺ 429.2999, found 429.3001.

### Example 9 (Synthesis of non-natural beta-acids)

### Synthesis of AH10-F2

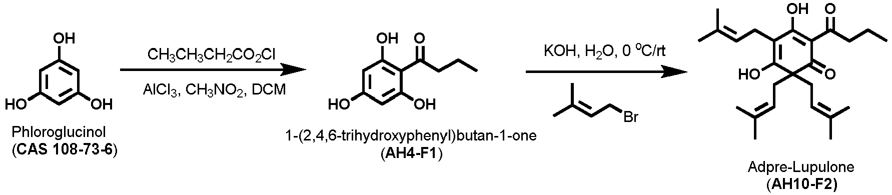

### 1-(2,4,6-trihydroxyphenyl)butan-1-one (AH4-F1) [6]

According to the general procedure of acylation A, butyryl chloride was used. AH4-F1 was isolated as a light yellow solid (212 mg, 14%). **¹H NMR** (400 MHz, CDsOD) δ 5.80 (s, 2H), 3.01 (t, J = 7.8, 7.0 Hz, 2H), 1.68 (h, J = 7.4 Hz, 2H), 0.97 (t, J = 7.4 Hz, 3H). **¹³C NMR** (101 MHz, CD₃OD) δ 207.28, 165.93, 165.80, 105.36, 95.69, 46.78, 19.44, 14.36.

### 2-butyryl-3,5-dihydroxy-4,6,6-tris(3-methylbut-2-en-1-yl)cyclohexa-2,4-dien-1-one (AH10-F2)

According to the general procedure of prenylation, 1-(2,4,6-trihydroxyphenyl)butan-1-one was used. AH10-F2 was isolated as a light yellow oil (45 mg, 11%). **¹H NMR** (400 MHz, CDsOD) δ 5.01 (brt, J = 7.1, 2.8 Hz, 1H), 4.76 (br tsept, J = 7.5, 3.0, 1.5 Hz, 2H), 3.13 - 3.06 (m, 2H), 2.94 (t, J = 7.5 Hz, 2H), 2.68 - 2.51 (m, 4H), 1.73 (d, J = 1.3 Hz, 3H), 1.67 (d, J = 1.5 Hz, 3H), 1.65 - 1.58 (m, 2H), 1.57 (d, J = 1.3 Hz, 6H), 1.55 (d, J = 1.4 Hz, 6H), 0.97 (t, J = 7.4 Hz, 3H). **¹³C NMR** (101 MHz, CDsOD) δ 204.68, 198.05, 190.80, 172.93, 135.67, 132.52, 123.28, 119.20, 112.27, 109.00, 41.28, 38.68, 28.57, 26.05, 23.70, 21.65, 18.11, 14.32. **HRMS (ESI+)** *m*/*z* calcd for C₂₅H₃₆NaO₄ [M + Na]⁺ 423.2506, found 423.2497.

### Synthesis of AH 18-F4

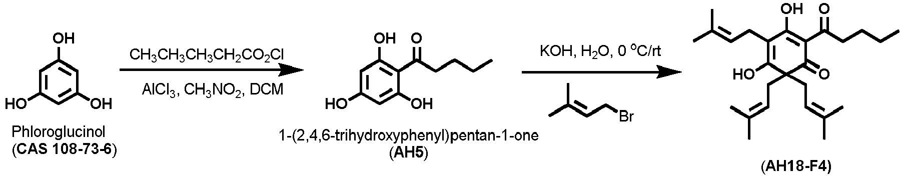

### 1-(2,4,6-trihydroxyphenyl)pent-1-one (AH5) [8]

According to the general procedure of acylation A, valeroyl chloride was used. AH5 was isolated as a light yellow solid (420 mg, 25%). **¹H NMR** (400 MHz, CDsOD) δ 5.80 (s, 2H), 3.08 - 2.96 (m, 2H), 1.68 - 1.55 (m, 2H), 1.45 - 1.31 (m, 2H), 0.93 (t, J = 7.3 Hz, 3H). **¹³C NMR** (101 MHz, CDsOD) δ 207.49, 165.73, 105.31, 95.68, 44.53, 28.34, 23.66, 14.29.

### 3,5-dihydroxy-4,6,6-tris(3-methylbut-2-en-1-yl)-2-pentanoylcyclohexa-2,4-dien-1-one (AH18-F4)

According to the general procedure of prenylation, 1-(2,4,6-trihydroxyphenyl)pent-1-one was used. AH18-F4 was isolated as a light yellow oil (44 mg, 7%). **¹H NMR** (400 MHz, CDsOD) δ 5.02 (tsept, J = 7.0, 1.4 Hz, 1H), 4.76 (tsept, J = 6.0, 3.1 Hz, 2H), 3.09 (d, J = 7.0 Hz, 2H), 3.01 - 2.91 (m, 2H), 2.62 (td, J = 12.7, 11.9, 7.8 Hz, 4H), 1.73 (d, J = 1.3 Hz, 3H), 1.67 (d, J = 1.5 Hz, 3H), 1.62 - 1.50 (m, 12H), 1.44 - 1.34 (m, 2H), 0.94 (t, J = 7.3 Hz, 3H). **¹³C NMR** (126 MHz, CDsOD) δ 204.65, 198.12, 190.87, 174.41, 135.67, 132.58, 123.22, 119.21, 112.28, 109.04, 41.27, 38.77, 33.49, 28.59, 26.02, 23.72, 21.61, 18.09, 17.96, 14.30. **HRMS (ESI+)** *m*/*z* calcd for C₂₆H₃₈NaO₄ [M + Na]⁺ 437.2662, found 437.2654

### Synthesis of AH41-F1

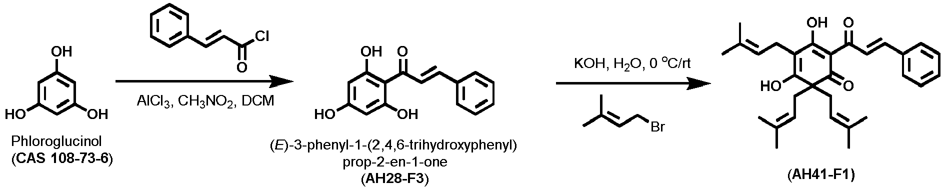

### (E)-3-phenyl-1-(2,4,6-trihydroxyphenyl)pent-1-one (AH28-F3) [9]

According to the general procedure of acylation A, cinnamoyl chloride was used. AH28-F3 was isolated as a yellow solid (625 mg, 31%). **¹H NMR** (400 MHz, CDsOD) δ 8.21 (d, J = 15.7 Hz, 1H), 7.71 (d, J = 15.7 Hz, 1H), 7.65 - 7.56 (m, 2H), 7.43 - 7.34 (m, 3H), 5.86 (s, 2H). **¹³C NMR** (101 MHz, CD₃OD) δ 193.98, 166.54, 166.10, 142.82, 137.02, 131.03, 129.93, 129.27, 128.91, 105.94, 96.02.

### 2-cinnamoyl-3,5-dihydroxy-4,6,6-tris(3-methylbut-2-en-1-yl)cyclohexa-2,4-dien-1-one (AH41-F1)

According to the general procedure of prenylation, (E)-3-phenyl-1-(2,4,6-trihydroxyphenyl)prop-2-en-1-one was used. AH41-F1 was isolated as a light yellow oil (54 mg, 20%). **¹H NMR** (400 MHz, CDsOD) δ 8.21 (d, J = 15.9 Hz, 1H), 7.76 (d, J = 15.8 Hz, 1H), 7.59 - 7.51 (m, 2H), 7.38 - 7.27 (m, 4H), 4.99 - 4.89 (m, 1H), 4.74 - 4.68 (m, 2H), 3.07 - 2.99 (m, 2H), 2.56 (tt, J = 21.3, 9.9 Hz, 4H), 1.65 (d, J = 1.3 Hz, 2H), 1.58 (d, J = 1.4 Hz, 2H), 1.49 (d, J = 1.4 Hz, 6H), 1.45 (d, J = 1.3 Hz, 6H). **¹³C NMR** (101 MHz, CD₃OD) δ 187.42, 136.82, 135.86, 132.56, 131.52, 130.06, 129.58, 125.04, 123.34, 119.28, 61.52, 38.48, 27.97, 26.04, 21.81, 18.10, 18.05, 18.02. **HRMS (ESI+)** *m*/*z* calcd for C₃₀H₃₆NaO₄ [M + Na]⁺ 483.2506, found 483.2502.

### Synthesis of AH44-F2

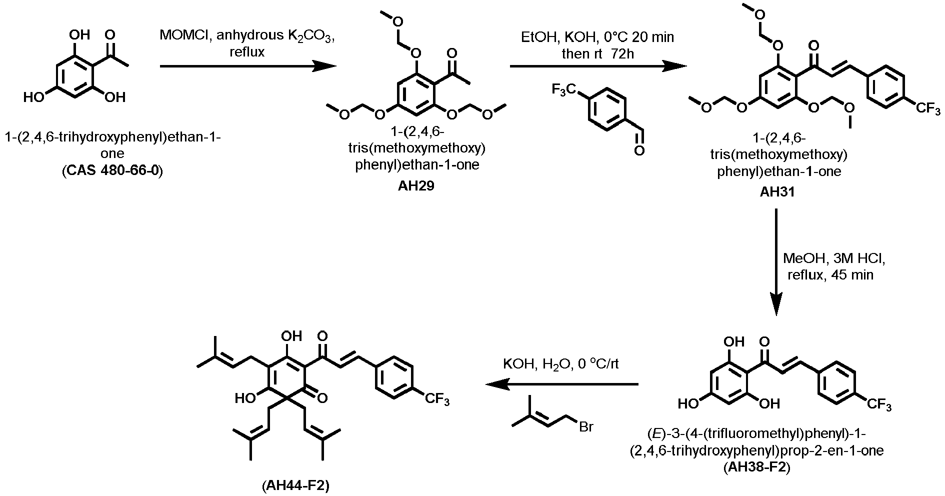

### 1-(2-hydroxy-4,6-bis(methoxymethoxy)phenyl)ethan-1-one (AH29) [10]

To a mixture of 1-(2,4,6-trihydroxyphenyl)ethan-1-one (1 equiv., 1.00 g, 5.95 mmol) and anhydrous K₂CO₃ (8 equiv., 6.58 g, 47.58 mmol) in dry acetone, chloromethyl methyl ether (2.5 eq, 1.2 g, 1.13 mL, 14.87 mmol) and the mixture was refluxed for 3 h. The mixture was cooled to room temperature and the solvent was evaporated. The product was extracted with DCM and the combined organic layers were washed with brine, dried over MgSO₄, filtered and concentrated under reduced pressure. The resulting crude was purified by chromatography on a silica gel column (cyclohexane/ethyl acetate: 9/1) to give AH29 1-[2-hydroxy-4,6-bis(methoxymethoxy)phenyl]ethan-1-one (840 mg, 55%) as a colourless oil. **¹H NMR** (400 MHz, CDCl₃) δ 13.71 (s, 1H), 6.25 (dd, *J* = 7.0, 2.4 Hz, 2H), 5.25 (s, 2H), 5.17 (s, 2H), 3.51 (s, 3H), 3.47 (s, 3H), 2.65 (s, 3H). **¹³C NMR** (101 MHz, CDCl₃) δ 203.35, 166.96, 163.58, 160.49, 107.08, 97.30, 94.61, 94.15, 56.85, 56.59, 33.15.

### (E)-1-(2-hydroxy-4,6-bis(methoxymethoxy)phenyl)-3-(4-(trifluoromethyl)phenyl)prop-2-en-1-one (AH31) [11]

To a stirred solution of 1-[2-hydroxy-4,6-bis(methoxymethoxy)phenyl]ethan-1-one (200 mg, 0.78 mmol, 1 equiv.) in EtOH (8 mL) cooled to 0 °C in an ice bath, a solution of KOH (438 mg, 7.8 mmol, 10 equiv.) in H₂O (1.56 mL) was added dropwise. After stirring for 20 min at 0 °C, 4-(trifluoromethyl)benzaldehyde (0.21 mL, 1.56 mmol, 2 equiv.) was added dropwise. After stirring for 20 min at 0 °C, the reaction was allowed to reach room temperature and stirred for 24 h. The mixture was poured into ice water, adjusted to pH 2-3 with 1 M HCl and then extracted with ethyl acetate. The organic layer was washed with water and saturated brine and dried over anhydrous MgSO_{4.} After removal of solvents, the products were purified by silica gel column chromatography (cyclohexane/EtOAc, 9:1). AH31 was isolated as a yellow powder (226 mg, 70%). **¹H NMR** (400 MHz, CDCl₃) δ 13.68 (s, 1H), 7.97 (d, J = 15.6 Hz, 1H), 7.75 (d, J = 15.7 Hz, 1H), 7.71 - 7.63 (m, 4H), 6.33 (d, J = 2.3 Hz, 1H), 6.25 (d, J = 2.3 Hz, 1H), 5.29 (s, 2H), 5.20 (s, 2H), 3.53 (s, 3H), 3.49 (s, 3H). **¹³C NMR** (101 MHz, CDCl₃) δ 192.64, 167.60, 164.00, 160.05, 140.27, 139.07, 139.05, 129.96, 128.43, 126.05, 126.01, 107.56, 97.67, 95.40, 94.95, 94.24, 57.09, 56.66.

### (E)-3-(4-(trifluoromethyl)phenyl)-1-(2,4,6-trihydroxyphenyl)prop-2-en-1-one (AH38-F2) [11]

To a stirred solution of *(E)-1-(2-hydroxy-4,6-bis(methoxymethoxy)phenyl)-3-(4-(trifluoromethyl)phenyl)prop-2-en-1-one* (520 mg, 1.26 mmol, 1 equiv.) in MeOH (17.4 mL) was added dropwise 3M HCl (6.95 mL, 16.53 equiv). The mixture was refluxed for 45 min. The solvents were removed under reduced pressure and the residue was diluted with water, and extracted with ethyl acetate. The organic layer was washed with water and brine, dried over anhydrous MgSO₄ and evaporated under reduced pressure. The residue was purified by silica gel column chromatography (cyclohexane/EtOAc 6:4) to give (E)-3-(4-(trifluoromethyl)phenyl)-1-(2,4,6-trihydroxyphenyl)prop-2-en-1-one (AH38-F2) as yellow powder (294 mg, 72%). **¹H NMR** (400 MHz, DMSO-d₆) δ 12.44 (s, 2H), 10.54 (s, 1H), 8.19 (d, J = 15.8 Hz, 1H), 7.88 (d, J = 8.1 Hz, 2H), 7.80 (d, J = 8.2 Hz, 2H), 7.71 (d, J = 15.8 Hz, 1H), 5.87 (s, 2H). **¹³C NMR** (101 MHz, DMSO-d₆) δ 191.41, 165.48, 164.54, 139.19, 139.09, 130.31, 128.74, 125.93, 125.89, 125.85, 125.81, 104.40, 94.95.

### (E)-3,5-dihydroxy-4,6,6-tris(3-methylbut-2-en-1-yl)-2-(3-(4-(trifluoromethyl)phenyl)acryloyl)cyclohexa-2,4-dien-1-one (AH44-F2)

According to the general procedure of prenylation, (E)-3-(4-(trifluoromethyl)phenyl)-1-(2,4,6-trihydroxyphenyl)prop-2-en-1-one was used. AH44-F2 was isolated as a light yellow oil (26 mg, 16%). **¹H NMR** (400 MHz, CD₃OD) δ 8.37 (d, J = 15.9 Hz, 1H), 7.94 - 7.77 (m, 3H), 7.73 (d, J = 8.2 Hz, 2H), 5.08 - 4.99 (m, 1H), 4.84 - 4.77 (m, 2H), 3.13 (d, J = 6.9 Hz, 2H), 2.81 - 2.53 (m, 4H), 1.78 - 1.73 (m, 3H), 1.68 (s, 3H), 1.59 (d, J = 1.4 Hz, 6H), 1.57 - 1.53 (m, 6H). **¹³C NMR** (126 MHz, CDsOD) δ 187.18, 140.63, 135.98, 132.67, 130.18, 130.00, 129.85, 128.15, 126.93, 126.90, 123.49, 119.20, 118.89, 118.44, 26.02, 18.08. **HRMS (ESI+)** *m*/*z* calcd for C₃₁H₃₅NaF₃O₄ [M + Na]⁺ 551.2380, found 551.2372.

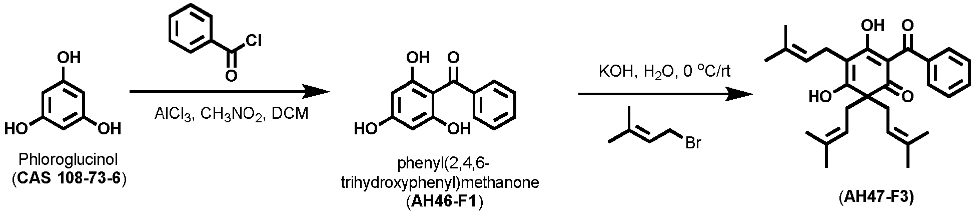

### Phenyl(2,4,6-trihydroxyphenyl)methanone (AH46-F1) [12]

According to the general procedure of acylation A, benzoyl chloride was used. AH46-F1 was isolated as a light yellow solid (463 mg, 25%). **¹H NMR** (400 MHz, CD₃OD) δ 7.61 - 7.55 (m, 2H), 7.50 - 7.43 (m, 1H), 7.41 - 7.34 (m, 2H), 5.86 (s, 2H). **¹³C NMR** (101 MHz, CD₃OD) δ 200.62, 165.94, 163.84, 142.92, 132.06, 129.31, 128.63, 105.87, 95.81.

### 2-benzoyl-3,5-dihydroxy-4,6,6-tris(3-methylbut-2-en-1-yl)cyclohexa-2,4-dien-1-one (AH47-F3) [13]

According to the general procedure of prenylation, phenyl(2,4,6-trihydroxyphenyl)methanone was used. AH47-F3 was isolated as a light yellow oil (19 mg, 4%). **¹H NMR** (400 MHz, CD₃OD) δ 7.50 - 7.30 (m, 5H), 5.07 (td, *J* = 7.0, 6.2, 3.7 Hz, 1H), 4.91 - 4.85 (m, 2H), 3.20 - 3.09 (m, 2H), 2.70 - 2.48 (m, 4H), 1.75 (s, 3H), 1.70 (s, 3H), 1.64 - 1.62 (m, 6H), 1.61 - 1.59 (m, 6H). **¹³C NMR** (101 MHz, CD₃OD) δ 197.44, 134.61, 131.41, 127.47, 127.26, 121.73, 117.85, 26.58, 24.71, 24.63, 20.39, 16.77, 16.59.

### Example 10 (Synthesis of Geranylated Analogues of Beta-Acids)

### Synthesis of Geranylated Analogue of Lupulone

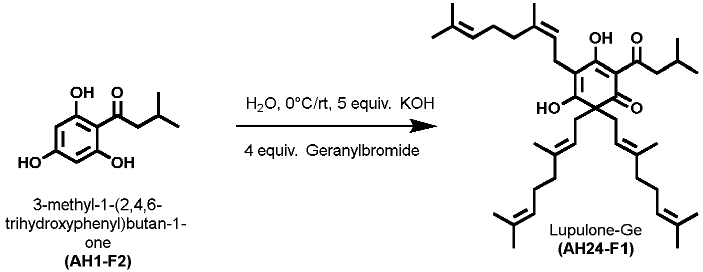

### 2-methyl-1-(2,4,6-trihydroxyphenyl)propan-1-one (AH24-F1) [14]

According to the general procedure of geranylation, 3-methyl-1-(2,4,6-trihydroxyphenyl)butan-1-one was used (AH1-F2). AH24-F1 was isolated as a colourless liquid (9.5 mg, 3%). **¹H NMR** (400 MHz, CDCl₃) δ 5.69 - 5.52 (m, 1H), 5.49 - 5.31 (m, 3H), 2.79 - 2.68 (m, 3H), 2.65 - 2.51 (m, 2H), 2.37 - 2.20 (m, 4H), 2.19 - 1.86 (m, 9H), 1.86- 1.57 (m, 27H), 1.07 - 0.93 (m, 9H), 0.93 - 0.77 (m, 3H). **¹³C NMR** (101 MHz, CDCl₃) δ 150.45, 142.69, 140.81, 134.40, 133.92, 133.47, 133.38, 131.45, 127.75, 124.90, 121.88, 120.93, 120.79, 119.61, 119.02, 116.51, 116.14, 108.50, 41.25, 34.69, 34.64, 31.79, 31.61, 30.97, 30.76, 29.68, 29.14, 28.08, 27.70, 26.78, 25.42, 23.63, 23.59, 23.15, 23.08, 21.46, 21.36, 20.98, 20.36, 19.89.

### Synthesis of Geranylated Analogue of Co-Lupulone

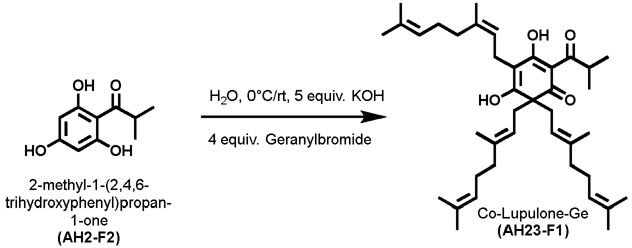

### 2-methyl-1-(2,4,6-trihydroxyphenyl)propan-1-one (AH23-F1)

According to the general procedure of geranylation, 2-methyl-1-(2,4,6-trihydroxyphenyl)propan-1-one (AH2-F2) was used. AH23-F1 was isolated as a colourless liquid (13 mg, 4%). **¹H NMR** (400 MHz, CDCl₃) δ 5.61 - 5.42 (m, 1H), 5.40 - 5.27 (m, 3H), 4.69 - 4.58 (m, 2H), 2.70 - 2.63 (m, 2H), 2.57 - 2.47 (m, 2H), 2.30 - 2.20 (m, 3H), 2.10 - 1.78 (m, 8H), 1.78 - 1.41 (m, 26H), 1.31 - 1.11 (m, 4H), 1.00 - 0.89 (m, 9H). **¹³C NMR** (101 MHz, CDCl₃) δ 150.45, 142.69, 140.80, 134.40, 133.92, 133.47, 133.38, 131.45, 127.75, 124.90, 121.87, 120.93, 120.80, 119.61, 119.02, 116.51, 116.14, 108.51, 41.25, 34.65, 34.29, 31.79, 31.65, 31.61, 30.97, 30.76, 29.68, 29.14, 28.08, 27.70, 26.78, 25.42, 23.63, 23.59, 23.15, 22.50, 21.46, 21.36, 20.98, 20.36, 19.89.

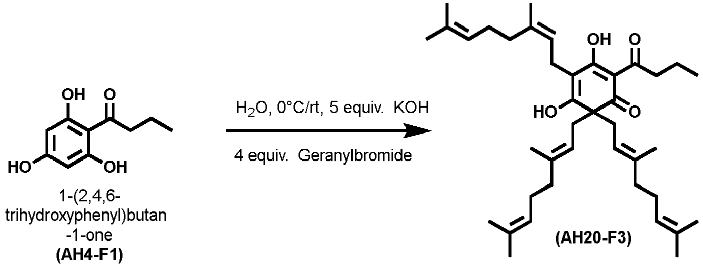

### 2-methyl-1-(2,4,6-trihydroxyphenyl)propan-1-one (AH20-F3)

According to the general procedure of geranylation, 1-(2,4,6-trihydroxyphenyl)butan-1-one (AH4-F1) was used. AH20-F3 was isolated as a colourless liquid (36 mg, 12%). **¹H NMR** (400 MHz, CDCl₃) δ 5.03 (tddd, J = 8.5, 6.9, 4.2, 2.7 Hz, 3H), 4.93 (h, J = 3.6 Hz, 3H), 2.94 - 2.86 (m, 2H), 2.63 (dd, J = 13.9, 7.8 Hz, 2H), 2.52 (dt, J = 14.8, 7.8 Hz, 3H), 2.33 (td, J = 15.1, 14.7, 6.9 Hz, 2H), 2.13 - 1.84 (m, 16H), 1.70 - 1.62 (m, 16H), 1.60 - 1.50 (m, 24H), 0.99 (t, J = 7.4 Hz, 3H). **¹³C NMR** (101 MHz, CDCl₃) δ 139.83, 138.48, 131.49, 131.42, 124.10, 124.00, 123.52, 120.52, 118.72, 118.11, 65.53, 61.08, 41.22, 40.09, 40.02, 36.17, 34.27, 33.88, 29.71, 26.65, 26.57, 25.66, 25.65, 18.42, 17.63, 16.24, 16.15, 13.94. **HRMS (ESI+)** *m*/*z* calcd for C₄₀H₆₁O₄ [M + H]⁺ 605.4564, found 605.4568.

### Example 11 (Synthesis of Acetylated Protected Natural Beta-Acids)

### Synthesis of Acetylated Protected Analogue of Lupulone

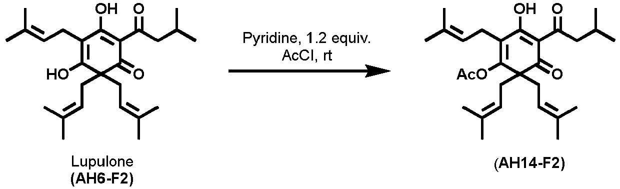

### 3-hydroxy-2,6,6-tris(3-methylbut-2-en-1-yl)-4-(3-methylbutanoyl)-5-oxocyclohexa-1,3-dien-1-yl acetate (AH14-F2)

According to the general procedure of acetylation, 3,5-dihydroxy-4,6,6-tris(3-methylbut-2-en-1-yl)-2-(3-methylbutanoyl)cyclohexa-2,4-dien-1-one was used. AH14-F2 was isolated as a colourless liquid (25 mg, 25%). **¹H NMR** (400 MHz, CD₃OD) δ 5.06 - 4.94 (m, 1H), 4.90 (d, J = 8.5 Hz, 2H), 2.39 (ddd, J = 48.1, 14.9, 7.6 Hz, 2H), 2.28 (d, J = 3.1 Hz, 3H), 2.06 (dq, J = 13.3, 6.7 Hz, 1H), 1.68 (d, J = 6.0 Hz, 6H), 1.61 - 1.51 (m, 12H), 0.95 (dd, J = 6.8, 3.9 Hz, 6H). **¹³C NMR** (101 MHz, CD₃OD) δ 206.67, 204.35, 197.46, 197.15, 167.86, 167.78, 136.32, 135.67, 133.93, 133.27, 131.58, 127.53, 122.14, 121.58, 119.55, 119.04, 114.33, 111.42, 54.81, 38.20, 36.92, 27.37, 27.26, 26.05, 25.97, 24.68, 24.35, 23.07, 22.95, 20.68, 18.14, 18.07. **HRMS (ESI+)** *m*/*z* calcd for C₂₈H₄₀O₅ [M + H]⁺ 457.2948, found 457.2946.

### Synthesis of Acetylated Protected Analogue of Co-Lupulone

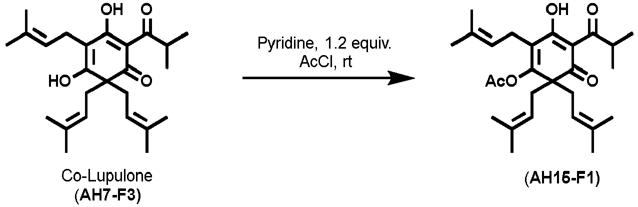

### 3,5-dihydroxy-2-isobutyryl-4,6,6-tris(3-methylbut-2-en-1-yl)cyclohexa-2,4-dien-1-one (AH15-F1)

According to the general procedure of acetylation, 3,5-dihydroxy-2-isobutyryl-4,6,6-tris(3-methylbut-2-en-1-yl)cyclohexa-2,4-dien-1-one was used. AH15-F1 was isolated as a colourless liquid (22 mg, 21%). **¹H NMR** (400 MHz, CD₃OD) δ 5.07 - 4.93 (m, 1H), 4.89 (td, J = 7.6, 6.5, 1.5 Hz, 2H), 4.13 - 3.90 (m, 1H), 2.97 (dd, J = 22.4, 7.0 Hz, 2H), 2.72 (dt, J = 34.2, 11.5 Hz, 2H), 2.53 - 2.30 (m, 2H), 2.28 (s, 3H), 1.68 (s, 6H), 1.61 - 1.51 (m, 12H), 1.11 (d, J = 6.8 Hz, 6H). **¹³C NMR** (101 MHz, CD₃OD) δ 206.67, 204.35, 197.46, 197.15, 167.86, 167.78, 136.32, 135.67, 133.93, 133.27, 131.58, 127.53, 122.14, 121.58, 119.55, 119.04, 114.33, 111.42, 54.81, 38.20, 36.92, 27.37, 27.26, 26.05, 25.97, 24.68, 24.35, 23.07, 22.95, 20.68, 18.14, 18.07. **HRMS (ESI+)** *m*/*z* calcd for C₂₇H₃₈O₅ [M + H]⁺ 443.2792, found 443.2793.

### Example 12 (Synthesis of Prenylated Phloroglucinols)

### Synthesis of Prenylated Phloroglucinols

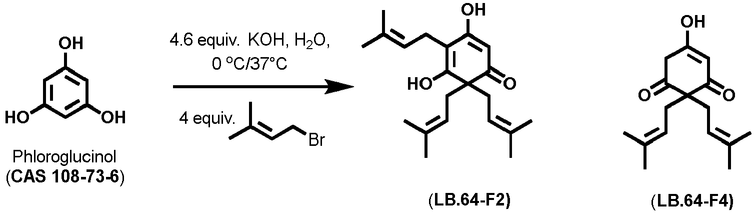

In a round-bottomed flask equipped with a stirrer, potassium hydroxide (1.02 g, 18.24 mmol, 4.6 equiv.) was added to a suspension of phloroglucinol (0.5 g, 3.96 mmol, 1 equiv.) in water (40 mL) at room temperature. The reaction mixture was stirred for 5 min, during which it became a transparent violet solution. At this point, prenyl bromide (2.36 g, 1.86 mL, 15.84 mmol, 4 equiv.) was added dropwise, causing a temporary white precipitate. After stirring for 60 min at room temperature, the temperature was raised to 37 °C and the reaction mixture was stirred for another 60 min. The reaction mixture was then acidified with 1N aqueous HCl and extracted with diethyl ether. The combined organic layers were washed with brine, dried with MgSO4, filtered and concentrated under reduced pressure. The residue was purified by column chromatography on silica gel in cyclohexane/EtOAc 9:1 to 5:5 (v/v).

### 5-hydroxy-2,2,4-tris(3-methylbut-2-en-1-yl)cyclohex-4-ene-1,3-dione (LB.64-F2)

After 3 column chromatographies LB.64-F2 was isolated as a pale yellow oil (400 mg, 31 %). **¹H NMR** (400 MHz, CDCl₃) δ 5.17 (t, *J* = 7.4 Hz, 1H), 4.88 - 4.83 (m, 2H), 3.23 (s, 2H), 3.19 (d, *J* = 7.4 Hz, 2H), 2.63 - 2.56 (m, 2H), 2.53 - 2.46 (m, 2H), 1.81 - 1.77 (m, 6H), 1.62 (s, 6H), 1.57 (s, 6H). **¹³C NMR** (101 MHz, CDCl₃) δ 206.59, 154.16, 153.33, 137.73, 135.76, 122.46, 121.16, 118.35, 117.56, 56.39, 35.90, 26.08, 26.00, 22.18, 18.08, 17.98. **HRMS (ESI+)** *m*/*z* calcd for C₂₁H₃₀O₃ [M + H]⁺ 331.2268, found 331.2267.

### 5-hydroxy-2,2-bis(3-methylbut-2-en-1-yl)cyclohex-4-ene-1,3-dione (LB.64-F4)

After column chromatography purification, the desired product (yellow oil) was washed repeatedly with ethyl acetate to form solid, which was recrystallized from methanol. LB.64-F4 was isolated as pale yellow crystals (70 mg, 7 %). **MP** 157-158 °C. **¹H NMR** (400 MHz, CDCl₃) δ 7.97 (br s, 1H), 5.90 (s, 1H), 4.93 - 4.85 (m, 2H), 3.22 (s, 2H), 2.64 (dd, *J* = 13.9, 8.2 Hz, 2H), 2.53 (dd, *J* = 14.0, 7.1 Hz, 2H), 1.62 (s, 6H), 1.58 (s, 6H). **¹³C NMR** (126 MHz, CDCl₃) δ 206.81, 191.14, 185.51, 136.18, 117.96, 107.09, 61.18, 47.88, 35.88, 26.04, 17.98. **HRMS (ESI+)** *m*/*z* calcd for C₁₆H₂₃O₃ [M + H]⁺ 263.1642, found 263.1639.

### Example 13 (Synthesis of Diacylated Prenylated Phloroglucinols)

### Synthesis of Diacylated Phloroglucinols

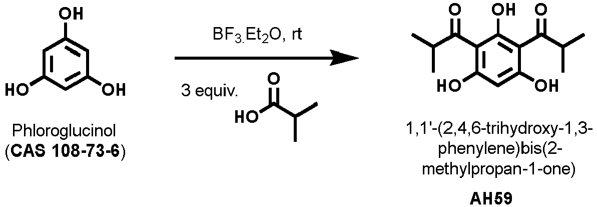

### 1,1'-(2,4,6-trihydroxy-1,3-phenylene)bis(2-methylpropan-1-one) (AH59) [15]

Isobutyric acid (1.05 g, 11.89 mmol, 3 equiv.) was dissolved in excess of BF₃-Et₂O (5.63 g, 4.89 mL, 39.65 mmol, 10 equiv.) at room temperature. After 10 min, phloroglucinol (0.50 g, 3.96 mmol, 1 equiv.) was added in portions and the resulting suspension was refluxed for 3 h. The reaction mixture was cooled to room temperature, quenched by pouring into ice-cold water (100 mL) and extracted with EtOAc (2 × 50 mL). The organic layers were successively washed with saturated NaHCOs (2 × 50 mL) and brine (2 × 50 mL). After drying over MgSO₄, the solvent was removed under reduced pressure and the crude product was purified by column chromatography on silica gel, c-Hex/EtOAc 8:2. AH59 was isolated as a pale yellow oil (350 mg, 33%). **¹H NMR** (400 MHz, CD₃OD) δ 5.85 (s, 1H), 3.96 (hept, J = 6.7 Hz, 2H), 1.15 (d, J = 6.7 Hz, 12H). **¹³C NMR** (101 MHz, CD₃OD) δ 212.36, 172.96, 172.54, 169.87, 104.17, 96.05, 27.98, 19.56.

### Synthesis of Diacylated Monoprenylated Phloroglucinol

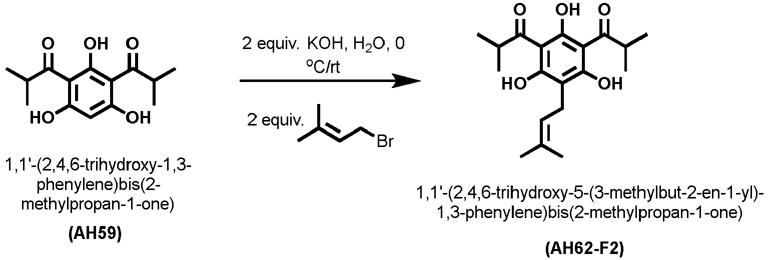

### 2-methyl-1-(2,4,6-trihydroxy-3-(3-methylbut-2-en-1-yl)phenyl)propan-1-one (AH62-F2)

To a stirred solution of 1,1'-(2,4,6-trihydroxy-1,3-phenylene)*bis*(2-methylpropan-1-one) (0.10 g, 0.38 mmol, 1 equiv.) in water (1 mL) under an argon atmosphere at 0 °C, potassium hydroxide (42 mg, 0.75 mmol, 2 equiv.) was added in one portion. The reaction mixture immediately turned yellow, at which point prenyl bromide (112 mg, 0.088 mL, 0.75 mmol, 2 equiv.) was added dropwise over several minutes. The reaction was allowed to reach room temperature and stirred for 2 hours. The reaction mixture was then acidified with aqueous 1N HCl and extracted with EtOAc. The combined organic layers were washed with brine, dried over MgSO₄, filtered and concentrated under reduced pressure. The residue was purified by column chromatography on silica gel in cyclohexane/EtOAc 99:1 (v/v). AH62-F2 was isolated as a pale yellow oil (45 mg, 36%). **¹H NMR** (400 MHz, CD₃OD) δ 5.07 (dddd, J = 8.3, 5.4, 2.9, 1.4 Hz, 1H), 3.99 (p, J = 6.7 Hz, 2H), 3.30 - 3.26 (m, 2H), 1.76 (d, J = 1.2 Hz, 3H), 1.67 (t, J = 1.4 Hz, 3H), 1.17 (dd, J = 6.7 Hz, 12H). **¹³C NMR** (101 MHz, CD₃OD) δ 212.79, 169.93, 167.17, 133.39, 122.84, 108.10, 104.55, 40.65, 25.93, 21.78, 19.73, 17.99. **HRMS (ESI+)** *m*/*z* calcd for C₁₉H₂₅O₅ [M - H]⁻ 333.1707, found 333.1698.

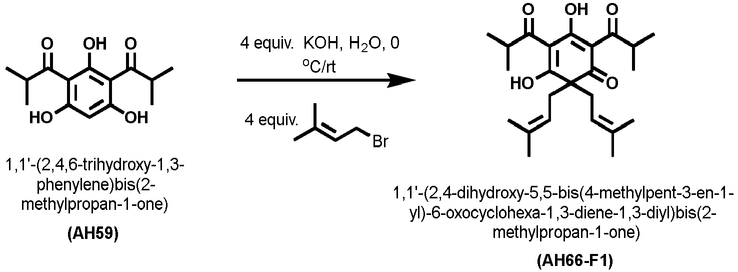

### 1,1'-(2,4-dihydroxy-5,5-bis(3-methylbut-2-en-1-yl)-6-oxocyclohexa-1,3-diene-1,3-diyl)bis(2-methylpropan-1-one) (AH66-F1)

To a stirred solution of 1,1'-(2,4,6-trihydroxy-1,3-phenylene)*bis*(2-methylpropan-1-one) (0.10 g, 0.38 mmol, 1 equiv.) in water (1 mL) under an argon atmosphere at 0 °C, potassium hydroxide (84.3 mg, 1.50 mmol, 4 equiv.) was added in one portion. The reaction mixture immediately turned yellow, at which point prenyl bromide (224 mg, 0.18 mmol, 4 equiv.) was added dropwise over several minutes. The reaction was allowed to reach room temperature and stirred for 2 hours. The reaction mixture was then acidified with aqueous 1N HCl and extracted with EtOAc. The combined organic layers were washed with brine, dried over MgSO₄, filtered and concentrated under reduced pressure. The residue was purified by column chromatography on silica gel in cyclohexane/EtOAc 99:1 (v:v). AH66-F1 was isolated as a pale yellow oil (37 mg, 24%). **¹H NMR** (400 MHz, CD₃OD) δ 4.81 (t, J = 8.0 Hz, 2H), 4.07 - 3.94 (m, 2H), 2.86 - 2.59 (m, 4H), 1.59 - 1.50 (m, 12H), 1.22 - 1.09 (m, 12H). **¹³C NMR** (101 MHz, CD₃OD) δ 137.60, 136.82, 118.90, 118.32, 61.65, 39.20, 38.04, 27.98, 25.99, 19.74, 19.37, 17.98. **HRMS (ESI+)** *m*/*z* calcd for C₂₄H₃₄NaO₅ [M + Na]⁻ 425.2299, found 425.2287.

### Example 14 (Synthesis of a Fluorescent Analogue of Beta-Acid)

### Synthesis of a Fluorescent Analogue of Beta-Acid

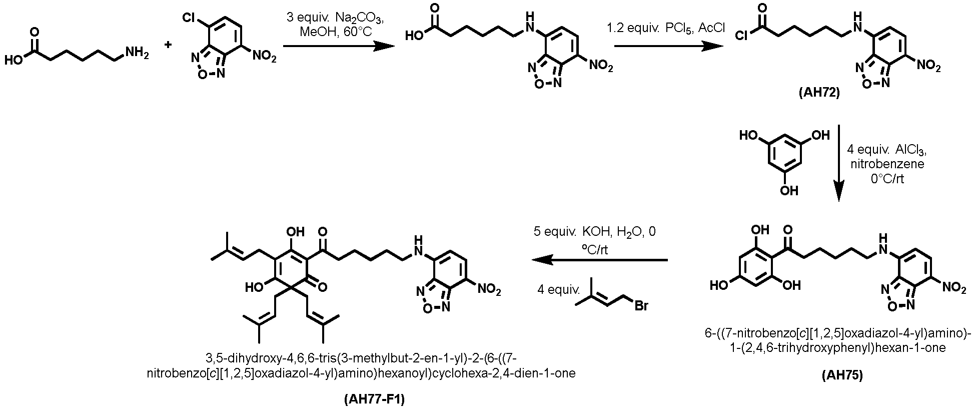

### 6-((7-nitrobenzo[c][1,2,5]oxadiazol-4-yl)amino)hexanoic acid [16]

Sodium bicarbonate (2.58 g, 30.7 mmol, 3 equiv.) and 6-aminohexanoic acid (2.64 g, 20.1 mmol, 2 equiv.) were added to H₂O (20 mL). 4-chloro-7-nitrobenzofurazan (2.04 g, 10.2 mmol, 1 equiv.) dissolved in MeOH (80 mL) was added and the mixture was stirred at 60 °C for 30 min. The solution was cooled in ice, acidified to pH 2.0 with concentrated HCl, and the MeOH was removed by rotary evaporation. Excess water (200 mL) was added and the suspension was homogeneously dispersed by bath sonication. A fine black powder was collected by filtration, washed with water, and dried under vacuum to give the desired compound as a white solid product. 6-((7-Nitrobenzo[c][1,2,5]oxadiazol-4-yl)amino)hexanoic acid was isolated with 2.63 g as a black solid (88% yield) in good purity. **¹H NMR** (500 MHz, DMSO-*d₆*) δ 9.49 (t, J = 6.0 Hz, 1H), 8.45 (d, J = 8.9 Hz, 1H), 6.36 (d, J = 9.0 Hz, 1H), 3.43 (q, J = 6.8 Hz, 2H), 2.21 (t, J = 7.3 Hz, 2H), 1,67 (p, J = 7.4 Hz, 2H), 1.54 (p, J = 7.4 Hz, 2H), 1,42 - 1,32 (m, 2H). **¹³C NMR** (126 MHz, DMSO-*d₆*) δ 174.44, 145.18, 144.44, 137.91, 120.55, 99.07, 43.25, 33.57, 27.36, 25.98, 24.18.

### 6-((7-nitrobenzo[c][1,2,5]oxadiazol-4-yl)amino)hexanoyl chloride (AH72) [17]

To a suspension of 6-[(7-nitro-2,1,3-benzoxadiazol-4-yl)amino]hexanoic acid (1.00 g, 3.4 mmol, 1 equiv.) in acetyl chloride (8.6 mL) under stirring, PCl₅ (0.86 g, 4.12 mmol, 1.2 equiv.) was added slowly. The reaction medium is slowly heated to 50°C and became clear on reflux. The reaction medium was cooled with an ice bath and then 60 mL of petroleum ether was added to the flask before being placed at -18°C for a day from which pure brown powder crystallized. It was filtered, washed with petroleum ether and dried to afford 680 mg of 6-((7-nitrobenzo[c][1,2,5]oxadiazol-4-yl)amino)hexanoyl chloride as a brown solid AH72 (64% yield). **¹H NMR** (500 MHz, CDCl₃) δ 8.46 (d, *J* = 8.6 Hz, 1H), 6.39 (s, 1H), 6.18 (d, *J* = 8.9 Hz, 1H), 3.54 (t, *J* = 7.2 Hz, 2H), 2.96 (t, *J* = 7.1 Hz, 2H), 1.92 - 1.76 (m, 4H), 1.63 - 1.49 (m, 2H). **¹³C NMR** (126 MHz, CDCl₃) δ 173.80, 144.35, 143.97, 136.78, 136.66, 124.07, 98.82, 77.16, 46.86, 43.78, 28.24, 25.90, 24.74.

### 6-((7-nitrobenzo[c][1,2,5]oxadiazol-4-yl)amino)-1-(2,4,6-trihydroxyphenyl)hexan-1-one (AH75)

AlCl₃ (1.68 g, 12.59 mmol, 4 equiv.) was slowly and carefully added to a solution of phloroglucinol (397 mg, 3.15 mmol, 1 equiv.) in nitrobenzene (12.59 mL) at 0 °C. After stirring at this temperature for 10 min under nitrogen atmosphere, acid chloride 6-[(7-nitro-2,1,3-benzoxadiazol-4-yl)amino]hexanoyl chloride (984 mg, 3.15 mmol, 1 equiv.) was added. Then, the ice bath was removed, and the mixture was stirred at room temperature for 16 h. The crude reaction mixture was quenched with water (25 mL). The mixture was extracted with EtOAc (5 × 25 mL), washed with brine, and concentrated *in vacuum.* The nitrobenzene was removed by filtration through silica (cyclohexane then EtOAc). The residue was purified by column chromatography on silica gel in cyclohexane/EtOAc 50:50 - 0:100 (v/v). AH75 was isolated as an orange powder (493 mg, 39%). **¹H NMR** (400 MHz, DMSO-*d₆*) δ 12.21 (s, 2H), 10.32 (s, 1H), 9.54 (t, J = 5.8 Hz, 1H), 8.50 (d, J = 8.9 Hz, 1H), 6.40 (d, J = 9.0 Hz, 1H), 5.79 (s, 2H), 3.47 (d, J = 7.3 Hz, 2H), 3.00 (t, J = 7.3 Hz, 2H), 1.78 - 1.55 (m, 4H), 1.51 - 1.32 (m, 2H). **¹³C NMR** (101 MHz, DMSO-*d₆*) δ 204.97, 170.31, 164.50, 164.16, 158.88, 145.16, 144.42, 144.15, 137.93, 120.51, 103.72, 99.05, 94.61, 94.04, 59.74, 43.26, 42.88, 39.52, 27.57, 26.22, 23.97, 20.75, 14.08.

### 3,5-dihydroxy-4,6,6-tris(3-methylbut-2-en-1-yl)-2-(6-((7-nitrobenzo[c][1,2,5]oxadiazol-4-yl)amino)hexanoyl)cyclohexa-2,4-dien-1-one (AH77-F1)

To a stirred solution of 6-[(7-nitro-2,1,3-benzoxadiazol-4-yl)amino]-1-(2,4,6-trihydroxyphenyl)hexan-1-one (150 mg, 0.37 mmol, 1 equiv.) in water (3.69 mL) under an argon atmosphere at 0 °C, potassium hydroxide (105 mg, 1.86 mmol, 5 equiv.) was added in one portion. The reaction mixture immediately turned yellow. At this point, prenyl bromide (222 mg, 0.17 mL, 1.49 mmol, 4 equiv.) was added dropwise over several minutes until the reaction mixture became cloudy. The reaction was then allowed to reach room temperature and stirred for 2 hours. The reaction mixture was then acidified with an aqueous solution of 1 N HCl and extracted with EtOAc. The combined organic layers were washed with brine, dried over MgSO₄, filtered and concentrated under reduced pressure. The crude was purified by column chromatography on silica gel (cHex/EtOAc 98:2). Since separation was difficult (diprenylated by-product in the order of a few %), the fractions enriched in the desired product were then purified by preparative thin-layer chromatography (toluene/EtOAc 7:3 v/v) to give 23 mg of pure compound AH77-F1 as an orange powder in 10 % yield. **¹H NMR** (500 MHz, CD₃OD) δ 8.50 (d, J = 7.2 Hz, 1H), 6.36 (d, J = 7.1 Hz, 1H), 5.02 (br t, J = 4.6 Hz, 1H), 4.76 (br t, J = 6 Hz, 0.9 Hz, 2H), 3.55 (br s, 2H), 3.05 (d, J = 5.5 Hz, 2H), 2.96 (br t, 2H), 2.55 (br t, 4H), 1.82 (m, 2H), 1.72 (s, 3H), 1.65 (s, 3H), 1.55 - 1.52 (m, 12H), 1.35-1.15 (m, 4H). **¹³C NMR** (126 MHz, CD₃OD) δ 203.37, 198.89, 188.30, 146.70, 145.88, 145.54, 138.60, 135.65, 135.05, 132.78, 131.79, 124.06, 122.93, 119.80, 111.29, 108.39, 99.61, 61.53, 59.64, 44.63, 41.61, 39.06, 30.75, 28.99, 27.93, 26.15, 26.03, 21.74, 18.12, 17.97, 17.81, 14.45. **HRMS (ESI+)** m/z calcd for C₃₃H₄₂NaN₄O₇ [M + Na]⁺ 629.2946, found 629.2935.

### Example 15 (Antiviral Activity against Human Coronaviruses In Vitro)

Antiviral activity of natural and synthetic compounds was evaluated against four human coronaviruses. Three highly pathogenic coronaviruses were tested, severe acute respiratory syndrome coronavirus (SARS-CoV), responsible of 2002-2003 outbreak, Middle-East respiratory syndrome coronavirus (MERS-CoV), identified in 2012, high mortality rate of 35%, and still ongoing in Saudi Arabia, and SARS-CoV-2, responsible of the COVID-19 pandemic, 6.9 million of reported deaths (sept 2023). A mild coronavirus was also evaluated, human coronavirus 229E, HCoV-229E, responsible of common cold.

### SARS-CoV-2 antiviral assay

Dose-response experiments were conducted to determine half-maximal inhibitory concentration, IC₅₀ of the compounds on severe acute respiratory syndrome coronavirus-2 (SARS-CoV-2). Vero81-derived F1G-red cells [18] seeded in 384-well plate were inoculated with SARS-CoV-2 at a multiplicity of infection (MOI) of 0.2, at 37°C, in the presence of each compound at 1.56, 3.13, 6.25, 12.5 and 25 µM. Images were acquired 16 h later using an InCell-6500 automated confocal microscope (Cytiva, Marlborough, United States). Infected cells and total cell numbers were quantified as previously described [18]. Percentage of infection relative to the DMSO controls were obtained and IC₅₀ were calculated using Prism 10 software (Graph Pad Inc., La Jolla, CA, USA). IC₅₀ are presented in Table 2. The compounds were classified in 4 categories depending on their IC50, +++: IC₅₀ < 7 µM; ++: IC₅₀ < 14 µM; +: IC₅₀ < 25 µM, -: IC₅₀ > 25 µM. The most active compounds were natural lupulone and colupulone, synthetic colupulone and synthetic diacylated monoprenylated colupulone (AH62-F2).

**Table 2. IC₅₀ of pure and synthetic compounds on SARS-CoV-2**

| **code** | **name** | **Formula** | **IC₅₀*** | **According to the invention (Y/N)** |
|---|---|---|---|---|
| (natural) | Colupulone | I, II, V | +++ | Y |
| LB.70-F2 | Synthetic colupulone | I, II, V | +++ | Y |
| (natural) | Lupulone | I, II, V | +++ | Y |
| AH8-F2 | Synthetic adlupulone | I, II, V | + | Y |
| AH39-F1 | Synthetic prelupulone | I, II, V | + | Y |
| AH16-F2 | Synthetic acetolupulone | I, II, V | - | Y |
| AH17-F2 | Synthetic postlupulone | I, II, V | - | Y |
| AH40-F1 | Synthetic adprelupulone | I, II, V | - | Y |
| AH10-F2 | | I, II, V | ++ | Y |
| AH41-F1 | | I, II, V | + | Y |
| AH18-F4 | | I, II, V | ++ | Y |
| AH15-F1 | 5-acetyl colupulone | I, II, V | ++ | Y |
| AH62-F2 | diacylated monoprenylated Colupulone | I, III, X | +++ | Y |
| AH66-F1 | Diprenylated diacylated colupulone | I, II, IX | + | Y |

| | | | | |
|---|---|---|---|---|
| *+++: IC₅₀ < 7 µM; ++: IC₅₀ < 14 µM; +: IC₅₀ < 25 µM, -: IC₅₀ > 25 µM. | | | | |

### HCoV-229E antiviral assay

Dose-response experiments were conducted to determine IC₅₀ of the compounds on Human coronavirus 229E (HCoV-229E). A recombinant HCoV-229E expressing the luciferase reporter gene was used (HCoV-229E-Luc). On the day before infection, 6 × 10⁴ Huh-7/TMPRSS2 cells were seeded in 96-well plates at 37 °C. The cells were inoculated with HCoV-229E-Luc at a MOI of 0.5 in a final volume of 50 µL for 1 h at 37 °C in the presence of each compound at 1.56, 3.13, 6.25, 12.5 and 25 µM, or extracts at concentrations between 0.1 to 50 µg/mL. The virus was removed and replaced with culture medium containing the different compounds for 6 h at 37 °C. The cells were lysed in 20 µL of Renilla Lysis Buffer (Promega, Madison, WI, USA), and luciferase activity was quantified using a Tristar LB 941 luminometer (Berthold Technologies, Bad Wildbad, Germany) with the Renilla Luciferase Assay System (Promega) as recommended by the manufacturer. Luciferase activity was calculated relative to the control, DMSO, for which a value of 100 was attributed. IC₅₀ were calculated using Prism 10 software. IC₅₀ are presented in Table 3, Table 4 and Table 5. The compounds were classified in 4 categories depending on their IC50, +++: IC₅₀ < 3 µM; ++: IC₅₀ < 12 µM; +: IC₅₀ < 24 µM, -: IC₅₀ > 24 µM. The most active compounds were natural lupulone and colupulone, synthetic lupulone and colupulone, synthetic diacylated monoprenylated colupulone (AH62-F2) and synthetic AH10-F2 (Table 3). The results presented in Table 4 showed that *Humulus lupulus* extract beta-acid fraction containing a mixture of lupulone, colupulone and ad-lupulone is the most active of the extracts. The results presented in Table 5 showed that the more the extracts are rich in beta-acids (lupulone, colupulone, ad-lupulone) the more they are active.

**Table 3. IC₅₀ of pure and synthetic compounds on HCoV-229E**

| **Code** | **Name** | **Formula** | **IC₅₀*** | **According to the invention (Y/N)** |
|---|---|---|---|---|
| (natural) | Lupulone | I, II, V | +++ | Y |
| AH6-F2 | Synthetic lupulone | I, II, V | +++ | Y |
| (natural) | Humulone | | - | N |
| (natural) | Co-lupulone | I, II, V | +++ | Y |
| LB.70-F2 | Synthetic co-lupulone | I, II, V | +++ | Y |
| (natural) | Co-humulone | | - | N |
| (natural) | Ad-lupulone | I, II, V | ++ | Y |
| AH8-F2 | Synthetic ad-lupulone | I, II, V | ++ | Y |
| AH17-F2 | Synthetic post-lupulone | I, II, V | - | Y |
| AH16-F2 | Synthetic aceto-lupulone | I, II, V | ++ | Y |
| AH39-F1 | Synthetic pre-lupulone | I, II, V | ++ | Y |
| AH40-F1 | Synthetic ad-prelupulone | I, II, V | ++ | Y |
| AH10-F2 | | I, II, V | +++ | Y |
| AH18-F4 | | I, II, V | ++ | Y |
| AH77-F1 | | I, II, V | ++ | Y |
| AH41-F1 | | I, II, V | ++ | Y |
| AH44-F2 | | I, II, V | ++ | Y |
| AH47-F3 | | I, II, V | ++ | Y |
| AH55-F3 | Monoprenylated lupulone | I, II, X | + | Y |
| AH52-F1 | Monoprenylated acetolupulone | I, III, X | - | Y |
| AH56-F3 | Monoprenylated colupulone | I, II, X | - | Y |
| AH57-F3 | Monoprenylated adlupulone | I, II X | + | Y |
| LB68-F3 | Diprenylated lupulone | I, II, IX | ++ | Y |
| AH62-F2 | monoprenylated diacylated colupulone | I, III, X | +++ | Y |
| AH66-F1 | diprenylated diacylated colupulone | I, II, IX | ++ | Y |
| CAS 80902-65-4 | Monoprenylated phloroglucinol (LB.135-F6) | | - | N |

| | | | | |
|---|---|---|---|---|
| * +++: IC₅₀ < 3 µM; ++: IC₅₀ < 12 µM; +: IC₅₀ < 24 µM, -: IC₅₀ > 24 µM | | | | |

**Table 4. IC₅₀ (in µg/mL) of hop derived products on HCoV-229E**

| **hop derived products** | **IC₅₀** |
|---|---|
| Hop (*Humulus lupulus*) methanolic crude extract | 6.38 |
| Dichloromethane sub-extract | 4.19 |
| Alpha-acid fraction | 3.96 |
| Beta-acid fraction | 1.02 |

**Table 5. Beta-acid content and IC₅₀ (in µg/mL) on HCoV-229E of hop crude methanolic extracts**

| **Hop (*Humulus lupulus*) extract** | **Co-lupulone** | **Lupulone** | **Ad-lupulone** | **Total** | **IC₅₀** |
|---|---|---|---|---|---|
| Aves 1 | 18.06 | 17.55 | 11.21 | 46.82 | 4.17 |
| Hem 1 | 17.24 | 16.32 | 9.56 | 43.12 | 3.74 |
| Nugget | 13.25 | 8.86 | 7.39 | 29.5 | 8.14 |
| Douai 5 | 6.51 | 4.15 | 3.47 | 14.13 | 10.90 |
| Aves 5 | 6.34 | 5.03 | 3.43 | 14.8 | 15.58 |

### SARS-CoV and MERS-CoV antiviral assay

To validate the pan-coronavirus activity of the compound, antiviral assays were performed with two highly pathogenic human coronaviruses, SARS-CoV and MERS-CoV. Calu-3 cells seeded in 24-well plates 24 h before inoculation were inoculated with severe acute respiratory syndrome coronavirus (SARS-CoV) and Middle-East respiratory syndrome coronavirus (MERS-CoV) at a MOI of 0.3 in the presence of 1.56, 3.13, 6.25 and 12.5 µM colupulone or AH62-F2 for 1 h at 37°C. Then, the inoculum was removed by 3 washings with DMEM and fresh medium containing colupulone or AH62-F2, at the same concentrations, were added for 15 h at 37°C. Cell supernatants were collected and the amount of infectious virus was determined by infectivity titration. Therefore, Vero-E6 (SARS-CoV) and Huh-7 (MERS-CoV), seeded in 96-well plates, were inoculated with 100 µL of 1/10 serially diluted supernatants (ranging from 10⁻¹ to 10⁻⁸). Cells were incubated with the virus dilutions for 5 days at 37°C and 5% CO₂. Then, the 50% tissue culture infectious dose (TCID50) was determined by assessing the CPE in each well by light microscopy and the 50% end point was calculated according to the method of Reed and Muench (Figure 5). The results showed that both colupulone and AH62-F2 are able to reduce SARS-CoV and MERS-CoV infectious titers by more than 1 × Log10 or 2 × Log10 in a dose-dependent manner.

### Example 16 (Antiviral Activity in the Human Preclinical Model, Primary Airway Cells)

To validate the antiviral capacity of the compounds, antiviral assays against SARS-CoV-2 and HCoV-229E were conducted in human primary epithelial nasal cells (Mucilair^{™}, Epithelix), a preclinical model. The air interface of Mucilair^{™} was rinsed with 100 µL of medium for 10 min 3 times to remove mucosal secretion. The cells were then inoculated at the apical membrane with SARS-CoV-2 or HCoV-229E-Luc at a MOI of 0.2 for 1 h at 37°C. Inoculum was removed and the cells were rinsed with Mucilair^{™} culture medium (Epithelix). In parallel, compounds were added in the basolateral medium. For SARS-CoV-2 assay, 48 h post-infection, viruses secreted at the apical membrane were collected by adding 200 µL of medium in the apical chamber. Viral titers were determined as described for SARS-CoV assay (Example 10). For HCoV-229E assay, cells were lysed and luciferase activity quantified as described (Figure 6). The results showed that colupulone is able to reduce both SARS-CoV-2 and HCoV-229E infection in the human primary epithelial nasal cells.

### References

**[1]** Mandova, T.; Saivish, M.V.; La Serra, L.; Nogueira, M.L.; Da Costa, F.B. Identification of Potential Antiviral Hops Compounds against Chikungunya Virus. Int. J. Mol. Sci. 2023, 24, 3333.
**[2]** Paguet, A.S.; Siah, A.; Lefèvre, G.; Moureu, S. Cadalen, T.; Samaillie, J.; Michels, F.; Deracinois, B.; Flahaut, C.; Dos Santos, H. A.; Etienne-Debaecker, A.; Rambaud, C.; Chollet, S.; Molinié, R.; Fontaine, J.-X.; Waterlot, C.; Fauconnier, M.-L.; Sahpaz, S.; Rivière, C. Multivariate analysis of chemical and genetic diversity of wild Humulus lupulus L. (hop) collected in situ in northern France. Phytochem. 2023, 205, 113508.
**[3]** Paguet, A.S.; Siah, A.; Lefèvre, G.; Vandenberghe, M.; Lutun, D.; Degardin, N.; Samaillie, J.; Mathiron, D.; Dermont, C.; Michels, F.; Fauconnier, M.-L.; Chollet, S.; Molinié, R.; Fontaine, J.-X.; Sahpaz, S.; Rivière, C. Phytochemical characterisation and aromatic potential for brewing of wild hops (Humulus lupulus L.) from Northern France: Towards a lead for local hop varieties. Food Chem. 2024, 433, 137302.
**[4]** ICH, 2005. International conference on harmonisation of technical Requirements for registration of pharmaceuticals for human use. In: Handbook of Transnational Economic Governance Regimes. Brill, Nijhoff, pp. 1041-1053. https://doi.org/ 10.1163/ej.9789004163300.i-1081.897.
**[5]** Bocquet, L., Sahpaz, S., Bonneau, N., Beaufay, C., Mahieux, S., Samaillie, J., Roumy, V., Jacquin, J., Bordage, S., Hennebelle, T., Chai, F., Quetin-Leclercq, J., Neut, C., Rivière, C., 2019. Phenolic compounds from Humulus lupulus as natural antimicrobial products: new weapons in the fight against methicillin resistant Staphylococcus aureus, Leishmania mexicana and Trypanosoma brucei strains. Molecules 24 (6), E1024 pii.
**[6]** Wu, J.; Mu, R.; Sun, M.; Zhao, N.; Pan, M.; Li, H.; Dong, Y.; Sun, Z.; Bai, J.; Hu, M.; Nathan, C.F.; Javid, B.; Liu, G. Derivatives of Natural Product Agrimophol as Disruptors of Intrabacterial pH Homeostasis in Mycobacterium tuberculosis. ACS Infect. Dis. 2019, 5(7), 1087-1104.
**[7]** Cheng, M.-J.; Cao,J.-Q.; Yang, X.-Y.; Zhong, L.-P.; Hu, L.-J.; Lu, X.; Hou, B.-L.; Hu, Y.-J.; Wang, Y.; You, X.-F.; Wang, L.; Ye, Y.-C.; Li, C.-C. Catalytic asymmetric total syntheses of myrtucommuacetalone, myrtucommuacetalone B, and callistrilones A, C, D and E. Chem. Sci. 2018, 9, 1488-1495
**[8]** Tan, H.; Liu, H.; Zhao, L.; Yuan, Y.; Li, B.; Jiang, Y.; Gong, L. Qiu, S. Structure-activity relationships and optimization of acyclic acylphloroglucinol analogues as novel antimicrobial agents. Eur. J. Med. Chem. 2017, 125, 492-499.
**[9]** Ng, C.H.; Rullah, K.; Abas, F.; Lam, K.W.; Ismail, I.S.; Jamaludin, F.; Shaari, K. Hits-to-Lead Optimization of the Natural Compound 2,4,6-Trihydroxy-3-geranyl-acetophenone (tHGA) as a Potent LOX Inhibitor: Synthesis, Structure-Activity Relationship (SAR) Study, and Computational Assignment. Molecules 2018, 23, 2509.
**[10]** Jiang, W.-J.; Ishiuchi, K.; Furukawa, M.; Takamiya, T.; Kitanaka, S.; lijima, H. Stereospecific inhibition of nitric oxide production in macrophage cells by flavanonols: Synthesis and the structure-activity relationship. Bioorg. Med. Chem. 2015, 23, 6922-6929
**[11]** Ebright, R. H.; Freundlich, J.; Mittal, N.; Jaskowski, M.; Shen, J. Preparation of arylpropanoyl, arylpropenoyl, and arylcyclopropanecarboxyl phloroglucinols useful as bacterial RNA polymerase inhibitors for the treatment of bacterial infection. WO2017100645 A2 2017-06-15
**[12]** Thanh Luan, N. N.; Okada, T.; Toyooka, N. Concise Total Synthesis of rac-Betuphenone F. Eur. J. Org. Chem. 2023, 26, e2023000
**[13]** Collins, M.; Laws, D.R.J.; McGuinness, J. D.; Elvidge, J. A. Chemistry of hop constituents. Part XXXVIII. Alkenylation of 2-acylcyclohexane-1,3,5-triones and further evidence concerning the fine structure of hop β-acids. J. Chem. Soc. C 1971, 3814-3818.
**[14]** Sakai, Kunikazu; Satoh, Y.; Doi, K.; Kitamura, K. Preparation of polyhydroxyphenol derivatives as preventive and therapeutic agents for bone and cartilage diseases. WO9743235 A1 1997-11-20
**[15]** Ż 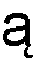 d 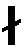 o-Dobrowolska, A.; Hammerer, L.; Pavkov-Keller, T.; Gruber, K.; Kroutil, W. Rational Engineered C-Acyltransferase Transforms Sterically Demanding Acyl Donors. *ACS Catal.* **2020,** *10(2),* 1094-1101.
**[16]** Jida, M.; Sanchez, C.P.; Mounien, S.; Urgin, K.; Ehrhardt, K.; Elhabiri, M.; Lanzer, M.; Davioud-Charvet, E. A fluorescent pH indicator and substrate of PfCRT for detecting P. falciparum strains with reduced responsiveness to quinoline antimalarial drugs. ACS Inf. Dis. 2017, 3, 119-131.
**[17]** Jürss, R.; Maelicke, A. Synthesis of NBD-5-acylcholine. J. Biol. Chem. 1983, 258(17), 10272.
**[18]** Desmarets, L.; Callens, N.; Hoffmann, E.; Danneels, A.; Lavie, M.; Couturier, C.; Dubuisson, J.; Belouzard, S.; Rouillé, Y. A reporter cell line for the automated quantification of SARS-CoV-2 infection in living cells. Front. Microbiol. 2022, 13, 1031204.
**[19]** Sakai, K.; Satoh, Y.; Doi, K.; Kitamura, K. Polyhydroxybenzene Derivatives and Preventive/Remedy for Bone and Cartilage Diseases PCT/JP1998/002266 1998-05-22, WO/1998/052899 1998-11-26.

## Claims

1. A compound of formula (I): 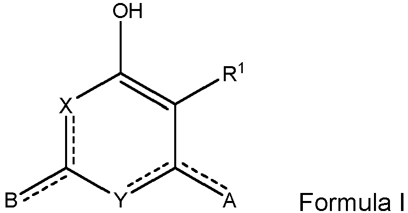 wherein,
- R¹ is a substituted or unsubstituted, saturated or unsaturated, linear or branched, acyl group comprising 10 or fewer carbon atoms, optionally comprising an aryl moiety;
- A represents O or OH
- B represents O, OH, or OR⁶, in which R⁶ represents group or a C₁ to C₃ alkyl chain optionally comprising a C₁ to C₃ alkoxy or a phenyl;
provided that
- when A is O, then Y is C(R²)₂, in which each R² group independently represents H or a linear or branched C₅ to C₁₀ unsaturated hydrocarbon chain comprising at least one alkene moiety;
- when A is OH, then Y is CR³, in which R³ represents H or a linear or branched C₅ to C₁₀ unsaturated hydrocarbon chain comprising at least one alkene moiety;
- when B is O, then X is C(R⁴)₂, in which each R⁴ independently represents H or a linear or branched C₅ to C₁₀ unsaturated hydrocarbon chain comprising at least one alkene moiety;
- when B is OH or OR⁶, then X is CR⁵, in which R⁵ represents H, a linear or branched C₅ to C₁₀ unsaturated hydrocarbon chain comprising at least one alkene moiety or a substituted or unsubstituted, saturated or unsaturated, linear or branched, acyl group comprising 10 or fewer carbon atoms, optionally comprising an aryl moiety;
- wherein the symbol 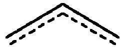 represents a chain comprising one double bond, the position of the double bond being linked to the nature of A and/or B,
for use in the treatment of diseases caused by a virus chosen from coronaviruses belonging to the Coronaviridae family.

2. The compound for use according to claim 1, wherein the virus belongs to the Orthocoronavirinae subfamily, and preferably either to the Alphacoronavirus genus and the Duvinacovirus subgenus (HCoV-229E, Human coronavirus 229E), or to the Betacoronavirus genus and the Sarbecovirus subgenus (SARS-CoV, Severe acute respiratory syndrome coronavirus ; SARS-CoV-2, Severe acute respiratory syndrome coronavirus-2, original Wuhan strain and D614G, Alpha, Beta, Delta and Omicron variants), or to the Betacoronavirus genus and Merbecovirus subgenus (MERS-CoV, Middle-East respiratory syndrome coronavirus).

3. The compound for use according to claim 1 or 2, wherein the linear or branched C₅ to C₁₀ unsaturated hydrocarbon chain(s) comprising at least one alkene moiety are selected from prenyl and geranyl groups.

4. The compound for use according to any preceding claims, chosen from the compounds of formula (II): 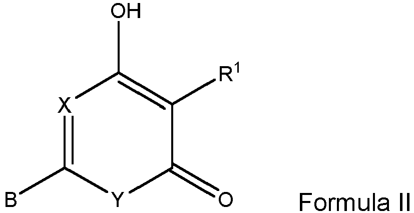 wherein B is OH or OR⁶, and R¹, R⁶, X and Y are defined as in any of claims 1 to 3.

5. The compound for use according to any preceding claims, chosen from the compounds of formula (III): 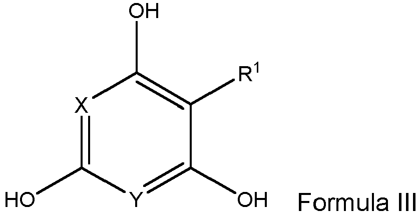 wherein R¹, X and Y are defined as in any of claims 1 to 3.

6. The compound for use according to any preceding claims, chosen from the compounds of formula (IV): 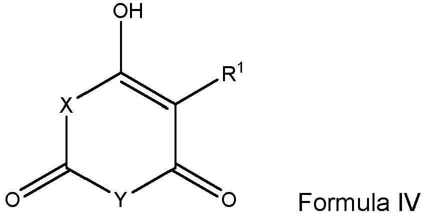 wherein R¹, X and Y are defined as in any of claims 1 to 3.

7. The compound for use according to any preceding claims, chosen from the compounds of formula (V): 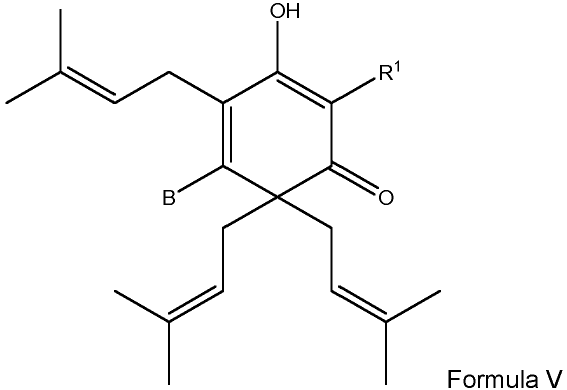 wherein B is OH or OR⁶, R¹ and R⁶ being defined as in any of claims 1 to 3.

8. The compound for use according to any preceding claims, chosen from the compounds of formula (VI): 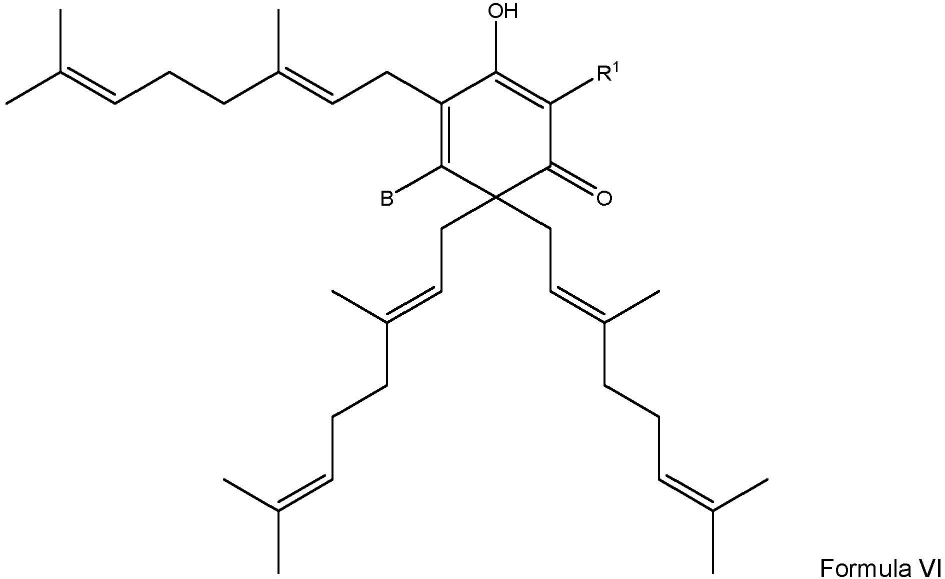 wherein B is OH or OR⁶, R¹ and R⁶ being defined as in any of claims 1 to 3.

9. The compound for use according to any preceding claims, chosen from the compounds of formula (VII): 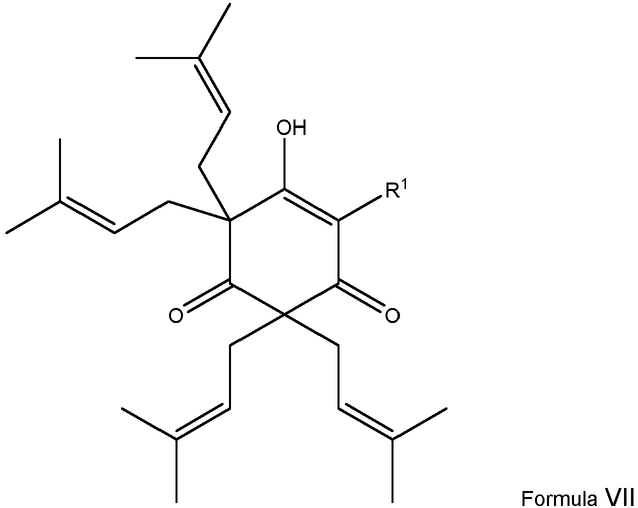 wherein R¹ is defined as in any of claims 1 to 3.

10. The compound for use according to any preceding claims, chosen from the compounds of formula (IX): 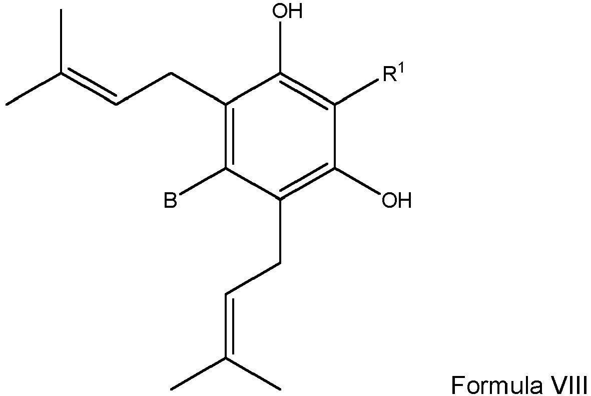 wherein B is OH or OR⁶, R¹ and R⁶ being defined as in any of claims 1 to 3.

11. The compound for use according to any preceding claims, chosen from the compounds of formula (IX): 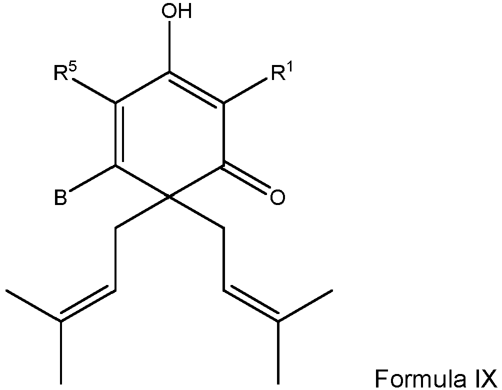 wherein B is OH or OR⁶, R¹, R⁵ and R⁶ being as in any of claims 1 to 3.

12. The compound for use according to any preceding claims, chosen from the compounds of formula (X): 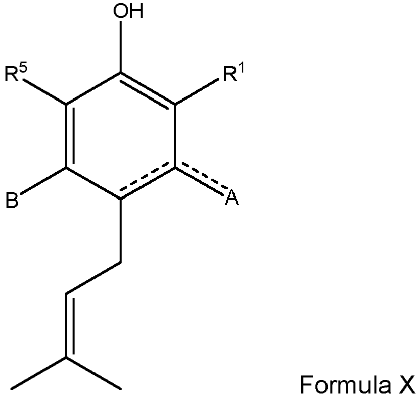 wherein A is O or OH and B is OH or OR⁶, R⁵ is H or a substituted or unsubstituted, saturated or unsaturated, linear or branched, acyl group comprising 10 or fewer carbon atoms, optionally comprising an aryl moiety and R⁶ is defined as above as in any of claims 1 to 6.

13. A compound of formula (VIII-2): 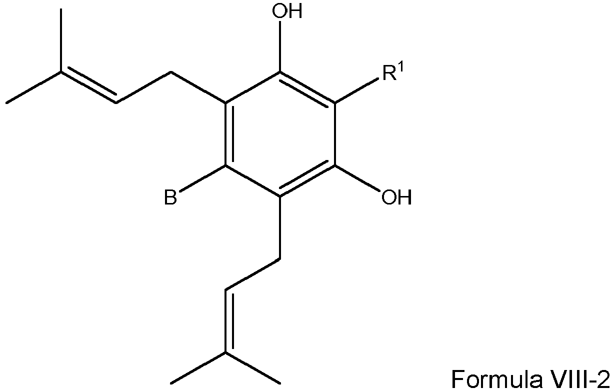 wherein:
- B is OH or OR⁶, R⁶ being defined as above, and
- R¹ is a substituted or unsubstituted, saturated or unsaturated, linear or branched, acyl group comprising 10 or fewer carbon atoms and comprising an aryl moiety, preferably R¹ is chosen from the following structures:

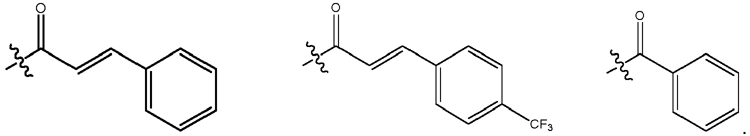

14. A compound of formula (IX-2): 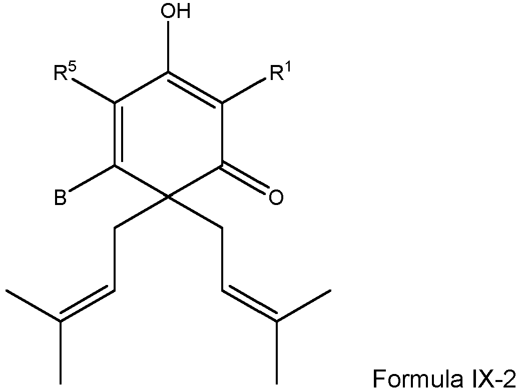 wherein B is OH or OR⁶, R¹, R⁵ and R⁶ being as defined above, preferably, R¹ and R⁵ are branched acyl groups comprising 4 or fewer carbon atoms, or linear acyl group comprising 10 or fewer carbon atoms.

15. A compound of formula (X-2): 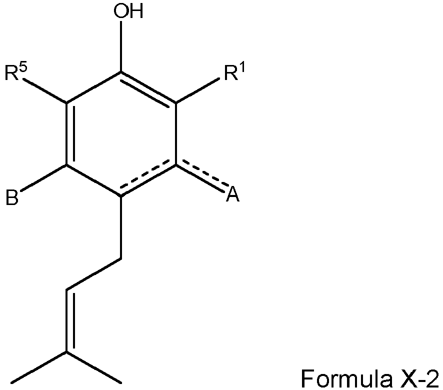 wherein A is O or OH and B is OH or OR⁶, R⁵ is H or a substituted or unsubstituted, saturated or unsaturated, linear or branched, acyl group comprising 10 or fewer carbon atoms, optionally comprising an aryl moiety and R⁶ is defined as above.

16. A compound of formula (VI-2): 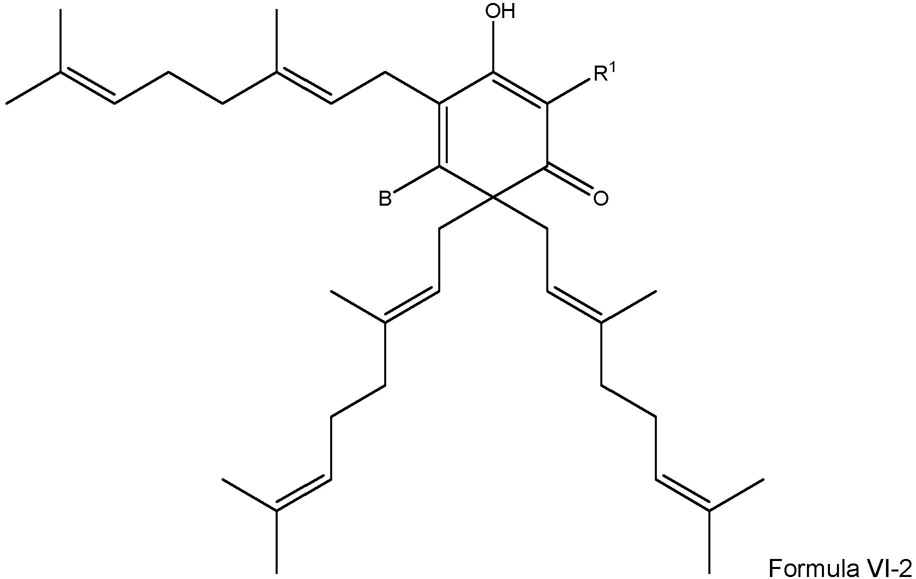 wherein B is OH or OR⁶, R¹ and R⁶ being defined as above, preferably, R¹ is a substituted or unsubstituted, saturated or unsaturated, linear or branched, acyl group comprising 4 or fewer carbon atoms, optionally comprising an aryl moiety.
